# EUROPEAN PATENT APPLICATION

(11) **EP 4 014 988 A1**
(43) Date of publication of application: **22.06.2022**
(21) Application number: 20851845.6
(22) Date of filing: 12.08.2020
(51) Int. Cl.: A61K 39/02, A61K 39/04, A61K 39/12, A61P 35/00, A61P 37/04, C12N 5/0783, G01N 33/68

(54) **NOVEL TREATMENT AND PREVENTION OF DISEASE BASED ON IMMUNOLOGICAL MEMORY**

(30) Priority: 13.08.2019 JP 2019148551; 06.12.2019 WO PCT/JP2019/047945; 06.12.2019 TW 108144810
(71) Applicant: National Institutes of Biomedical Innovation, Health and Nutrition, Ibaraki-shi, Osaka 567-0085 (JP); Zeria Pharmaceutical Co., Ltd., Tokyo 103-8351 (JP)
(72) Inventor: ISHII, Ken, Ibaraki-shi, Osaka 567-0085 (JP); KOBAYASHI, Nobuyoshi, Tokyo 103-8351 (JP); OHIRA, Yuta, Tokyo 103-8351 (JP); SETO, Koichi, Toyoake-shi, Aichi 470-1192 (JP)
(74) Representative: Engelhard, Markus
(86) International application number: PCT/JP2020/030710
(87) International publication number: WO 2021/029424

(57) **Abstract**

The present disclosure provides a composition for preventing an immune disorder, recovering from an immune disorder, preventing an immune disorder from occurring and preventing or treating a disease, disorder or condition. The present disclosure provides a composition for preventing an immune disorder, recovering from an immune disorder, preventing an immune disorder from occurring and preventing or treating a disease, disorder or condition in a subject, said composition comprising an antigen component, which is specific to the subject (or immunological memory of which remains in the subject), against a component which is different from a causative factor of the disease, disorder or condition.

## Description

### [Technical Field]

The present disclosure relates to a new disease therapy and prevention based on immunological memory. In one aspect, the present disclosure relates to mechanism based cancer prevention/therapy. The present disclosure relates to, in a specific example, a cancer therapy method or prevention method using a memory response by a non-tumor antigen from a pathogen from a past infection such as a Mycobacterium tuberculosis extract as an individualization marker.

### [Background Art]

The technology for therapy of diseases has been developed from various approaches.

For example for cancer therapy, current cancer therapy is largely classified into the following three categories. (1) A therapeutic method for introducing a cancer specific antigen (WT1 or cancer specific neoantigen) with an adjuvant base, and a therapeutic vaccine or a cancer antigen dependent chimeric T cell, or a therapeutic method for introducing an antigen presenting cell (DC), which requires high medical expenses as well as a cancer antigen for each patient. While a specific immunotherapy can be administered due to the use of a specific cancer antigen, therapy can be administered to only certain cancer antigen bearing patients. Since a cancer antigen is required, such a therapeutic method cannot be used for healthy individuals. Thus, the method cannot be used for prevention. (2) An inhibitor against a checkpoint molecule suppressing cancer immunity. An immune checkpoint molecule is a molecule that regulates a T cell response in the body. An immune checkpoint molecule inhibitor inhibits an immunosuppressive molecule expressed on a cancer cell or regulatory T-cell of a host and promotes antitumor immunity. However, the problem to be solved of these agents is that they exhibit a useful effect, but have potent side effects such as increased risk of inducing an autoimmune disease or the like because the action on immune reactions that should be suppressed are also disabled. Therefore, the agents are not suitable for preventive treatment targeted for healthy individuals. (3) A vaccine for use in preventing oncogenesis by prevention of infections. While this includes a vaccine intended for preventing HPV infections associated with cervical cancer, the target cancer is limited.

### [Summary of Invention]

### [Solution to Problem]

The inventors have developed a novel disease therapy and prevention based on immunological memory as a result of diligent study. As an aspect thereof, the present disclosure provides a mechanism based cancer prevention/therapy. A representative example thereof includes a cancer therapy method or prevention method using a memory response by a non-tumor antigen from a pathogen from a past infection such as a Mycobacterium tuberculosis extract as an individualization marker.

Therefore, the present disclosure provides the following as representative aspects.
(Item 1) A composition for use in activating a regulatory T cell (Treg) having immunological memory of a non-target antigen component, which is suppressed in a subject, against a target, comprising the non-target antigen component.
(Item 2) The composition of any one of the preceding item, wherein the activation of Treg imparts an ability to kill the target or an immunostimulatory action against the target.
(Item 3) A composition for use in activating a regulatory T cell (Treg) having immunological memory of a non-tumor antigen component, which is suppressed in a subject, comprising the non-tumor antigen component.
(Item 4) The composition of any one of the preceding items, wherein the activation of Treg imparts an ability to kill tumor or an immunostimulatory action against tumor.
(Item 5) The composition of any one of the preceding items, wherein the Treg is a memory T cell.
(Item 6) The composition of any one of the preceding items, wherein the Treg is CD4 positive.
(Item 7) The composition of any one of the preceding items, wherein the antigen component comprises a protein.
(Item 8) The composition of any one of the preceding items, wherein the antigen component comprises an antigen selected from the group consisting of a pathogen of an infection or a part thereof, an antigen associated with anamnesis, and an antigen associated with vaccination history.
(Item 9) The composition of any one of the preceding items, wherein the antigen component comprises a human *Mycobacterium tuberculosis* hot water extract or an influenza virus antigen.
(Item 9A) The composition of any one of the preceding items, further comprising one or more features of any one or more of the preceding items and the following items.
(Item 10) A composition characterized in that the composition is used in a method comprising checking whether the antigen component has immunological memory of Treg in the subject, and administering the antigen component to the subject if the subject has immunological memory of the antigen component.
(Item 11) A composition for use in treating or preventing a disease, disorder, or condition associated with an immunological abnormality of a subject, the composition comprising an antigen component that is specific in the subject to a component that is different from a causative agent of the disease, disorder, or condition.
(Item 12) The composition of any one of the preceding items, wherein the disease, disorder, or condition comprises cancer, and wherein, preferably, the antigen component is a non-tumor antigen component.
(Item 13) The composition of any one of the preceding items, wherein the antigen component is specific to a memory T-cell of the subject.
(Item 14) The composition of any one of the preceding items, wherein the memory T cell is a memory regulatory T cell (IL-2 producing).
(Item 15) The composition of any one of the preceding items, wherein the antigen component has an immunostimulatory action.
(Item 16) The composition of any one of the preceding items, wherein the antigen component antigen-dependently acts on memory CD4 positive T cells.
(Item 17) The composition of any one of the preceding items, wherein the antigen component has activity to bias a ratio of presence between Foxp3 positive Treg cells and IFN-γ producing T cells.
(Item 18) The composition of any one of the preceding items, wherein the bias is an increase in IFN-γ producing T cells compared to Foxp3 positive Treg cells.
(Item 19) The composition of any one of the preceding items, wherein the antigen component has activity to bias a ratio of presence between Foxp3 positive Treg cells and type 1 helper T cells.
(Item 20) The composition of any one of the preceding items, wherein the IFN-γ producing T cells comprise type 1 helper T cells.
(Item 21) The composition of any one of the preceding items, wherein the antigen component has activity to bias a ratio of presence of Th1 cells.
(Item 22) The composition of any one of the preceding items, wherein the IFN-γ producing T cells are T-bet positive Th1 cells.
(Item 23) The composition of any one of the preceding items, wherein the antigen component that is specific has a capability to enhance at least one selected from the group consisting of IFN-γ producing capability, IL-2 producing capability, and TNF-α producing capability in a sample from the subject.
(Item 24) The composition of any one of the preceding items, wherein the antigen component is a protein.
(Item 25) A biomarker for determining whether a non-tumor antigen component has anticancer action on a subject, the biomarker comprising at least one selected from the group consisting of whether the antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item 26) A composition or a kit comprising an agent or means for detecting a biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item 27) The composition of any one of the preceding items, wherein the (non-tumor) antigen component comprises an antigen associated with anamnesis and vaccination history.
(Item 28) The composition of any one of the preceding items, wherein the subject is a subject with a history of an infection, and the antigen component comprises an antigen to the infection.
(Item 29) The composition of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic *Escherichia coli,* toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item 30) The composition of any one of the preceding items, wherein the subject is a subject with BCG vaccination history, tuberculosis infection history, or antigen responsiveness to *Mycobacterium tuberculosis,* and the antigen component comprises a human *Mycobacterium tuberculosis* hot water extract.
(Item 31) The composition of any one of the preceding items, wherein the subject is a subject with influenza vaccination history, influenza infection history, or antigen responsiveness to an influenza virus, and the antigen component comprises an influenza virus.
(Item 32) The composition of any one of the preceding items, wherein the disease, disorder, or condition comprises melanoma.
(Item 33) The composition of any one of the preceding items, wherein the antigen component comprises a protein, a part thereof, or a peptide.
(Item 34) The composition of any one of the preceding items, wherein the antigen component comprises a component that can elicit an immune response via CD4 positive T cells.
(Item 35) The composition of any one of the preceding items, wherein the subject is checked as to whether the subject can elicit an antitumor immune response via CD4 positive T cells, and if the subject can elicit an antitumor immune response via CD4 positive T cells, the composition is administered.
(Item 36) A composition for use in treating or preventing a disease, disorder, or condition associated with an immunological abnormality of a subject, the composition comprising an antigen component that is specific in the subject to a component that is different from a causative agent of the disease, disorder, or condition, wherein
   the disease, disorder, or condition comprises melanoma,
   the antigen component is a protein, a part thereof, or a peptide, and can elicit an immune response via CD4 positive T cells, and
   the cancer comprises cancer that is treatable and preventable with an immune response via CD4 positive T cells,
   wherein the subject is checked as to whether the subject can elicit an antitumor immune response via CD4 positive T cells, and if the subject can elicit an antitumor immune response via CD4 positive T cells, the composition is administered.
(Item 37) A composition for use in treating or preventing cancer or tumor in a subject, the composition comprising a non-tumor antigen component, wherein the non-tumor antigen component activates a regulatory T cell (Treg) having immunological memory of the non-tumor antigen component, which is suppressed in the subject, and wherein the Treg has an effect of promoting regulatory activity or antitumor immunological action on cancer or tumor.
(Item 38) A method of manufacturing or otherwise providing a composition for use in preventing or treating cancer of a subject, the method comprising:
   A) identifying a non-tumor antigen specific to the subject;
   B) identifying whether a subject has immunological memory of the non-tumor antigen, and selecting a non-tumor antigen for which the subject has the immunological memory; and
   C) manufacturing or otherwise providing the selected non-tumor antigen.
(Item 39) The method of any one of the preceding items, having one or more features in any one of the preceding items in B).
(Item 40) A method of determining whether a non-tumor antigen of a subject can prevent or treat cancer of the subject, the method comprising:
   B) identifying whether the subject has immunological memory of the non-tumor antigen, and identifying that cancer of the subject can be prevented or treated if the subject has the immunological memory.
(Item 41) The method of any one of the preceding items, having one or more features in any one of the preceding items in B).
(Item 42) A method for use in preventing or treating a disease, disorder, or condition associated with an immunological abnormality, comprising:
   a) obtaining an antigen responsiveness profile of a subject;
   b) identifying an antigen component or a combination of antigen components from the antigen responsiveness profile, based on whether the antigen component or the combination of antigen components has shown or shows to present immune response to the subject; and
   c) administering to the subject the antigen component or the combination of antigen components identified in step b) at a sufficient amount to elicit an immune response in the subject.
(Item 43) The method of any one of the preceding items, wherein the disease, disorder, or condition comprises cancer.
(Item 44) The method of any one of the preceding items, wherein the obtaining an antigen responsiveness profile comprises checking a past physical condition of a subject, checking whether one or more of candidate antigens has responsiveness in a sample from the subject, or both.
(Item 45) The method of any one of the preceding items, wherein the obtaining an antigen responsiveness profile comprises identifying an antigen component or a combination of antigen components that elicit an immune response via CD4 positive T cells.
(Item 46) The method of any one of the preceding items, wherein
   i) the antigen responsiveness profile comprises at least one selected from the group consisting of interview, anamnesis or vaccination history based on a Maternal and Child Health Handbook, an equivalent thereof, or the like, and a combination thereof, and/or
   ii) the checking whether one or more of candidate antigens has responsiveness comprises collecting a bodily fluid (e.g., blood) from the subject and separating peripheral blood cells, and then measuring whether the peripheral blood cells produce a cytokine in response to an antigen associated with the antigen profile, and other biomarkers.
(Item 47) The method of any one of the preceding items, further comprising periodically testing responsiveness of the antigen and confirming that responsiveness is maintained.
(Item 48) The method of any one of the preceding items, wherein a) and b) are performed with the following steps:
   i) obtaining a past physical condition of a subject;
   ii) collecting blood from the subject and separating peripheral blood cells, and then measuring whether the peripheral blood cells produce a cytokine in response to an antigen corresponding to the physical condition, and other biomarkers; and
   iii) identifying a suitable antigen component or a combination antigen components from a result of ii).
(Item 49) The method of any one of the preceding items, wherein the past physical condition comprises anamnesis and vaccination history.
(Item 50) The method of any one of the preceding items, wherein the physical condition comprises history of infection, and the cancer vaccine comprises an antigen component or a combination of antigen components to the infection.
(Item 51) The method of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic *Escherichia coli,* toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item 52) The method of any one of the preceding items, wherein the physical condition comprises BCG vaccination history, tuberculosis infection history, or antigen responsiveness to *Mycobacterium tuberculosis,* and the antigen component or combination of antigen components comprises a human *Mycobacterium tuberculosis* hot water extract.
(Item 53) The method of any one of the preceding items, wherein the physical condition comprises influenza vaccination history, influenza infection history, or antigen responsiveness to an influenza virus, and the antigen component or combination of antigen components comprises an influenza virus.
(Item 54) The method of any one of the preceding items, wherein the administration comprises subcutaneous administration or intradermal administration.
(Item 55) The method of any one of the preceding items, wherein the subject is in a state before onset of cancer, after a cancer treatment, an early stage of onset of cancer, or a precancerous condition.
(Item 56) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item 57) The method of any one of the preceding items, wherein the subject exhibits immunological resistance.
(Item 58) The method of any one of the preceding items, wherein step ii) comprises measuring induction of cells producing IFN-γ, IL-2, TNF-α, or a plurality of the cytokines concurrently.
(Item 59) The method of any one of the preceding items, wherein the antigen responsiveness profile is obtained by performing companion diagnosis in advance based on anamnesis and vaccination history.
(Item 60) The method of any one of the preceding items, wherein the past physical condition and the antigen component or combination of antigen components are tuberculosis infection history and a human *Mycobacterium tuberculosis* hot water extract.
(Item 61) The method of any one of the preceding items, wherein the past physical condition and the antigen component or combination of antigen components are influenza infection history and an influenza virus.
(Item 62) The method of any one of the preceding items, wherein the past physical condition and the antigen component or combination of antigen components are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic *Escherichia coli,* toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item 63) The method of any one of the preceding items, wherein the subject is a subject who has BCG vaccination history or tuberculosis infection history, or is confirmed to have antigen responsiveness,
wherein the *Mycobacterium tuberculosis* extract is prophylactically administered before onset or administered in an early stage of onset of cancer, and an extract from *Mycobacterium tuberculosis* is subcutaneously or intradermally administered by a conventional method in an early stage of onset of cancer or a precancerous condition.
(Item 64) The method of any one of the preceding items, wherein the subject is a subject who has influenza vaccination history or influenza infection history, or is confirmed to have antigen responsiveness,
wherein the influenza vaccine is prophylactically administered before onset, administered to prevent recurrence after therapy, or administered in an early stage of onset of cancer, and an influenza vaccine is subcutaneously or intradermally administered in an early stage of onset of cancer or a precancerous condition.
(Item 65) A method of preventing or treating cancer immunity based on any one of the preceding items, comprising revaccinating the antigen component or combination of antigen components.
(Item 66) A method of preventing or treating cancer of a subject with a non-tumor component, wherein the component is an antigen or extract identified by interview and/or identified by reference to anamnesis or vaccination history of the subject, wherein the subject is an individual with an infection history or vaccination history described in any one of the preceding items, wherein the component is administered to prevent recurrence after therapy, administered prophylactically before onset, or administered in an early stage of onset of cancer to the subject, and the component is optionally administered in an early stage of onset of cancer or a precancerous condition.
(Item 67) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item 68) The method of any one of the preceding items, wherein the subject is a patient exhibiting immunological resistance.
(Item 69) The method of any one of the preceding items, wherein the identification of responsiveness is characterized by infection history, vaccination history, and measuring induction of cells producing IFN-γ, IL-2, TNF-α, or a plurality of the cytokines concurrently using peripheral blood.
(Item 70) The method of any one of the preceding items, wherein the *Mycobacterium tuberculosis* extract is a hot water extract of human *Mycobacterium tuberculosis* or other extract from *Mycobacterium tuberculosis* (highly safe extract).
(Item 71) The method of any one of the preceding items, wherein the influenza virus is a human influenza virus or other extract from an influenza virus (highly safe extract).
(Item 72) The method of any one of the preceding items, wherein the antigen component is a protein.
(Item 73) A vaccine formulation comprising the antigen of any one of the preceding items and an adjuvant base.
(Item 74) The vaccine formulation of any one of the preceding items, wherein the adjuvant base comprises a substance promoting a Th1 immune response.
(Item 75) The vaccine formulation of any one of the preceding items, wherein the vaccine formulation is used for personalized medicine.
(Item 76) A composition for use in treating or preventing a disease, disorder, or condition associated with an immunological abnormality of a subject, wherein the composition comprise an antigen component that is specific in the subject to a component that is different from a causative agent of the disease, disorder, or condition, and is subcutaneously or intratumorally administered once a day (first week) and once a week (second week and thereafter).
(Item 77) The composition of any one of the preceding items, wherein the antigen component is contained at about 0.001 µg or more per unit formulation.
(Item 78) A method of treating or preventing cancer or tumor in a subject, comprising:
   a) identifying a non-tumor antigen specific to the subject based on an antigen responsiveness profile;
   b) identifying whether the subject has immunological memory of the non-tumor antigen to identify a subject having the immunological memory; and
   c) administering the non-tumor antigen to the subject identified as having the immunological memory.
(Item 79) The method of any one of the preceding items, wherein the antigen responsiveness profile comprises vaccination history and/or infection history.
(Item 80) The method of any one of the preceding items, wherein the identifying a subject having the immunological memory stimulates peripheral blood mononuclear cells (PBMC) isolated from the subject or infiltrating immune cells isolated from a tumor mass with the non-tumor antigen, measuring cytokine production, and identifying a subject with an amount of cytokine production increased a predetermined factor compared to the amount before stimulation as a subject having the immunological memory.
(Item 81) The method of any one of the preceding items, wherein the non-tumor antigen is administered once a day (first week) and once a week (second week and thereafter).
(Item 82) The method of any one of the preceding items, wherein the non-tumor antigen is administered at about 0.001 µg/dose to about 1 mg/dose.
(Item 83) A composition for use in treating or preventing a disease, disorder, or condition associated with an immunological abnormality of a subject, comprising mHSP10 and/or MTB12 and/or lipoprotein LpqH.
(Item 84) The composition, kit, biomarker, vaccine formulation, or method of any one of the preceding items, comprising a plurality of agents comprising the antigen component.
(Item 85) The composition, kit, biomarker, vaccine formulation, or method of any one of the preceding items, wherein each component of the plurality of agents is provided as separate compositions.
(Item A1) A method for activating a regulatory T cell (Treg) having immunological memory of a non-target antigen component, which is suppressed in a subject, comprising administering an effective amount of the non-target antigen component to the subject.
(Item A2) The method of any one of the preceding items, wherein the activation of Treg imparts an ability to kill the target or an immunostimulatory action against the target.
(Item A3) A method for activating a regulatory T cell (Treg) having immunological memory of a non-tumor antigen component, which is suppressed in a subject, comprising administering an effective amount of the non-tumor antigen component to the subject.
(Item A4) The method of any one of the preceding items, wherein the activation of Treg imparts an ability to kill tumor or an immunostimulatory action against tumor.
(Item A5) The method of any one of the preceding items, wherein the Treg is a memory T cell.
(Item A6) The method of any one of the preceding items, wherein the Treg is CD4 positive.
(Item A7) The method of any one of the preceding items, wherein the antigen component comprises a protein.
(Item A8) The method of any one of the preceding items, wherein the antigen component comprises an antigen selected from the group consisting of a pathogen of an infection or a part thereof, an antigen associated with anamnesis, and an antigen associated with vaccination history.
(Item A9) The method of any one of the preceding items, wherein the antigen component comprises a human *Mycobacterium tuberculosis* hot water extract or an influenza virus antigen.
(Item A9A) The method of any one of the preceding items, further comprising one or more features of any one or more of the preceding items and the following items.
(Item A10) A method comprising checking whether the antigen component has immunological memory of Treg in the subject, and administering an effective amount of the antigen component to the subject if the subject has immunological memory for the antigen component.
(Item A11) A method for use in treating or preventing a disease, disorder, or condition associated with an immunological abnormality of a subject, comprising administering to the subject an effective amount of an antigen component that is specific in the subject to a component that is different from a causative agent of the disease, disorder, or condition.
(Item A12) The method of any one of the preceding items, wherein the disease, disorder, or condition comprises cancer, and wherein, preferably, the antigen component is a non-tumor antigen component.
(Item A13) The method of any one of the preceding items, wherein the antigen component is specific to a memory T-cell of the subject.
(Item A14) The method of any one of the preceding items, wherein the memory T cell is a memory regulatory T cell (IL-2 producing).
(Item A15) The method of any one of the preceding items, wherein the antigen component has an immunostimulatory action.
(Item A16) The method of any one of the preceding items, wherein the antigen component antigen-dependently acts on memory CD4 positive T cells.
(Item A17) The method of any one of the preceding items, wherein the antigen component has activity to bias a ratio of presence between Foxp3 positive Treg cells and IFN-γ producing T cells.
(Item A18) The method of any one of the preceding items, wherein the bias is an increase in IFN-γ producing T cells compared to Foxp3 positive Treg cells.
(Item A19) The method of any one of the preceding items, wherein the antigen component has activity to bias a ratio of presence between Foxp3 positive Treg cells and type 1 helper T cells.
(Item A20) The method of any one of the preceding items, wherein the IFN-γ producing T cells comprise type 1 helper T cells.
(Item A21) The method of any one of the preceding items, wherein the antigen component has activity to bias a ratio of presence of Th1 cells.
(Item A22) The method of any one of the preceding items, wherein the IFN-γ producing T cells are T-bet positive Th1 cells.
(Item A23) The method of any one of the preceding items, wherein the antigen component that is specific has a capability to enhance at least one selected from the group consisting of IFN-γ producing capability, IL-2 producing capability, and TNF-α producing capability in a sample from the subject.
(Item A24) The method of any one of the preceding items, wherein the antigen component is a protein.
(Item A25) A method for determining whether a non-tumor antigen component has anticancer action on a subject, comprising determining using a biomarker comprising at least one selected from the group consisting of whether the antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item A26) A method for detecting a biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item A27) The method of any one of the preceding items, wherein the (non-tumor) antigen component comprises an antigen associated with anamnesis and vaccination history.
(Item A28) The method of any one of the preceding items, wherein the subject is a subject with a history of an infection, and the antigen component comprises an antigen to the infection.
(Item A29) The method of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic *Escherichia coli,* toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item A30) The method of any one of the preceding items, wherein the subject is a subject with BCG vaccination history, tuberculosis infection history, or antigen responsiveness to *Mycobacterium tuberculosis,* and the antigen component comprises a human *Mycobacterium tuberculosis* hot water extract.
(Item A31) The method of any one of the preceding items, wherein the subject is a subject with influenza vaccination history, influenza infection history, or antigen responsiveness to an influenza virus, and the antigen component comprises an influenza virus.
(Item A32) The method of any one of the preceding items, wherein the disease, disorder, or condition comprises melanoma.
(Item A33) The method of any one of the preceding items, wherein the antigen component comprises a protein, a part thereof, or a peptide.
(Item A34) The method of any one of the preceding items, wherein the antigen component comprises a component that can elicit an immune response via CD4 positive T cells.
(Item A35) The method of any one of the preceding items, wherein the subject is checked as to whether the subject can elicit an antitumor immune response via CD4 positive T cells, and if the subject can elicit an antitumor immune response via CD4 positive T cells, the composition is administered.
(Item A36) A method for use in treating or preventing a disease, disorder, or condition associated with an immunological abnormality of a subject, comprising administering to the subject an effective amount of an antigen component that is specific in the subject to a component that is different from a causative agent of the disease, disorder, or condition, wherein
   the disease, disorder, or condition comprises melanoma,
   the antigen component is a protein, a part thereof, or a peptide, and can elicit an immune response via CD4 positive T cells, and
   the cancer comprises cancer that is treatable and preventable with an immune response via CD4 positive T cells,
   wherein the subject is checked as to whether the subject can elicit an antitumor immune response via CD4 positive T cells, and if the subject can elicit an antitumor immune response via CD4 positive T cells, the antigen component is administered.
(Item A37) A method for treating or preventing cancer or tumor in a subject, comprising administering an effective amount of a non-tumor antigen component, wherein the non-tumor antigen component activates a regulatory T cell (Treg) having immunological memory of the non-tumor antigen component, which is suppressed in the subject, and wherein the Treg has an effect of promoting regulatory activity or antitumor immunological action on cancer or tumor.
(Item A38) A method of manufacturing or otherwise providing a composition for use in preventing or treating cancer of a subject, the method comprising:
   A) identifying a non-tumor antigen specific to the subject;
   B) identifying whether a subject has immunological memory of the non-tumor antigen, and selecting a non-tumor antigen for which the subject has the immunological memory; and
   C) manufacturing or otherwise providing the selected non-tumor antigen.
(Item A39) The method of any one of the preceding items, having one or more features in any one of the preceding items in B).
(Item A40) A method of determining whether a non-tumor antigen of a subject can prevent or treat cancer of the subject, the method comprising:
   B) identifying whether the subject has immunological memory of the non-tumor antigen, and identifying that cancer of the subject can be prevented or treated if the subject has the immunological memory.
(Item A41) The method of any one of the preceding items, having one or more features in any one of the preceding items in B).
(Item A42) A method for use in preventing or treating a disease, disorder, or condition associated with an immunological abnormality, comprising:
   a) obtaining an antigen responsiveness profile of a subject;
   b) identifying an antigen component or a combination of antigen components from the antigen responsiveness profile, wherein the antigen component or combination of antigen components is identified based on whether the antigen component or combination of antigen components has shown or shows to present immune response to the subject; and
   c) administering to the subject the antigen component or combination of antigen components identified in step b) at a sufficient amount to elicit an immune response in the subject.
(Item A43) The method of any one of the preceding items, wherein the disease, disorder, or condition comprises cancer.
(Item A44) The method of any one of the preceding items, wherein the obtaining an antigen responsiveness profile comprises checking a past physical condition of a subject, checking whether one or more of candidate antigens has responsiveness in a sample from the subject, or both.
(Item A45) The method of any one of the preceding items, wherein the obtaining an antigen responsiveness profile comprises identifying an antigen component or a combination of antigen components that elicits an immune response via CD4 positive T cells.
(Item A46) The method of any one of the preceding items, wherein
   i) the antigen responsiveness profile comprises at least one selected from the group consisting of interview, anamnesis or vaccination history based on a Maternal and Child Health Handbook, an equivalent thereof, or the like, and a combination thereof, and/or
   ii) the checking whether one or more of candidate antigens has responsiveness comprises collecting a bodily fluid (e.g., blood) from the subject and separating peripheral blood cells, and then measuring whether the peripheral blood cells produce a cytokine in response to an antigen associated with the antigen profile, and other biomarkers.
(Item A47) The method of any one of the preceding items, further comprising periodically testing responsiveness of the antigen and confirming that responsiveness is maintained.
(Item A48) The method of any one of the preceding items, wherein a) and b) are performed with the following steps:
   i) obtaining a past physical condition of a subject;
   ii) collecting blood from the subject and separating peripheral blood cells, and then measuring whether the peripheral blood cells produce a cytokine in response to an antigen corresponding to the physical condition, and other biomarkers; and
   iii) identifying a suitable antigen component or a combination of antigen components from a result of ii).
(Item A49) The method of any one of the preceding items, wherein the past physical condition comprises anamnesis and vaccination history.
(Item A50) The method of any one of the preceding items, wherein the physical condition comprises history of infection, and the cancer vaccine comprises an antigen component or a combination of antigen components to the infection.
(Item A51) The method of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item A52) The method of any one of the preceding items, wherein the physical condition comprises BCG vaccination history, tuberculosis infection history, or antigen responsiveness to Mycobacterium tuberculosis, and the antigen component or combination of antigen components comprises a human Mycobacterium tuberculosis hot water extract.
(Item A53) The method of any one of the preceding items, wherein the physical condition comprises influenza vaccination history, influenza infection history, or antigen responsiveness to an influenza vaccine, and the antigen component or combination of antigen components comprises an influenza virus.
(Item A54) The method of any one of the preceding items, wherein the administration comprises subcutaneous administration or intradermal administration.
(Item A55) The method of any one of the preceding items, wherein the subject is in a state before onset of cancer, after a cancer treatment, an early stage of onset of cancer, or a precancerous condition.
(Item A56) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item A57) The method of any one of the preceding items, wherein the subject exhibits immunological resistance.
(Item A58) The method of any one of the preceding items, wherein step ii) comprises measuring induction of cells producing IFN-γ, IL-2, TNF-α, or a plurality of the cytokines concurrently.
(Item A59) The method of any one of the preceding items, wherein the antigen responsiveness profile is obtained by performing companion diagnosis in advance based on anamnesis and vaccination history.
(Item A60) The method of any one of the preceding items, wherein the past physical condition and the antigen component or combination of antigen components are tuberculosis infection history and a human Mycobacterium tuberculosis hot water extract.
(Item A61) The method of any one of the preceding items, wherein the past physical condition and the antigen component or combination of antigen components are influenza infection history and an influenza virus.
(Item A62) The method of any one of the preceding items, wherein the past physical condition and the antigen component of combination of antigen components are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item A63) The method of any one of the preceding items, wherein the subject is a subject who has BCG vaccination history or tuberculosis infection history, or is confirmed to have antigen responsiveness,
   wherein the Mycobacterium tuberculosis extract is prophylactically administered before onset or administered in an early stage of onset of cancer, and an extract from Mycobacterium tuberculosis is subcutaneously or intradermally administered by a conventional method in an early stage of onset of cancer or a precancerous condition.
(Item A64) The method of any one of the preceding items, wherein the subject is a subject who has influenza vaccination history or influenza infection history, or is confirmed to have antigen responsiveness,
   wherein the influenza vaccine is prophylactically administered before onset, administered to prevent recurrence after therapy, or administered in an early stage of onset of cancer, and an influenza vaccine is subcutaneously or intradermally administered in an early stage of onset of cancer or a precancerous condition.
(Item A65) A method of preventing or treating cancer immunity based on any one of the preceding items, comprising revaccinating the antigen component or combination of antigen components.
(Item A66) A method of preventing or treating cancer of a subject with a non-tumor component, wherein the component is an antigen or extract identified by interview and/or identified by reference to anamnesis or vaccination history of the subject, wherein the subject is an individual with an infection history or vaccination history described in any one of the preceding items, wherein the method comprises administering an effective amount of the component to prevent recurrence after therapy, administered prophylactically before onset, or administered in an early stage of onset of cancer to the subject, and optionally comprises administering an effective amount of the component in an early stage of onset of cancer or a precancerous condition.
(Item A67) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item A68) The method of any one of the preceding items, wherein the subject is a patient exhibiting immunological resistance.
(Item A69) The method of any one of the preceding items, wherein the identification of responsiveness is characterized by infection history, vaccination history, and measuring induction of cells producing IFN-γ, IL-2, TNF-α, or a plurality of the cytokines concurrently using peripheral blood.
(Item A70) The method of any one of the preceding items, wherein the Mycobacterium tuberculosis extract is a hot water extract of human Mycobacterium tuberculosis or other extract from Mycobacterium tuberculosis (highly safe extract).
(Item A71) The method of any one of the preceding items, wherein the influenza virus is a human influenza virus or other extract from an influenza virus (highly safe extract).
(Item A72) The method of any one of the preceding items, wherein the antigen component is a protein.
(Item A73) A vaccine formulation comprising the antigen of any one of the preceding items and an adjuvant base.
(Item A74) The vaccine formulation of any one of the preceding items, wherein the adjuvant base comprises a substance promoting a Th1 immune response.
(Item A75) The vaccine formulation of any one of the preceding items, wherein the vaccine formulation is used for personalized medicine.
(Item A76) A method for use in treating or preventing a disease, disorder, or condition associated with an immunological abnormality of a subject, comprising subcutaneously or intratumorally administering an effective amount an antigen component that is specific in the subject to a component that is different from a causative agent of the disease, disorder, or condition once a day (first week) and once a week (second week and thereafter).
(Item A77) The method of any one of the preceding items, wherein the antigen component is contained at about 0.001 µg of more per unit formulation.
(Item A78) A method of treating or preventing cancer or tumor in a subject, comprising:
   a) identifying a non-tumor antigen specific to the subject based on an antigen responsiveness profile;
   b) identifying whether the subject has immunological memory of the non-tumor antigen to identify a subject having the immunological memory; and
   c) administering an effective amount of the non-tumor antigen to the subject identified as having the immunological memory.
(Item A79) The method of any one of the preceding items, wherein the antigen responsiveness profile comprises vaccination history and/or infection history.
(Item A80) The method of any one of the preceding items, wherein the identifying a subject having the immunological memory stimulates peripheral blood mononuclear cells (PBMC) isolated from the subject or infiltrating immune cells isolated from a tumor mass with the non-tumor antigen, measuring cytokine production, and identifying a subject with an amount of cytokine production increased a predetermined factor compared to the amount before stimulation as a subject having the immunological memory.
(Item A81) The method of any one of the preceding items, wherein the non-tumor antigen is administered once a day (first week) and once a week (second week and thereafter).
(Item A82) The method of any one of the preceding items, wherein the non-tumor antigen is administered at about 0.001 µg/dose to about 1 mg/dose.
(Item A83) A method for use in treating or preventing a disease, disorder, or condition associated with an immunological abnormality of a subject, comprising administering mHSP10 and/or MTB12 and/or lipoprotein LpqH.
(Item A84) The method of any one of the preceding items, comprising administering a plurality of agents comprising the antigen component.
(Item A85) The method of any one of the preceding items, wherein each component of the plurality of agents is administered as separate compositions.
(Item B1) A non-target antigen component for activating a regulatory T cell (Treg) having immunological memory of the non-target antigen component, which is suppressed in a subject, against a target.
(Item B2) The antigen component of any one of the preceding items, wherein the activation of Treg imparts an ability to kill the target or an immunostimulatory action against the target.
(Item B3) A non-tumor antigen component for activating a regulatory T cell (Treg) having immunological memory of the non-tumor antigen component, which is suppressed in a subject.
(Item B4) The antigen component of any one of the preceding items, wherein the activation of Treg imparts an ability to kill tumor or an immunostimulatory action against tumor.
(Item B5) The antigen component of any one of the preceding items, wherein the Treg is a memory T cell.
(Item B6) The antigen component of any one of the preceding items, wherein the Treg is CD4 positive.
(Item B7) The antigen component of any one of the preceding items, wherein the antigen component comprises a protein.
(Item B8) The antigen component of any one of the preceding items, wherein the antigen component comprises an antigen selected from the group consisting of a pathogen of an infection or a part thereof, an antigen associated with anamnesis, and an antigen associated with vaccination history.
(Item B9) The antigen component of any one of the preceding items, wherein the antigen component comprises a human Mycobacterium tuberculosis hot water extract or an influenza virus antigen.
(Item B9A) The antigen component of any one of the preceding items, further comprising one or more features of any one or more of the preceding items and the following items.
(Item B9B) An extract comprising the antigen component of any one of the preceding items.
(Item B10) An antigen component characterized in that the antigen component is used in a method comprising checking whether the antigen component has immunological memory of Treg in the subject, and administering the antigen component to the subject if the subject has immunological memory for the antigen component.
(Item B11) An antigen component for use in treating or preventing a disease, disorder, or condition associated with an immunological abnormality of a subject, wherein the antigen component is specific in the subject to a component that is different from a causative agent of the disease, disorder, or condition.
(Item B12) The antigen component of any one of the preceding items, wherein the disease, disorder, or condition comprises cancer, and wherein, preferably, the antigen component is a non-tumor antigen component.
(Item B13) The antigen component of any one of the preceding items, wherein the antigen component is specific to a memory T-cell of the subject.
(Item B14) The antigen component of any one of the preceding items, wherein the memory T cell is a memory regulatory T cell (IL-2 producing).
(Item B15) The antigen component of any one of the preceding items, wherein the antigen component has an immunostimulatory action.
(Item B16) The antigen component of any one of the preceding items, wherein the antigen component antigen-dependently acts on memory CD4 positive T cells.
(Item B17) The antigen component of any one of the preceding items, wherein the antigen component has activity to bias a ratio of presence between Foxp3 positive Treg cells and IFN-γ producing T cells.
(Item B18) The antigen component of any one of the preceding items, wherein the bias is an increase in IFN-γ producing T cells compared to Foxp3 positive Treg cells.
(Item B19) The antigen component of any one of the preceding items, wherein the antigen component has activity to bias a ratio of presence between Foxp3 positive Treg cells and type 1 helper T cells.
(Item B20) The antigen component of any one of the preceding items, wherein the IFN-γ producing T cells comprise type 1 helper T cells.
(Item B21) The antigen component of any one of the preceding items, wherein the antigen component has activity to bias a ratio of presence of Th1 cells.
(Item B22) The antigen component of any one of the preceding items, wherein the IFN-γ producing T cells are T-bet positive Th1 cells.
(Item B23) The antigen component of any one of the preceding items, wherein the antigen component that is specific has a capability to enhance at least one selected from the group consisting of IFN-γ producing capability, IL-2 producing capability, and TNF-α producing capability in a sample from the subject.
(Item B24) The antigen component of any one of the preceding items, wherein the antigen component is a protein.
(Item B25) A biomarker for determining whether a non-tumor antigen component has anticancer action on a subject, the biomarker comprising at least one selected from the group consisting of whether the antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item B26) A composition or a kit comprising an agent or means for detecting a biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item B27) The antigen component of any one of the preceding items, wherein the (non-tumor) antigen component comprises an antigen associated with anamnesis and vaccination history.
(Item B28) The antigen component of any one of the preceding items, wherein the subject is a subject with a history of an infection, and the antigen component comprises an antigen to the infection.
(Item B29) The composition of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item B30) The antigen component of any one of the preceding items, wherein the subject is a subject with BCG vaccination history, tuberculosis infection history, or antigen responsiveness to Mycobacterium tuberculosis, and the antigen component comprises a human Mycobacterium tuberculosis hot water extract.
(Item B31) The antigen component of any one of the preceding items, wherein the subject is a subject with influenza vaccination history, influenza infection history, or antigen responsiveness to an influenza virus, and the antigen component comprises an influenza virus.
(Item B32) The antigen component of any one of the preceding items, wherein the disease, disorder, or condition comprises melanoma.
(Item B33) The antigen component of any one of the preceding items, wherein the antigen component comprises a protein, a part thereof, or a peptide.
(Item B34) The antigen component of any one of the preceding items, wherein the antigen component comprises a component that can elicit an immune response via CD4 positive T cells.
(Item B35) The antigen component of any one of the preceding items, wherein the subject is checked as to whether the subject can elicit an antitumor immune response via CD4 positive T cells, and if the subject can elicit an antitumor immune response via CD4 positive T cells, the antigen component is administered.
(Item B36) An antigen component for use in treating or preventing a disease, disorder, or condition associated with an immunological abnormality of a subject, wherein the antigen component is an antigen component that is specific in the subject to a component that is different from a causative agent of the disease, disorder, or condition, wherein
   the disease, disorder, or condition comprises melanoma,
   the antigen component is a protein, a part thereof, or a peptide, and can elicit an immune response via CD4 positive T cells, and
   the cancer comprises cancer that is treatable and preventable with an immune response via CD4 positive T cells,
   wherein the subject is checked as to whether the subject can elicit an antitumor immune response via CD4 positive T cells, and if the subject can elicit an antitumor immune response via CD4 positive T cells, the antigen component is administered.
(Item B37) An antigen component for treating or preventing cancer or tumor in a subject, the antigen component comprising a non-tumor antigen component, wherein the non-tumor antigen component activates a regulatory T cell (Treg) having immunological memory of the non-tumor antigen component, which is suppressed in a subject, and wherein the Treg has an effect of promoting regulatory activity or antitumor immunological action on cancer or tumor.
(Item B38) A method of manufacturing or otherwise providing a composition for use in preventing or treating cancer of a subject, the method comprising:
   A) identifying a non-tumor antigen specific to the subject;
   B) identifying whether a subject has immunological memory of the non-tumor antigen, and selecting a non-tumor antigen for which the subject has the immunological memory; and
   C) manufacturing or otherwise providing the selected non-tumor antigen.
(Item B39) The method of any one of the preceding items, having one or more features in any one of the preceding items in B).
(Item B40) A method of determining whether a non-tumor antigen of a subject can prevent or treat cancer of the subject, the method comprising:
   B) identifying whether the subject has immunological memory of the non-tumor antigen, and identifying that cancer of the subject can be prevented or treated if the subject has the immunological memory.
(Item B41) The method of any one of the preceding items, having one or more features in any one of the preceding items in B).
(Item B42) A method for use in preventing or treating a disease, disorder, or condition associated with an immunological abnormality, comprising:
   a) obtaining an antigen responsiveness profile of a subject;
   b) identifying an antigen component or a combination of antigen components from the antigen responsiveness profile, wherein the antigen component or combination of antigen components is identified based on whether the antigen component or combination of antigen components has shown or shows to present immune response to the subject; and
   c) administering to the subject the antigen component or combination of antigen components identified in step b) at a sufficient amount to elicit an immune response in the subject.
(Item B43) The method of any one of the preceding items, wherein the disease, disorder, or condition comprises cancer.
(Item B44) The method of any one of the preceding items, wherein the obtaining an antigen responsiveness profile comprises checking a past physical condition of a subject, checking whether one or more of candidate antigens has responsiveness in a sample from the subject, or both.
(Item B45) The method of any one of the preceding items, wherein the obtaining an antigen responsiveness profile comprises identifying an antigen component or a combination of antigen components that elicit an immune response via CD4 positive T cells.
(Item B46) The method of any one of the preceding items, wherein
   i) the antigen responsiveness profile comprises at least one selected from the group consisting of interview, anamnesis or vaccination history based on a Maternal and Child Health Handbook, an equivalent thereof, or the like, and a combination thereof, and/or
   ii) the checking whether one or more of candidate antigens has responsiveness comprises collecting a bodily fluid (e.g., blood) from the subject and separating peripheral blood cells, and then measuring whether the peripheral blood cells produce a cytokine in response to an antigen associated with the antigen profile, and other biomarkers.
(Item B47) The method of any one of the preceding items, further comprising periodically testing responsiveness of the antigen and confirming that responsiveness is maintained.
(Item B48) The method of any one of the preceding items, wherein a) and b) are performed with the following steps:
   i) obtaining a past physical condition of a subject;
   ii) collecting blood from the subject and separating peripheral blood cells, and then measuring whether the peripheral blood cells produce a cytokine in response to an antigen corresponding to the physical condition, and other biomarkers; and
   iii) identifying a suitable antigen component or a combination of antigen components from a result of ii).
(Item B49) The method of any one of the preceding items, wherein the past physical condition comprises anamnesis and vaccination history.
(Item B50) The method of any one of the preceding items, wherein the physical condition comprises history of infection, and the cancer vaccine comprises an antigen component or a combination of antigen components to the infection.
(Item B51) The method of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item B52) The method of any one of the preceding items, wherein the physical condition comprises BCG vaccination history, tuberculosis infection history, or antigen responsiveness to Mycobacterium tuberculosis, and the antigen component or combination of antigen components comprises a human Mycobacterium tuberculosis hot water extract.
(Item B53) The method of any one of the preceding items, wherein the physical condition comprises influenza vaccination history, influenza infection history, or antigen responsiveness to an influenza virus, and the antigen component or combination of antigen components comprises an influenza virus.
(Item B54) The method of any one of the preceding items, wherein the administration comprises subcutaneous administration or intradermal administration.
(Item B55) The method of any one of the preceding items, wherein the subject is in a state before onset of cancer, after a cancer treatment, an early stage of onset of cancer, or a precancerous condition.
(Item B56) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item B57) The method of any one of the preceding items, wherein the subject exhibits immunological resistance.
(Item B58) The method of any one of the preceding items, wherein step ii) comprises measuring induction of cells producing IFN-γ, IL-2, TNF-α, or a plurality of the cytokines concurrently.
(Item B59) The method of any one of the preceding items, wherein the antigen responsiveness profile is obtained by performing companion diagnosis in advance based on anamnesis and vaccination history.
(Item B60) The method of any one of the preceding items, wherein the past physical condition and the antigen component or combination of antigen components are tuberculosis infection history and a human Mycobacterium tuberculosis hot water extract.
(Item B61) The method of any one of the preceding items, wherein the past physical condition and the antigen component or combination of antigen components are influenza infection history and an influenza virus.
(Item B62) The method of any one of the preceding items, wherein the past physical condition and the antigen component or combination of antigen components are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item B63) The method of any one of the preceding items, wherein the subject is a subject who has BCG vaccination history or tuberculosis infection history, or is confirmed to have antigen responsiveness,
   wherein the Mycobacterium tuberculosis extract is prophylactically administered before onset or administered in an early stage of onset of cancer, and an extract from Mycobacterium tuberculosis is subcutaneously or intradermally administered by a conventional method in an early stage of onset of cancer or a precancerous condition.
(Item B64) The method of any one of the preceding items, wherein the subject is a subject who has influenza vaccination history or influenza infection history, or is confirmed to have antigen responsiveness,
   wherein the influenza vaccine is prophylactically administered before onset, administered to prevent recurrence after therapy, or administered in an early stage of onset of cancer, and an influenza vaccine is subcutaneously or intradermally administered in an early stage of onset of cancer or a precancerous condition.
(Item B65) A method of preventing or treating cancer immunity based on any one of the preceding items, comprising revaccinating the antigen component or combination of antigen components.
(Item B66) A method of preventing or treating cancer of a subject with a non-tumor component, wherein the component is an antigen or extract identified by interview and/or identified by reference to anamnesis or vaccination history of the subject, wherein the subject is an individual with an infection history or vaccination history described in any one of the preceding items, wherein the component is administered to prevent recurrence after therapy, administered prophylactically before onset, or administered in an early stage of onset of cancer to the subject, and the component is optionally administered in an early stage of onset of cancer or a precancerous condition.
(Item B67) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item B68) The method of any one of the preceding items, wherein the subject is a patient exhibiting immunological resistance.
(Item B69) The method of any one of the preceding items, wherein the identification of responsiveness is characterized by infection history, vaccination history, and measuring induction of cells producing IFN-γ, IL-2, TNF-α, or a plurality of the cytokines concurrently using peripheral blood.
(Item B70) The method of any one of the preceding items, wherein the Mycobacterium tuberculosis extract is a hot water extract of human Mycobacterium tuberculosis or other extract from Mycobacterium tuberculosis (highly safe extract).
(Item B71) The method of any one of the preceding items, wherein the influenza virus is a human influenza virus or other extract from an influenza virus (highly safe extract).
(Item B72) The method of any one of the preceding items, wherein the antigen component is a protein.
(Item B73) A vaccine formulation comprising the antigen of any one of the preceding items and an adjuvant base.
(Item B74) The vaccine formulation of any one of the preceding items, wherein the adjuvant base comprises a substance promoting a Th1 immune response.
(Item B75) The vaccine formulation of any one of the preceding items, wherein the vaccine formulation is used for personalized medicine.
(Item B76) An antigen component for use in treating or preventing a disease, disorder, or condition associated with an immunological abnormality of a subject, wherein the antigen component is specific in the subject to a component that is different from a causative agent of the disease, disorder, or condition, and is subcutaneously or intratumorally administered once a day (first week) and once a week (second week and thereafter).
(Item B77) The antigen component of any one of the preceding items, wherein the antigen component is contained at about 0.001 *µ* g of more per unit formulation.
(Item B78) A non-tumor antigen component for use in a method of treating or preventing cancer or tumor in a subject, the method comprising:
   a) identifying a non-tumor antigen specific to the subject based on an antigen responsiveness profile;
   b) identifying whether the subject has immunological memory of the non-tumor antigen to identify a subject having the immunological memory; and
   c) administering the non-tumor antigen to the subject identified as having the immunological memory.
(Item B79) The antigen component of any one of the preceding items, wherein the antigen responsiveness profile comprises vaccination history and/or infection history.
(Item B80) The antigen component of any one of the preceding items, wherein the identifying a subject having the immunological memory stimulates peripheral blood mononuclear cells (PBMC) isolated from the subject or infiltrating immune cells isolated from a tumor mass with the non-tumor antigen, measuring cytokine production, and identifying a subject with an amount of cytokine production increased a predetermined factor compared to the amount before stimulation as a subject having the immunological memory.
(Item B81) The antigen component of any one of the preceding items, wherein the non-tumor antigen is administered once a day (first week) and once a week (second week and thereafter).
(Item B82) The antigen component of any one of the preceding items, wherein the non-tumor antigen is administered at about 0.001 pg/dose to about 1 mg/dose.
(Item B83) mHSP10 and/or MTB12 and/or lipoprotein LpqH for use in treating or preventing a disease, disorder, or condition associated with an immunological abnormality of a subject.
(Item B84) The composition, antigen component, kit, biomarker, vaccine formulation, or method of any one of the preceding items, characterized by administering a plurality of agents comprising the antigen component.
(Item B85) The composition, antigen component, kit, biomarker, vaccine formulation, or method of any one of the preceding items, characterized in that each component of the plurality of agents is administered as separate compositions.
(Item C1) Use of a non-target antigen component in the manufacture of a composition for use in activating a regulatory T cell (Treg) having immunological memory of the non-target antigen component, which is suppressed in a subject, against a target.
(Item C2) The use of any one of the preceding items, wherein the activation of Treg imparts an ability to kill the target or an immunostimulatory action against the target.
(Item C3) Use of a non-tumor antigen component in the manufacture of a composition for use in activating a regulatory T cell (Treg) having immunological memory of the non-tumor antigen component, which is suppressed in a subject.
(Item C4) The use of any one of the preceding items, wherein the activation of Treg imparts an ability to kill tumor or an immunostimulatory action against tumor.
(Item C5) The use of any one of the preceding items, wherein the Treg is a memory T cell.
(Item C6) The use of any one of the preceding items, wherein the Treg is CD4 positive.
(Item C7) The use of any one of the preceding items, wherein the antigen component comprises a protein.
(Item C8) The use of any one of the preceding items, wherein the antigen component comprises an antigen selected from the group consisting of a pathogen of an infection or a part thereof, an antigen associated with anamnesis, and an antigen associated with vaccination history.
(Item C9) The use of any one of the preceding items, wherein the antigen component comprises a human Mycobacterium tuberculosis hot water extract or an influenza virus antigen.
(Item C9A) The use of any one of the preceding items, further comprising one or more features of any one or more of the preceding items and the following items.
(Item C10) Use of an antigen component in the manufacture of a composition used in a method comprising checking whether the antigen component has immunological memory of Treg in the subject, and administering the antigen component to the subject if the subject has immunological memory of the antigen component.
(Item C11) Use of an antigen component in the manufacture of a composition for use in treating or preventing a disease, disorder, or condition associated with an immunological abnormality of a subject, wherein the antigen component is an antigen component that is specific in the subject to a component that is different from a causative agent of the disease, disorder, or condition.
(Item C12) The use of any one of the preceding items, wherein the disease, disorder, or condition comprises cancer, and wherein, preferably, the antigen component is a non-tumor antigen component.
(Item C13) The use of any one of the preceding items, wherein the antigen component is specific to a memory T-cell of the subject.
(Item C14) The use of any one of the preceding items, wherein the memory T cell is a memory regulatory T cell (IL-2 producing).
(Item C15) The use of any one of the preceding items, wherein the antigen component has an immunostimulatory action.
(Item C16) The use of any one of the preceding items, wherein the antigen component antigen-dependently acts on memory CD4 positive T cells.
(Item C17) The use of any one of the preceding items, wherein the antigen component has activity to bias a ratio of presence between Foxp3 positive Treg cells and IFN-γ producing T cells.
(Item C18) The use of any one of the preceding items, wherein the bias is an increase in IFN-γ producing T cells compared to Foxp3 positive Treg cells.
(Item C19) The use of any one of the preceding items, wherein the antigen component has activity to bias a ratio of presence between Foxp3 positive Treg cells and type 1 helper T cells.
(Item C20) The use of any one of the preceding items, wherein the IFN-γ producing T cells comprise type 1 helper T cells.
(Item C21) The use of any one of the preceding items, wherein the antigen component has activity to bias a ratio of presence of Th1 cells.
(Item C22) The use of any one of the preceding items, wherein the IFN-γ producing T cells are T-bet positive Th1 cells.
(Item C23) The use of any one of the preceding items, wherein the antigen component that is specific has a capability to enhance at least one selected from the group consisting of IFN-γ producing capability, IL-2 producing capability, and TNF- α producing capability in a sample from the subject.
(Item C24) The use of any one of the preceding items, wherein the antigen component is a protein.
(Item C25) A biomarker for determining whether a non-tumor antigen component has anticancer action on a subject, the biomarker comprising at least one selected from the group consisting of whether the antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item C26) A composition or a kit comprising an agent or means for detecting a biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item C27) The use of any one of the preceding items, wherein the (non-tumor) antigen component comprises an antigen associated with anamnesis and vaccination history.
(Item C28) The use of any one of the preceding items, wherein the subject is a subject with a history of an infection, and the antigen component comprises an antigen to the infection.
(Item C29) The use of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item C30) The use of any one of the preceding items, wherein the subject is a subject with BCG vaccination history, tuberculosis infection history, or antigen responsiveness to Mycobacterium tuberculosis, and the antigen component comprises a human Mycobacterium tuberculosis hot water extract.
(Item C31) The use of any one of the preceding items, wherein the subject is a subject with influenza vaccination history, influenza infection history, or antigen responsiveness to an influenza virus, and the antigen component comprises an influenza virus.
(Item C32) The use of any one of the preceding items, wherein the disease, disorder, or condition comprises melanoma.
(Item C33) The use of any one of the preceding items, wherein the antigen component comprises a protein, a part thereof, or a peptide.
(Item C34) The use of any one of the preceding items, wherein the antigen component comprises a component that can elicit an immune response via CD4 positive T cells.
(Item C35) The use of any one of the preceding items, wherein the subject is checked as to whether the subject can elicit an antitumor immune response via CD4 positive T cells, and if the subject can elicit an antitumor immune response via CD4 positive T cells, the composition is administered.
(Item C36) Use of an antigen component in the manufacture of a composition for use in treating or preventing a disease, disorder, or condition associated with an immunological abnormality of a subject, wherein the antigen component is an antigen component that is specific in the subject to a component that is different from a causative agent of the disease, disorder, or condition, wherein
   the disease, disorder, or condition comprises melanoma,
   the antigen component is a protein, a part thereof, or a peptide, and can elicit an immune response via CD4 positive T cells, and
   the cancer comprises cancer that is treatable and preventable with an immune response via CD4 positive T cells,
   wherein the subject is checked as to whether the subject can elicit an antitumor immune response via CD4 positive T cells, and if the subject can elicit an antitumor immune response via CD4 positive T cells, the composition is administered.
(Item C37) Use of a non-tumor antigen component in the manufacture of a composition for use in treating or preventing cancer or tumor in a subject, wherein the non-tumor antigen component activates a regulatory T cell (Treg) having immunological memory of the non-tumor antigen component, which is suppressed in a subject, and wherein the Treg has an effect of promoting regulatory activity or antitumor immunological action on cancer or tumor.
(Item C38) A method of manufacturing or otherwise providing a composition for use in preventing or treating cancer of a subject, the method comprising:
   A) identifying a non-tumor antigen specific to the subject;
   B) identifying whether a subject has immunological memory of the non-tumor antigen, and selecting a non-tumor antigen for which the subject has the immunological memory; and
   C) manufacturing or otherwise providing the selected non-tumor antigen.
(Item C39) The method of any one of the preceding items, having one or more features in any one of the preceding items in B).
(Item C40) A method of determining whether a non-tumor antigen of a subject can prevent or treat cancer of the subject, the method comprising:
   B) identifying whether the subject has immunological memory of the non-tumor antigen, and identifying that cancer of the subject can be prevented or treated if the subject has the immunological memory.
(Item C41) The method of any one of the preceding items, having one or more features in any one of the preceding items in B).
(Item C42) A method for use in preventing or treating a disease, disorder, or condition associated with an immunological abnormality, comprising:
   a) obtaining an antigen responsiveness profile of a subject;
   b) identifying an antigen component or a combination of antigen components from the antigen responsiveness profile, wherein the antigen component or combination of antigen components is identified based on whether the antigen component or combination of antigen components has shown or shows to present immune response to the subject; and
   c) administering to the subject the antigen component or combination of antigen components identified in step b) at a sufficient amount to elicit an immune response in the subject.
(Item C43) The method of any one of the preceding items, wherein the disease, disorder, or condition comprises cancer.
(Item C44) The method of any one of the preceding items, wherein the obtaining an antigen responsiveness profile comprises checking a past physical condition of a subject, checking whether one or more of candidate antigens has responsiveness in a sample from the subject, or both.
(Item C45) The method of any one of the preceding items, wherein the obtaining an antigen responsiveness profile comprises identifying an antigen component or a combination of antigen components that elicits an immune response via CD4 positive T cells.
(Item C46) The method of any one of the preceding items, wherein
   i) the antigen responsiveness profile comprises at least one selected from the group consisting of interview, anamnesis or vaccination history based on a Maternal and Child Health Handbook, an equivalent thereof, or the like, and a combination thereof, and/or
   ii) the checking whether one or more of candidate antigens has responsiveness comprises collecting a bodily fluid (e.g., blood) from the subject and separating peripheral blood cells, and then measuring whether the peripheral blood cells produce a cytokine in response to an antigen associated with the antigen profile, and other biomarkers.
(Item C47) The method of any one of the preceding items, further comprising periodically testing responsiveness of the antigen and confirming that responsiveness is maintained.
(Item C48) The method of any one of the preceding items, wherein a) and b) are performed with the following steps:
   i) obtaining a past physical condition of a subject;
   ii) collecting blood from the subject and separating peripheral blood cells, and then measuring whether the peripheral blood cells produce a cytokine in response to an antigen corresponding to the physical condition, and other biomarkers; and
   iii) identifying a suitable antigen component or a combination of antigen components from a result of ii).
(Item C49) The method of any one of the preceding items, wherein the past physical condition comprises anamnesis and vaccination history.
(Item C50) The method of any one of the preceding items, wherein the physical condition comprises history of infection, and the cancer vaccine comprises an antigen component or a combination of antigen components to the infection.
(Item C51) The method of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item C52) The method of any one of the preceding items, wherein the physical condition comprises BCG vaccination history, tuberculosis infection history, or antigen responsiveness to Mycobacterium tuberculosis, and the antigen component or combination of antigen components comprises a human Mycobacterium tuberculosis hot water extract.
(Item C53) The method of any one of the preceding items, wherein the physical condition comprises influenza vaccination history, influenza infection history, or antigen responsiveness to an influenza virus, and the antigen component or combination of antigen components comprises an influenza virus.
(Item C54) The method of any one of the preceding items, wherein the administration comprises subcutaneous administration or intradermal administration.
(Item C55) The method of any one of the preceding items, wherein the subject is in a state before onset of cancer, after a cancer treatment, an early stage of onset of cancer, or a precancerous condition.
(Item C56) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item C57) The method of any one of the preceding items, wherein the subject exhibits immunological resistance.
(Item C58) The method of any one of the preceding items, wherein step ii) comprises measuring induction of cells producing IFN-γ, IL-2, TNF-α, or a plurality of the cytokines concurrently.
(Item C59) The method of any one of the preceding items, wherein the antigen responsiveness profile is obtained by performing companion diagnosis in advance based on anamnesis and vaccination history.
(Item C60) The method of any one of the preceding items, wherein the past physical condition and the antigen component or combination of antigen components are tuberculosis infection history and a human Mycobacterium tuberculosis hot water extract.
(Item C61) The method of any one of the preceding items, wherein the past physical condition and the antigen component or combination of antigen components are influenza infection history and an influenza virus.
(Item C62) The method of any one of the preceding items, wherein the past physical condition and the antigen component or combination of antigen components are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.
(Item C63) The method of any one of the preceding items, wherein the subject is a subject who has BCG vaccination history or tuberculosis infection history, or is confirmed to have antigen responsiveness,
   wherein the Mycobacterium tuberculosis extract is prophylactically administered before onset or administered in an early stage of onset of cancer, and an extract from Mycobacterium tuberculosis is subcutaneously or intradermally administered by a conventional method in an early stage of onset of cancer or a precancerous condition.
(Item C64) The method of any one of the preceding items, wherein the subject is a subject who has influenza vaccination history or influenza infection history, or is confirmed to have antigen responsiveness,
   wherein the influenza vaccine is prophylactically administered before onset, administered to prevent recurrence after therapy, or administered in an early stage of onset of cancer, and an influenza vaccine is subcutaneously or intradermally administered in an early stage of onset of cancer or a precancerous condition.
(Item C65) A method of preventing or treating cancer immunity based on any one of the preceding items, comprising revaccinating the antigen component or combination of antigen components.
(Item C66) A method of preventing or treating cancer of a subject with a non-tumor component, wherein the component is an antigen or extract identified by interview and/or identified by reference to anamnesis or vaccination history of the subject, wherein the subject is an individual with an infection history or vaccination history described in any one of the preceding items, wherein the component is administered to prevent recurrence after therapy, administered prophylactically before onset, or administered in an early stage of onset of cancer to the subject, and the component is optionally administered in an early stage of onset of cancer or a precancerous condition.
(Item C67) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item C68) The method of any one of the preceding items, wherein the subject is a patient exhibiting immunological resistance.
(Item C69) The method of any one of the preceding items, wherein the identification of responsiveness is characterized by infection history, vaccination history, and measuring induction of cells producing IFN-γ, IL-2, TNF-α, or a plurality of the cytokines concurrently using peripheral blood.
(Item C70) The method of any one of the preceding items, wherein the Mycobacterium tuberculosis extract is a hot water extract of human Mycobacterium tuberculosis or other extract from Mycobacterium tuberculosis (highly safe extract).
(Item C71) The method of any one of the preceding items, wherein the influenza virus is a human influenza virus or other extract from an influenza virus (highly safe extract).
(Item C72) The method of any one of the preceding items, wherein the antigen component is a protein.
(Item C73) A vaccine formulation comprising the antigen of any one of the preceding items and an adjuvant base.
(Item C74) The vaccine formulation of any one of the preceding items, wherein the adjuvant base comprises a substance promoting a Th1 immune response.
(Item C75) The vaccine formulation of any one of the preceding items, wherein the vaccine formulation is used for personalized medicine.
(Item C76) Use of an antigen component that is specific in a subject to a component that is different from a causative agent of a disease, disorder, or condition in the manufacture of a composition for use in treating or preventing the disease, disorder, or condition associated with an immunological abnormality of the subject, wherein the composition is subcutaneously or intratumorally administered once a day (first week) and once a week (second week and thereafter).
(Item C77) The use of any one of the preceding items, wherein the antigen component is contained at about 0.001 µg or more per unit formulation.
(Item C78) Use of a non-tumor antigen component in the manufacture of a composition for use in a method of treating or preventing cancer or tumor in a subject, the method comprising:
   a) identifying a non-tumor antigen specific to the subject based on an antigen responsiveness profile;
   b) identifying whether the subject has immunological memory of the non-tumor antigen to identify a subject having the immunological memory; and
   c) administering the non-tumor antigen to the subject identified as having the immunological memory.
(Item C79) The use of any one of the preceding items, wherein the antigen responsiveness profile comprises vaccination history and/or infection history.
(Item C80) The use of any one of the preceding items, wherein the identifying a subject having the immunological memory stimulates peripheral blood mononuclear cells (PBMC) isolated from the subject or infiltrating immune cells isolated from a tumor mass with the non-tumor antigen, measuring cytokine production, and identifying a subject with an amount of cytokine production increased a predetermined factor compared to the amount before stimulation as a subject having the immunological memory. (Item C81) The use of any one of the preceding items, wherein the non-tumor antigen is administered once a day (first week) and once a week (second week and thereafter). (Item C82) The use of any one of the preceding items, wherein the non-tumor antigen is administered at about 0.001 µg/dose to about 1 mg/dose.
(Item C83) Use of mHSP10 and/or MTB12 and/or lipoprotein LpqH in the manufacture of a composition for use in treating or preventing a disease, disorder, or condition associated with an immunological abnormality of a subject. (Item C84) The use or method of any one of the preceding items, wherein the antigen component is administered with a plurality of agents.
(Item C85) The use or method of any one of the preceding items, wherein each component of the plurality of agents is provided as separate compositions.
(Item X1) A composition for use in treating or preventing a disease, disorder, or condition associated with an immunological abnormality of a subject, the composition comprising an antigen component that is specific in the subject (or for which the subject has immunological memory) to a component that is different from a causative agent of the disease, disorder, or condition.
(Item X2) The composition of any one of the preceding items, wherein the disease, disorder, or condition comprises cancer.
(Item X3) The composition of any one of the preceding items, wherein the non-tumor antigen component is specific to a memory T-cell of the subject.
(Item X4) The composition of any one of the preceding items, wherein the memory T cell is a memory regulatory T cell (IL-2 producing).
(Item X5) The composition of any one of the preceding items, wherein the non-tumor antigen component has an immunostimulatory action.
(Item X6) The composition of any one of the preceding items, wherein the non-tumor antigen component antigen-dependently acts on memory CD4 positive T cells.
(Item X7) The composition of any one of the preceding items, wherein the non-tumor antigen component has activity to bias a ratio of presence between Foxp3 positive Treg cells and IFN-γ producing T cells.
(Item X8) The composition of any one of the preceding items, wherein the bias is an increase in IFN-γ producing T cells compared to Foxp3 positive Treg cells.
(Item X9) The composition of any one of the preceding items, wherein the non-tumor antigen component has activity to bias a ratio of presence between Foxp3 positive Treg cells and type 1 helper T cells.
(Item X10) The composition of any one of the preceding items, wherein the IFN-γ producing T cells comprise type 1 helper T cells.
(Item X11) The composition of any one of the preceding items, wherein the non-tumor antigen component has activity to bias a ratio of presence of Th1 cells.
(Item X12) The composition of any one of the preceding items, wherein the IFN-γ producing T cells are T-bet positive Th1 cells.
(Item X13) The composition of any one of the preceding items, wherein the non-tumor antigen component that is specific to the subject (or for which the subject has immunological memory) has a capability to enhance at least one selected from the group consisting of IFN-γ producing capability, IL-2 producing capability, and TNF-α producing capability in a sample from the subject.
(Item X14) A biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item X15) A composition or a kit comprising an agent or means for detecting a biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, and (iv) alters IL-2 producing capability.
(Item X16) The method, biomarker, composition, or kit of any one of the preceding items, wherein the (non-tumor) antigen component comprises an antigen associated with anamnesis and vaccination history.
(Item X17) The method, biomarker, composition, or kit of any one of the preceding items, wherein the subject is a subject with a history of an infection, and the antigen component comprises an antigen to the infection.
(Item X18) The method, biomarker, composition, or kit of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item X19) The method, biomarker, composition, or kit of any one of the preceding items, wherein the subject is a subject with BCG vaccination history, tuberculosis infection history, or antigen responsiveness to Mycobacterium tuberculosis, and the antigen component comprises a human Mycobacterium tuberculosis hot water extract.
(Item X20) A method of manufacturing a composition for use in preventing or treating cancer of a subject, the method comprising:
   A) identifying a non-tumor antigen specific to the subject;
   B) identifying whether a subject has immunological memory of the non-tumor antigen, and selecting a non-tumor antigen for which the subject has the immunological memory; and
   C) manufacturing the selected non-tumor antigen.
(Item X21) The method of item X20, having one or more features in any one of items X2 to X20 in B).
(Item X22) A method of determining whether a non-tumor antigen of a subject can prevent or treat cancer of the subject, the method comprising:
   B) identifying whether the subject has immunological memory of the non-tumor antigen, and identifying that cancer of the subject can be prevented or treated if the subject has the immunological memory.
(Item X23) The method of item X22, having one or more features in any of items X2 to X21 in B).
(Item X23A) A method of treating or preventing a disease, disorder, or condition associated with an immunological abnormality of a subject, comprising administering an effective amount of an antigen component that is specific in the subject (or for which the subject has immunological memory) to a component that is different from a causative agent of the disease, disorder, or condition to the subject. (Item X23AA) The method of item X23A, having one or more features in any of one of items X2 to X23.
(Item X23B) An antigen component for use in treating or preventing a disease, disorder, or condition associated with an immunological abnormality of a subject, wherein the antigen component is an antigen component that is specific in the subject (or for which the subject has immunological memory) to a component that is different from a causative agent of the disease, disorder, or condition.
(Item X23BB) The antigen component of item X23B, having one or more features in any one of items X2 to X23.
(Item X23C) Use of an antigen component that is specific in a subject (or for which the subject has immunological memory) to a component that is different from a causative agent of the disease, disorder, or condition in a method of manufacturing a medicament for use in treating or preventing a disease, disorder, or condition associated with an immunological abnormality of the subject.
(Item X23CC) Use of item X23C, having one or more features in any one of items X2 to X23.
(Item X24) A method for use in preventing or treating a disease, disorder, or condition associated with an immunological abnormality, comprising:
   a) obtaining an antigen responsiveness profile of a subject;
   b) identifying an antigen or a combination of antigens responsive to the subject based on the antigen responsiveness profile; and
   c) administering to the subject the component at a sufficient amount to elicit an immune response in the subject.
(Item X25) The method of any one of the preceding items, wherein the disease, disorder, or condition comprises cancer.
(Item X26) The method of item X24 or X25, wherein the obtaining an antigen responsiveness profile comprises checking a past physical condition of a subject, checking whether one or more of candidate antigens has responsiveness in a sample from the subject, or both.
(Item X27) The method of any one of the preceding items, wherein i) the antigen responsiveness profile comprises at least one selected from the group consisting of interview, anamnesis or vaccination history based on a Maternal and Child Health Handbook, an equivalent thereof, or the like, and a combination thereof, and/or
   ii) the checking whether one or more of candidate antigens has responsiveness comprises collecting a bodily fluid (e.g., blood) from the subject and separating peripheral blood cells, and then measuring whether the peripheral blood cells produce a cytokine (IL-2, IFN-γ, TNF-α, or the like) in response to an antigen associated with the antigen profile, and other biomarkers.
(Item X28) The method of any one of the preceding items, further comprising periodically testing responsiveness of the antigen and confirming that responsiveness is maintained.
(Item X29) The method of any one of the preceding items, wherein a) and b) are performed with the following steps:
   i) obtaining a past physical condition of a subject;
   ii) collecting blood from the subject and separating peripheral blood cells, and then measuring whether the peripheral blood cells produce a cytokine (IL-2, IFN-γ, TNF-α, or the like) in response to an antigen corresponding to the physical condition, and other biomarkers; and
   iii) identifying a suitable antigen or a combination thereof from a result of ii).
(Item X30) The method of any one of the preceding items, wherein the past physical condition comprises anamnesis and vaccination history.
(Item X31) The method of any one of the preceding items, wherein the physical condition comprises history of infection, and the cancer vaccine comprises an antigen to the infection.
(Item X32) The method of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item X33) The method of any one of the preceding items, wherein the physical condition comprises BCG vaccination history, tuberculosis infection history, or antigen responsiveness to Mycobacterium tuberculosis, and the antigen or a combination thereof comprises a human Mycobacterium tuberculosis hot water extract.
(Item X34) The method of any one of the preceding items, wherein the administration comprises subcutaneous administration or intradermal administration.
(Item X35) The method of any one of the preceding items, wherein the subject is in a state before onset of cancer, an early stage of onset of cancer, after a cancer treatment, or a precancerous condition.
(Item X36) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item X37) The method of any one of the preceding items, wherein the subject exhibits immunological resistance.
(Item X38) The method of any one of the preceding items, wherein step ii) comprises measuring induction of cells producing IFN-γ, IL-2, TNF-α, or a plurality of the cytokines concurrently.
(Item X39) The method of any one of the preceding items, wherein the antigen responsiveness profile is obtained by performing companion diagnosis in advance based on anamnesis and vaccination history.
(Item X40) The method of any one of the preceding items, wherein the past physical condition and the component are tuberculosis infection history and a human Mycobacterium tuberculosis hot water extract.
(Item X41) The method of any one of the preceding items, wherein the past physical condition and the component are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item X42) The method of any one of the preceding items, wherein the subject is a patient with BCG vaccination history or tuberculosis infection history, an individual with vaccination history or infection history with an personalized therapeutic method targeting a healthy individual confirmed to have antigen responsiveness, an individual with Mycobacterium tuberculosis infection history, or a subject with a BCG vaccination history,
   wherein the Mycobacterium tuberculosis extract is prophylactically administered before onset or administered in an early stage of onset of cancer, and an extract from Mycobacterium tuberculosis is subcutaneously or intradermally administered by a conventional method in an early stage of onset of cancer or a precancerous condition.
(Item X43) A method of preventing or treating cancer immunity based on any one of the preceding items, comprising revaccinating the antigen.
(Item X44) A method of preventing or treating cancer of a subject with a non-tumor component, wherein the component is an antigen or extract associated with anamnesis and vaccination history, wherein the subject is an individual with an infection history or vaccination history described in any one of the preceding items, wherein the component is administered prophylactically before onset, administered to prevent recurrence after therapy, or administered in an early stage of onset of cancer to the subject, and the component is optionally administered in an early stage of onset of cancer or a precancerous condition.
(Item X45) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item X46) The method of any one of the preceding items, wherein the subject is a patient exhibiting immunological resistance.
(Item X47) The method of any one of the preceding items, wherein the identification of responsiveness is characterized by infection history, vaccination history, and measuring induction of cells producing IFN-γ, IL-2, TNF-α, or a plurality of the cytokines concurrently using peripheral blood.
(Item X48) The method of any one of the preceding items, wherein the Mycobacterium tuberculosis extract is a hot water extract of human Mycobacterium tuberculosis or other extract from Mycobacterium tuberculosis (highly safe extract).
(Item X49) A vaccine formulation comprising the antigen of any one of the preceding items and an adjuvant base.
(Item X49A) A vaccine formulation comprising an antigen to at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria, and an adjuvant base.
(Item X49B) The vaccine formulation of item X49A, wherein a physical condition of a subject of the vaccine formulation comprises BCG vaccination history, tuberculosis infection history, or having antigen responsiveness to Mycobacterium tuberculosis, and the antigen comprises a human Mycobacterium tuberculosis hot water extract.
(Item X50) The vaccine formulation of any one of the preceding items, wherein the adjuvant base comprises a substance promoting a Th1 immune response (e.g., nucleic acid-based base such as CpG).
(Item X50A) A composition for use in preventing or treating a disease, disorder, or condition associated with an immunological abnormality in a subject, the composition comprising a component identified by a) obtaining an antigen responsiveness profile of a subject and b) identifying an antigen or a combination of antigens responsive to the subject based on the antigen responsiveness profile, at an amount sufficient to elicit an immune response in the subject.
(Item X50A1) A composition for use in preventing or treating cancer of a subject comprising a non-tumor component, wherein the component is an antigen or extract associated with anamnesis and vaccination history, wherein the subject is an individual with infection history or vaccination history described in any one of the preceding items, wherein the component is administered prophylactically to the subject before onset, administered to prevent recurrence after therapy, or administered in an early stage of onset of cancer, and the component is optionally administered in an early stage of onset of cancer or a precancerous condition.
(Item X50AA) The component of item X50A or X50A1, having one or more features of any one of items X1 to X50.
(Item X50B) A component for use in preventing or treating a disease, disorder, or condition associated with an immunological abnormality in a subject, wherein the component is identified by a) obtaining an antigen responsiveness profile of a subject and b) identifying an antigen or a combination of antigens responsive to the subject based on the antigen responsiveness profile.
(Item X50B1) A non-tumor component for use in preventing or treating cancer of a subject, wherein the component is an antigen or extract associated with anamnesis and vaccination history, wherein the subject is an individual with infection history or vaccination history described in any one of the preceding items, wherein the component is administered prophylactically to the subject before onset, administered to prevent recurrence after therapy, or administered in an early stage of onset of cancer, and the component is optionally administered in an early stage of onset of cancer or a precancerous condition.
(Item X50BB) The component of item X50B or X50B1, having one or more features of any one of items X1 to X50AA.
(Item X50C) Use of a component in the manufacture of a medicament for use in preventing or treating a disease, disorder, or condition associated with an immunological abnormality in a subject, wherein the component is identified by a) obtaining an antigen responsiveness profile of a subject and b) identifying an antigen or a combination of antigens responsive to the subject based on the antigen responsiveness profile.
(Item X50C1) Use of a non-tumor component in the manufacture of a medicament for use in preventing or treating cancer of a subject, wherein the component is an antigen or extract associated with anamnesis and vaccination history, wherein the subject is an individual with infection history or vaccination history described in any one of the preceding items, wherein the component is administered prophylactically to the subject before onset, administered to prevent recurrence after therapy, or administered in an early stage of onset of cancer, and the component is optionally administered in an early stage of onset of cancer or a precancerous condition.
(Item X50CC) The use of item X50C or X50C1, having one or more features of any one of items X1 to X50BB.

The present disclosure also provides the following.
(Item 1) A composition for use in treating or preventing a disease, disorder, or condition associated with an immunological abnormality of a subject, the composition comprising an antigen component that is specific in the subject to a component that is different from a causative agent of the disease, disorder, or condition.
(Item 2) The composition of the preceding item, wherein the disease, disorder, or condition comprises cancer, and wherein, preferably, the antigen component is a non-tumor antigen component.
(Item 3) The composition of any one of the preceding items, wherein the antigen component is specific to a memory T-cell of the subject.
(Item 4) The composition of any one of the preceding items, wherein the memory T cell is a memory regulatory T cell (IL-2 producing).
(Item 5) The composition of any one of the preceding items, wherein the antigen component has an immunostimulatory action.
(Item 6) The composition of any one of the preceding items, wherein the antigen component antigen-dependently acts on memory CD4 positive T cells.
(Item 7) The composition of any one of the preceding items, wherein the antigen component has activity to bias a ratio of presence between Foxp3 positive Treg cells and IFN-γ producing T cells.
(Item 8) The composition of any one of the preceding items, wherein the bias is an increase in IFN-γ producing T cells compared to Foxp3 positive Treg cells.
(Item 9) The composition of any one of the preceding items, wherein the antigen component has activity to bias a ratio of presence between Foxp3 positive Treg cells and type 1 helper T cells.
(Item 10) The composition of any one of the preceding items, wherein the IFN-γ producing T cells comprise type 1 helper T cells.
(Item 11) The composition of any one of the preceding items, wherein the antigen component has activity to bias a ratio of presence of Th1 cells.
(Item 12) The composition of any one of the preceding items, wherein the IFN-γ producing T cells are T-bet positive Th1 cells.
(Item 13) The composition of any one of the preceding items, wherein the antigen component that is specific has a capability to enhance at least one selected from the group consisting of IFN-γ producing capability, IL-2 producing capability, and TNF-α producing capability in a sample from the subject.
(Item 14) A biomarker for determining whether a non-tumor antigen component has anticancer action on a subject, the biomarker comprising at least one selected from the group consisting of whether the antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item 14A) A method for determining whether a non-tumor antigen component has anticancer action on a subject, the method comprising the step of determining at least one of (i) whether the antigen component antigen-dependently acts on memory CD4 positive T cells, (ii) whether the antigen component alters memory regulatory T cells, (iii) whether the antigen component alters IFN-γ producing capability, (iv) whether the antigen component alters IL-2 producing capability, and (v) whether the antigen component alters TNF-α producing capability.
(Item 15) A composition or a kit comprising an agent or means for detecting a biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.
(Item 15A) A composition or a kit for determining whether a non-tumor antigen component has anticancer action on a subject, wherein the composition or the kit comprises an agent or device which determines at least one selected from the group consisting of (i) whether the non-tumor antigen component antigen-dependently acts on memory CD4 positive T cells, (ii) whether the non-tumor antigen component alters memory regulatory T cells, (iii) whether the non-tumor antigen component alters IFN-γ producing capability, (iv) whether the non-tumor antigen component alters IL-2 producing capability, and (v) whether the non-tumor antigen component alters TNF-α producing capability.
(Item 16) The composition of any one of the preceding items, wherein the (non-tumor) antigen component comprises an antigen associated with anamnesis and vaccination history.
(Item 17) The composition of any one of the preceding items, wherein the subject is a subject with a history of an infection, and the antigen component comprises an antigen to the infection.
(Item 18) The composition of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item 19) The composition of any one of the preceding items, wherein the subject is a subject with BCG vaccination history, tuberculosis infection history, or antigen responsiveness to Mycobacterium tuberculosis, and the antigen component comprises a human Mycobacterium tuberculosis hot water extract.
(Item 20) The composition of any one of the preceding items, wherein the subject is a subject with influenza vaccination history, influenza infection history, or antigen responsiveness to an influenza virus, and the antigen component comprises an influenza virus.
(Item 21) A method of manufacturing or otherwise providing a composition for use in preventing or treating cancer of a subject, the method comprising:
   A) identifying a non-tumor antigen specific to the subject;
   B) identifying whether a subject has immunological memory of the non-tumor antigen, and selecting a non-tumor antigen for which the subject has the immunological memory; and
   C) manufacturing or otherwise providing the selected non-tumor antigen.
(Item 22) The method of any one of the preceding items, having one or more features in any one of the preceding items in B).
(Item 23) A method of determining whether a non-tumor antigen of a subject can prevent or treat cancer of the subject, the method comprising:
   B) identifying whether the subject has immunological memory of the non-tumor antigen, and identifying that cancer of the subject can be prevented or treated if the subject has the immunological memory.
(Item 24) The method of any one of the preceding items, having one or more features in any one of the preceding items in B).
(Item 25) A method for use in preventing or treating a disease, disorder, or condition associated with an immunological abnormality, comprising:
   a) obtaining an antigen responsiveness profile of a subject;
   b) identifying an antigen component or a combination of antigen components from the antigen responsiveness profile, wherein the antigen component or combination of antigen components is identified based on whether the antigen component or combination of antigen components has shown or shows to present immune response to the subject; and
   c) administering to the subject the antigen component or combination of antigen components identified in step b) at a sufficient amount to elicit an immune response in the subject.
(Item 26) The method of any one of the preceding items, wherein the disease, disorder, or condition comprises cancer.
(Item 27) The method of any one of the preceding items, wherein the obtaining an antigen responsiveness profile comprises checking a past physical condition of a subject, checking whether one or more of candidate antigens has responsiveness in a sample from the subject, or both.
(Item 28) The method of any one of the preceding items, wherein i) the antigen responsiveness profile comprises at least one selected from the group consisting of interview, anamnesis or vaccination history based on a Maternal and Child Health Handbook, an equivalent thereof, or the like, and a combination thereof, and/or ii) the checking whether one or more of candidate antigens has responsiveness comprises collecting a bodily fluid (e.g., blood) from the subject and separating peripheral blood cells, and then measuring whether the peripheral blood cells produce a cytokine in response to an antigen associated with the antigen profile, and other biomarkers.
(Item 29) The method of any one of the preceding items, further comprising periodically testing responsiveness of the antigen and confirming that responsiveness is maintained.
(Item 30) The method of any one of the preceding items, wherein a) and b) are performed with the following steps:
   i) obtaining a past physical condition of a subject;
   ii) collecting blood from the subject and separating peripheral blood cells, and then measuring whether the peripheral blood cells produce a cytokine in response to an antigen corresponding to the physical condition, and other biomarkers; and
   iii) identifying a suitable antigen component or a combination of antigen components from a result of ii).
(Item 31) The method of any one of the preceding items, wherein the past physical condition comprises anamnesis and vaccination history.
(Item 32) The method of any one of the preceding items, wherein the physical condition comprises history of infection, and the cancer vaccine comprises an antigen component or a combination of antigen components to the infection.
(Item 33) The method of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item 34) The method of any one of the preceding items, wherein the physical condition comprises BCG vaccination history, tuberculosis infection history, or antigen responsiveness to Mycobacterium tuberculosis, and the antigen component or combination of antigen components comprises a human Mycobacterium tuberculosis hot water extract.
(Item 35) The method of any one of the preceding items, wherein the physical condition comprises influenza vaccination history, influenza infection history, or antigen responsiveness to an influenza virus, and the antigen component or combination of antigen components comprises an influenza virus.
(Item 36) The method of any one of the preceding items, wherein the administration comprises subcutaneous administration or intradermal administration.
(Item 37) The method of any one of the preceding items, wherein the subject is in a state before onset of cancer, after a cancer treatment, an early stage of onset of cancer, or a precancerous condition.
(Item 38) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item 39) The method of any one of the preceding items, wherein the subject exhibits immunological resistance.
(Item 40) The method of any one of the preceding items, wherein step ii) comprises measuring induction of cells producing IFN-γ, IL-2, TNF-α, or a plurality of the cytokines concurrently.
(Item 41) The method of any one of the preceding items, wherein the antigen responsiveness profile is obtained by performing companion diagnosis in advance based on anamnesis and vaccination history.
(Item 42) The method of any one of the preceding items, wherein the past physical condition and the antigen component or combination of antigen components are tuberculosis infection history and a human Mycobacterium tuberculosis hot water extract.
(Item 43) The method of any one of the preceding items, wherein the past physical condition and the antigen component or combination of antigen components are influenza infection history and an influenza virus.
(Item 44) The method of any one of the preceding items, wherein the past physical condition and the component are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item 45) The method of any one of the preceding items, wherein the subject is a patient with BCG vaccination history or tuberculosis infection history, or a subject confirmed to have antigen responsiveness,
   wherein the Mycobacterium tuberculosis extract is prophylactically administered before onset, administered to prevent recurrence after therapy, or administered in an early stage of onset of cancer, and an extract from Mycobacterium tuberculosis is subcutaneously or intradermally administered by a conventional method in an early stage of onset of cancer or a precancerous condition.
(Item 46) The method of any one of the preceding items, wherein the subject is a subject who has influenza vaccination history or influenza infection history, or is confirmed to have antigen responsiveness,
   wherein the influenza vaccine is prophylactically administered before onset, administered to prevent recurrence after therapy, or administered in an early stage of onset of cancer, and an influenza vaccine is subcutaneously or intradermally administered in an early stage of onset of cancer or a precancerous condition.
(Item 47) A method of preventing or treating cancer immunity based on any one of the preceding items, comprising revaccinating the antigen component or combination of antigen components.
(Item 48) A method of preventing or treating cancer of a subject with a non-tumor component, wherein the component is an antigen or extract identified by interview and/or identified by reference to anamnesis or vaccination history of the subject, wherein the subject is an individual with an infection history or vaccination history described in any one of the preceding items, wherein the component is administered to prevent recurrence after therapy, administered prophylactically before onset, or administered in an early stage of onset of cancer to the subject, and the component is optionally administered in an early stage of onset of cancer or a precancerous condition.
(Item 49) The method of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item 50) The method of any one of the preceding items, wherein the subject is a patient exhibiting immunological resistance.
(Item 51) The method of any one of the preceding items, wherein the identification of responsiveness is characterized by infection history, vaccination history, and measuring induction of cells producing IFN-γ, IL-2, TNF-α, or a plurality of the cytokines concurrently using peripheral blood.
(Item 52) The method of any one of the preceding items, wherein the Mycobacterium tuberculosis extract is a hot water extract of human Mycobacterium tuberculosis or other extract from Mycobacterium tuberculosis (highly safe extract).
(Item 53) The method of any one of the preceding items, wherein the influenza virus is a human influenza virus or other extract from an influenza virus (highly safe extract).
(Item 54) A vaccine formulation comprising the antigen of any one of the preceding items and an adjuvant base.
(Item 54A) A vaccine formulation comprising an antigen to at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria, and an adjuvant base.
(Item 54B) The vaccine formulation of any one of the preceding items, wherein a physical condition of a subject of the vaccine formulation comprises BCG vaccination history, tuberculosis infection history, or having antigen responsiveness to Mycobacterium tuberculosis, and the antigen comprises a human Mycobacterium tuberculosis hot water extract.
(Item 55) The vaccine formulation of any one of the preceding items, wherein the adjuvant base comprises a substance promoting a Th1 immune response.
(Item 56) The vaccine formulation of any one of the preceding items, wherein the vaccine formulation is used for personalized medicine.
(Item 25A) A composition for use in treating or preventing a disease, disorder, or condition associated with an immunological abnormality, comprising an antigen component or a combination of antigen components for use in a method of preventing or treating a disease, disorder, or condition associated with an immunological abnormality, comprising:
   a) obtaining an antigen responsiveness profile of a subject;
   b) identifying an antigen component or a combination of antigen components from the antigen responsiveness profile, wherein the antigen component or combination of antigen components is identified based on whether the antigen component or combination of antigen components has shown or shows to present immune response to the subject; and
   c) administering to the subject the antigen component or combination of antigen components identified in step b) at a sufficient amount to elicit an immune response in the subject.
(Item 26A) The antigen component or combination of antigen components or the composition of any one of the preceding items, wherein the disease, disorder, or condition comprises cancer.
(Item 27A) The antigen component or combination of antigen components or the composition of any one of the preceding items, wherein the obtaining an antigen responsiveness profile comprises checking a past physical condition of a subject, checking whether one or more of candidate antigens has responsiveness in a sample from the subject, or both.
(Item 28A) The antigen component or combination of antigen components or the composition of any one of the preceding items, wherein i) the antigen responsiveness profile comprises at least one selected from the group consisting of interview, anamnesis or vaccination history based on a Maternal and Child Health Handbook, an equivalent thereof, or the like, and a combination thereof, and/or (ii) the checking whether one or more of candidate antigens has responsiveness comprises collecting a bodily fluid (e.g., blood) from the subject and separating peripheral blood cells, and then measuring whether the peripheral blood cells produce a cytokine in response to an antigen associated with the antigen profile, and other biomarkers.
(Item 29A) The antigen component or combination of antigen components or the composition of any one of the preceding items, further comprising periodically testing responsiveness of the antigen and confirming that responsiveness is maintained.
(Item 30A) The antigen component or combination of antigen components or the composition of any one of the preceding items, wherein a) and b) are performed with the following steps:
   i) obtaining a past physical condition of a subject;
   ii) collecting blood from the subject and separating peripheral blood cells, and then measuring whether the peripheral blood cells produce a cytokine in response to an antigen corresponding to the physical condition, and other biomarkers; and
   iii) identifying a suitable antigen component or a combination of antigen components from a result of ii).
(Item 31A) The antigen component or combination of antigen components or the composition of any one of the preceding items, wherein the past physical condition comprises anamnesis and vaccination history.
(Item 32A) The antigen component or combination of antigen components or the composition of any one of the preceding items, wherein the physical condition comprises history of infection, and the cancer vaccine comprises an antigen component or a combination of antigen components to the infection.
(Item 33A) The antigen component or combination of antigen components or the composition of any one of the preceding items, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item 34A) The antigen component or combination of antigen components or the composition of any one of the preceding items, wherein the physical condition comprises BCG vaccination history, tuberculosis infection history, or antigen responsiveness to Mycobacterium tuberculosis, and the antigen component or combination of antigen components comprises a human Mycobacterium tuberculosis hot water extract.
(Item 35A) The antigen component or combination of antigen components or the composition of any one of the preceding items, wherein the physical condition comprises influenza vaccination history, influenza infection history, or antigen responsiveness to an influenza virus, and the antigen component or combination of antigen components comprises an influenza virus.
(Item 36A) The antigen component or combination of antigen components or the composition of any one of the preceding items, wherein the administration comprises subcutaneous administration or intradermal administration.
(Item 37A) The antigen component or combination of antigen components or the composition of any one of the preceding items, wherein the subject is in a state before onset of cancer, after a cancer treatment, an early stage of onset of cancer, or a precancerous condition.
(Item 38A) The antigen component or combination of antigen components or the composition of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item 39A) The antigen component or combination of antigen components or the composition of any one of the preceding items, wherein the subject exhibits immunological resistance.
(Item 40A) The antigen component or combination of antigen components or the composition of any one of the preceding items, wherein step ii) comprises measuring induction of cells producing IFN-γ, IL-2, TNF-α, or a plurality of the cytokines concurrently.
(Item 41A) The antigen component or combination of antigen components or the composition of any one of the preceding items, wherein the antigen responsiveness profile is obtained by performing companion diagnosis in advance based on anamnesis and vaccination history.
(Item 42A) The antigen component or combination of antigen components or the composition of any one of the preceding items, wherein the past physical condition and the antigen component or combination of antigen components are tuberculosis infection history and a human Mycobacterium tuberculosis hot water extract.
(Item 43A) The antigen component or combination of antigen components or the composition of any one of the preceding items, wherein the past physical condition and the antigen component or combination of antigen components are influenza infection history and an influenza virus.
(Item 44A) The antigen component or combination of antigen components or the composition of any one of the preceding items, wherein the past physical condition and the component are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.
(Item 45A) The antigen component or combination of antigen components or the composition of any one of the preceding items, wherein the subject is a subject who has BCG vaccination history or tuberculosis infection history, or is confirmed to have antigen responsiveness,
   wherein the Mycobacterium tuberculosis extract is prophylactically administered before onset or administered in an early stage of onset of cancer, and an extract from Mycobacterium tuberculosis is subcutaneously or intradermally administered by a conventional method in an early stage of onset of cancer or a precancerous condition.
(Item 46A) The antigen component or combination of antigen components or the composition of any one of the preceding items, wherein the subject is a subject who has influenza vaccination history or influenza infection history, or is confirmed to have antigen responsiveness,
   wherein the influenza vaccine is prophylactically administered before onset, administered to prevent recurrence after therapy, or administered in an early stage of onset of cancer, and an influenza vaccine is subcutaneously or intradermally administered in an early stage of onset of cancer or a precancerous condition.
(Item 47A) An antigen component or a combination of antigen components or a composition for use in a method of preventing or treating cancer immunity based on any one of the preceding items, the method comprising revaccinating the antigen component or combination of antigen components.
(Item 48A) A non-tumor component for use in a method of preventing or treating cancer of a subject, wherein the component is an antigen or extract identified by interview and/or identified by reference to anamnesis or vaccination history of the subject, wherein the subject is an individual with an infection history or vaccination history described in any one of the preceding items, wherein the component is administered to prevent recurrence after therapy, administered prophylactically before onset, or administered in an early stage of onset of cancer to the subject, and the component is optionally administered in an early stage of onset of cancer or a precancerous condition.
(Item 49A) The non-tumor component of any one of the preceding items, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.
(Item 50A) The non-tumor component of any one of the preceding items, wherein the subject is a patient exhibiting immunological resistance.
(Item 51A) The antigen or combination of antigens or the non-tumor component of any one of the preceding items, wherein the identification of responsiveness is characterized by infection history, vaccination history, and measuring induction of cells producing IFN-γ, IL-2, TNF-α, or a plurality of the cytokines concurrently using peripheral blood.
(Item 52A) The antigen or combination of antigens of any one of the preceding items, wherein the Mycobacterium tuberculosis extract is a hot water extract of human Mycobacterium tuberculosis or other extract from Mycobacterium tuberculosis (highly safe extract).
(Item 53A) The antigen or combination of antigens of any one of the preceding items, wherein the influenza virus is a human influenza virus or other influenza virus (highly safe extract).

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

### [Advantageous Effects of Invention]

The present disclosure provides a technology, which can effectively prevent or treat refractory diseases such as cancer with immunological memory of a host.

For cancer immunotherapy, immunotherapy using a tumor antigen is actively studied, but a clear therapeutic effect had not been obtained. However, the present disclosure was able to solve this. The present disclosure also unexpectedly found an aspect as "specific" immunotherapy of a human Mycobacterium tuberculosis hot water extract, which was considered "non-specific" immunotherapy using a non-tumor component, and conceived of providing this as a specific immunotherapy. This was not only able to provide specific therapy, but also preventive use. It was demonstrated, by elucidating the mechanism of action of the specific immunotherapy, that a suitable patient is selected and a suitable therapy is provided as personalized medicine, which can be used in various applications.

Regulatory T cells (Treg cells) are involved in many immune regulations, especially immunosuppression. Some checkpoint molecule inhibitors disable immunosuppression of regulatory T cells to enhance immunity. However, while these agents exhibit useful effects, the problem to be solved is in having potent side effects. The effect is limited to some of the patients, and a clear diagnostic drug that can distinguish patients is not available. Since side effects are potent if suitable distinction is not possible, distinction is required. While it was nearly impossible in the past, the present disclosure was able to provide a suitable therapeutic and prevention method by distinguishing patients using the immunological memory mechanism, and distinguishing and administering a suitable agent to a patient. This was confirmed by demonstrating a novel immunotherapy and cancer therapy and prevention method using memory responses by a non-tumor antigen as an personalized marker based on the regulatory action of a non-tumor antigen dependent regulatory T cell (Treg cell) due to past vaccination using a human Mycobacterium tuberculosis hot water extract or the like (confirmed to be safe in humans), used as a representative example. Therefore, the present disclosure provides a novel immunotherapy.

### [Brief Description of Drawings]

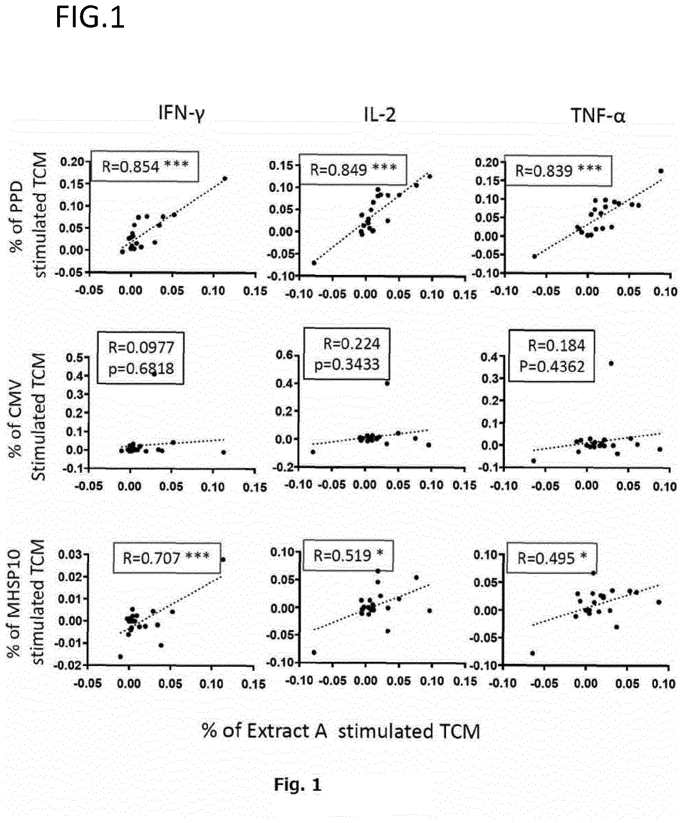

[Figure 1] Figure **1** is a diagram from studying the correlation between Mycobacterium tuberculosis infection and memory T cells, showing results of conducting an experiment under the conditions described in Example 1. The diagrams are, from the left of the top row, diagrams showing the correlation between production cells induced by PPD and production cells induced by Extract A for IFN-γ, IL-2, and TNF-α. The diagrams in the middle row are, from the left, diagrams showing the correlation between production cells induced by CMV and production cells induced by Extract A for IFN-γ, IL-2, and TNF-α. Diagrams in the bottom row are, from the left, diagrams showing the correlation between production cells induced by MHSP10 and production cells induced by Extract A for IFN-γ, IL-2, and TNF-α.

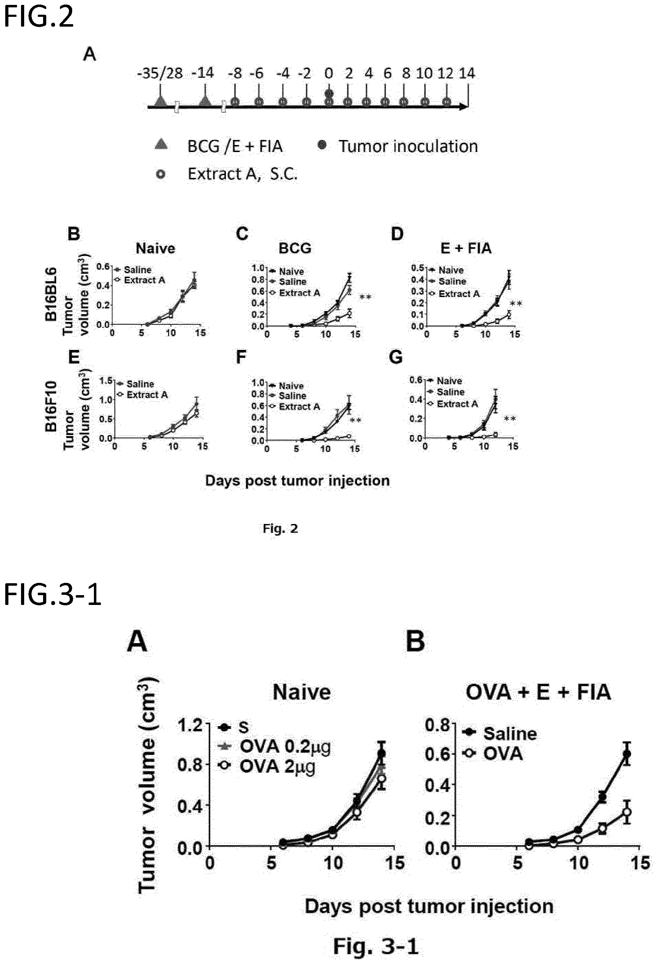

[Figure 2] Figure **2** is a diagram from studying the antitumor effect of Extract A depending on the difference in the immunological state. Example 2 describes the details thereof. Figure **2A** shows the administration schedule for BCG or Extract A + Freund's incomplete adjuvant (E + FIA), tumor cells, and Extract A. The top row of Figure **2B** shows results of transplanting B16BL6 to, from the left, (B) naive mouse, (C) BCG infected mouse, and (D) Extract A emulsion immunized mouse, and the bottom row shows results of transplanting B16F10 to, from the left, (E) naive mouse, (F) BCG infected mouse, and (G) Extract A emulsion immunized mouse. The horizontal axis indicates the number of days elapsed after transplantation of tumor, and the vertical axis indicates the change in tumor volume. * indicates that there is a statistically significant difference.

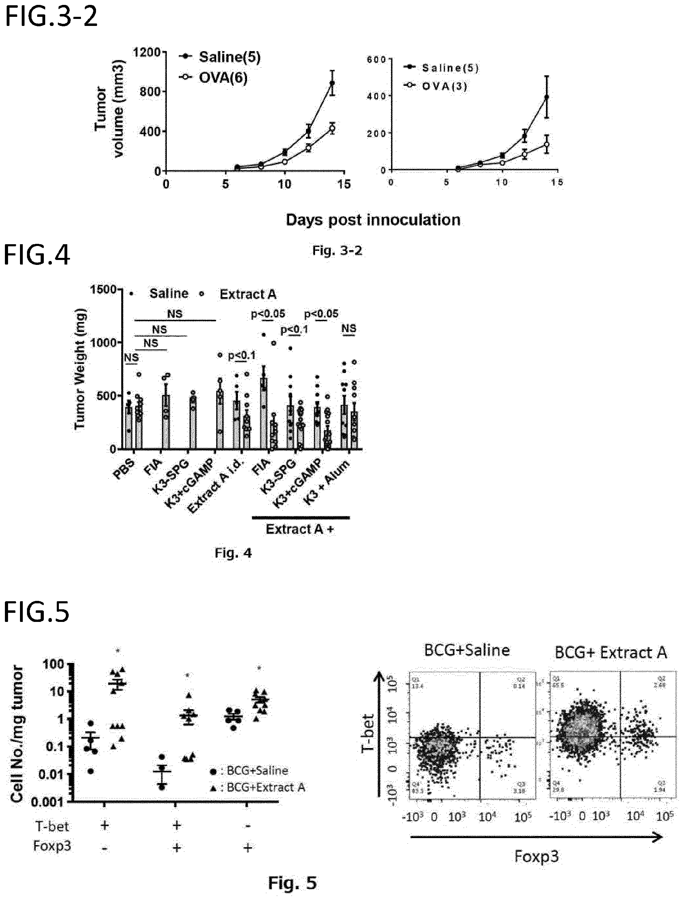

[Figure 3-1] Figures **3-1** and **3-2** show experimental results on the effect of suppressing tumor growth when a model antigen was administered to an animal with model antigen specific memory cells. Figure **3-1** shows the change in tumor growth of B16BL6 when ovalbumin was administered to, from the left, naive mouse (A) and ovalbumin + FIA + Extract A immunized mouse (B).
[Figure 3-2] Figure **3-2** shows changes in the tumor volume of B16BL6 when CD4T cells differentiated into ovalbumin specific Th1 were introduced and ovalbumin was administered to, from the left, Rag2 deficient mouse (left) and IL2Ry chain Rag2 double deficient mouse (right).
[Figure 4] Figure **4** shows the tumor weight of B16BL6 when Extract A was administered to mice immunized with a vaccine formulation comprising various adjuvant bases. Example 4 provides an explanation in detail. From the left, vaccines used in immunization four weeks before and two weeks before are shown. The results are from mice administered with phosphate buffered saline (PBS), FIA, adjuvant K3-SPG (see Kouji Kobiyama, et al., Proceedings of the National Academy of Sciences Feb 2014, 111 (8) 3086-3091; DOI: 10.1073/pnas.1319268111), K3 + cyclic GMP-AMP (cGAMP), intradermal administration of Extract A (id), Extract A + FIA, Extract A + K3 - SPG, Extract A + K3 + cGAMP, and Extract A + K3 + Alum. Black dots indicate results in animals administered with saline, and red dots indicate results in animals administered with Extract A.
[Figure 5] Figure **5** shows results of evaluating the effect of Extract A on tumor infiltrating lymphocytes (TIL) in transplanted tumor by flow cytometry, details of which are described in Example 5. The left panels shows, from the left, T-bet positive Foxp3 negative, T-bet positive Foxp3 positive, and T-bet negative Foxp3 positive TIL. The right panel shows results of a typical flow cytometry.

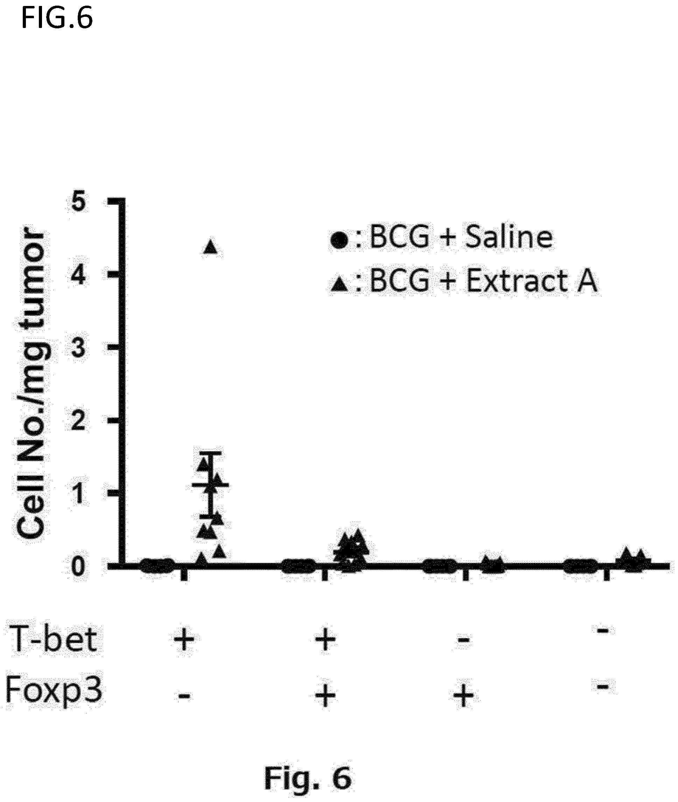

[Figure 6] Figure **6** shows results of evaluating the effect of Extract A on tumor infiltrating lymphocytes (TIL) in transplanted tumor by flow cytometry, details of which are described in Example 6. The figure shows, from the left, T-bet positive Foxp3 negative, T-bet positive Foxp3 positive, T-bet negative Foxp3 positive, and T-bet negative Foxp3 negative TIL producing IFN-γ by Extract A stimulation.

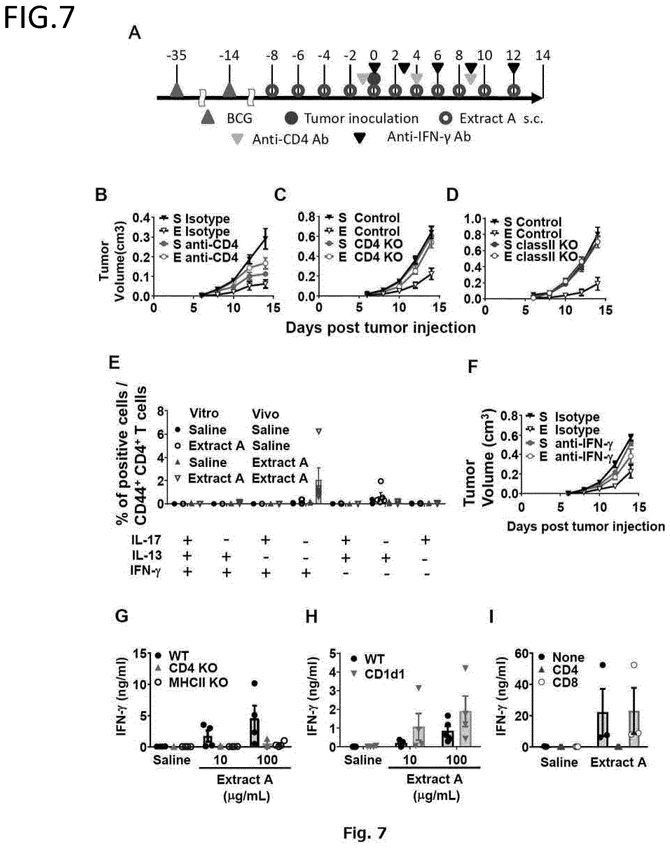

[Figure 7] Figure **7** shows results of related experiments for identifying cells that are essential for an antitumor effect due to Extract A. The details thereof are described in Example 8. Figure **7A** is a diagram showing the schedule for administration of BCG, tumor cells, Extract A, anti-CD4 antibody, and anti-IFN-γ antibody to mice. Figures **7B** to **7D** show the change in volume of subcutaneously transplanted B16BL6 when saline (S) or Extract A (E) was administered to mice for which CD4 positive cells were depleted using an anti-CD4 antibody after BCG infection (Figure **7B****),** or CD4 knockout mice (Figure **7C****)** or MHC class II knockout mice (Figure **7D****)** infected with BCG, respectively. Figure **7E** shows results of administering saline or Extract A to BCG infected mice and collecting spleen cells, and then stimulating with Extract A, staining intracellular cytokines, and performing FACS analysis. Figure **7F** shows the tumor volume when saline (S) or Extract A (E) was administered to mice depleted of IFN-γ by using anti-IFN-γ antibodies after BCG infection. Figure **7G** is a diagram showing the quantity of IFN-γ produced when splenocytes of BCG infected wild-type mice (WT), CD4 knockout mice (CD4 KO), and MHC class II knockout mice (MHCII KO) were stimulated with Extract A. Figure **7H** is a diagram showing the quantity of IFN-γ produced when splenocytes of BCG infected wild-type mice (WT) and CD1d1 knockout mice (CD1d1) were stimulated with Extract A. Figure **7I** is a diagram showing the quantity of IFN-γ produced when splenocytes of CD4 or CD8 depleted mice were stimulated with Extract A.

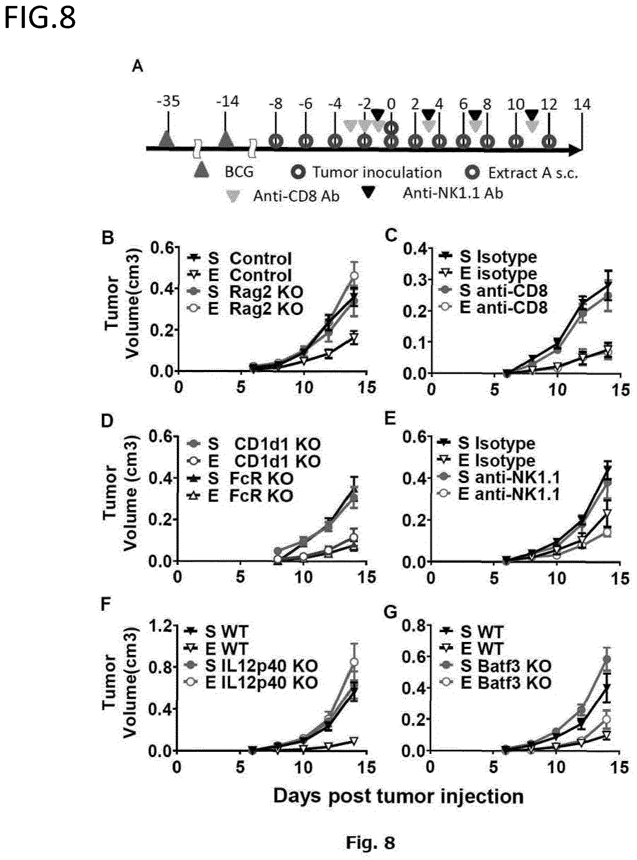

[Figure 8] Figure **8** shows the change in tumor volume of B16BL6 when Extract A was administered to BCG infected mice. The details thereof are described in Example 9. Figure **8A** is a diagram showing the experimental schedule. Figures **8B** to **G** show the tumor volume when saline (S) or Extract A (E) was administered after infecting Rag2 deficient mice (Figure **8B****),** CD8 positive cell depleted mice (Figure **8C****),** CD1d1 deficient mice and FcR deficient mice (Figure **8D**), NK1.1 positive cell depleted mice (Figure **8E****),** IL12p40 deficient mice (Figure **8F****),** and Batf3 deficient mice (Figure **8G****)** with BCG, respectively.

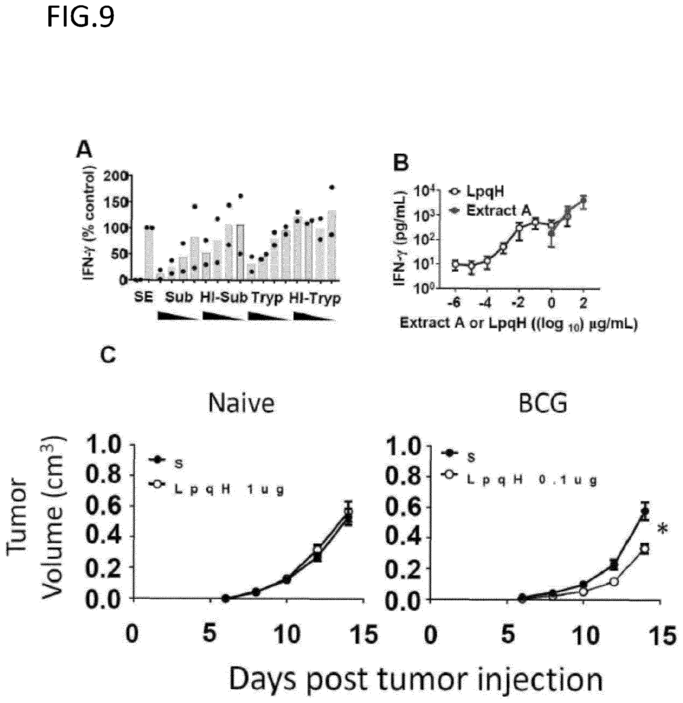

[Figure 9] Figure **9** shows results of an anti-tumor effect when a non-tumor antigen was administered to a BCG infected mouse. The details thereof are described in Example 10.
   Figure **9A** shows the quantity of IFN-γ secreted when splenocytes isolated from a BCG infected mouse was treated with subtilisin treated Extract A (Sub), heat inactivated subtilisin treated Extract A (HI-Sub), trypsin treated Extract A (Trp), or heat inactivated trypsin treated Extract A (HI-Trp) as a relative value, with the quantity of IFN-γ production from Extract A stimulation as 100%.
   Figure **9B** shows the quantity of IFN-γ produced when splenocytes isolated from a BCG infected mouse were stimulated with Extract A or LpqH. Figure **9C** shows the tumor volume when saline or LpqH was administered to naive mice and BCG infected mice.

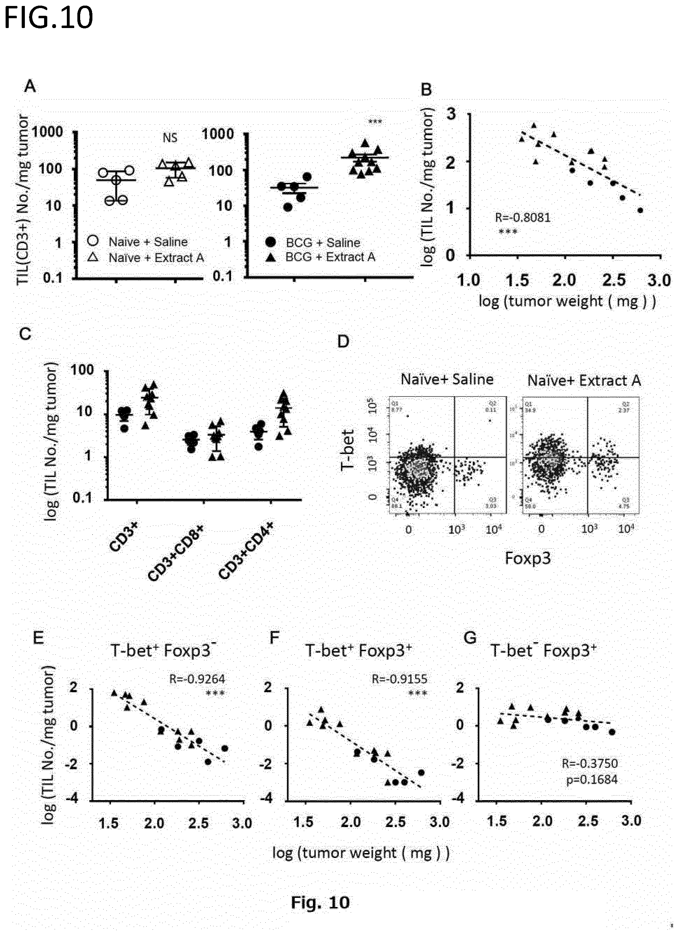

[Figure 10] Figure **10** is a diagram showing the result of analyzing the tumor microenvironment after administration of Extract A. The details thereof are described in Example 11. The left panel of Figure **10A** shows the TIL cell count per 1 mg of tumor of a naive mouse administered with saline or Extract A, and the right panel shows the TIL cell count per 1 mg of tumor of a BCG infected mouse administered with saline or Extract A. Figure **10B** shows the correlation between the tumor weight and TIL cell count per 1 mg of tumor. Figure **10C** shows the CD3 positive TIL cell count, CD3 positive CD8 positive TIL cell count, and CD3 positive CD4 positive TIL cell count per 1 mg of tumor of a BCG infected mouse administered with saline or Extract A. Figure **10D** shows a typical result of T-bet and Foxp3 expression for TIL of a naive mouse administered with saline or Extract A, analyzed by flow cytometry. Figures**10E** to **G** show the correlation between tumor weight and T-bet positive Foxp3 negative TIL cells (Figure **10E****),** T-bet positive Foxp3 positive TIL cells (Figure **10F****),** and T-bet negative Foxp3 positive TIL cells (Figure **10G**).

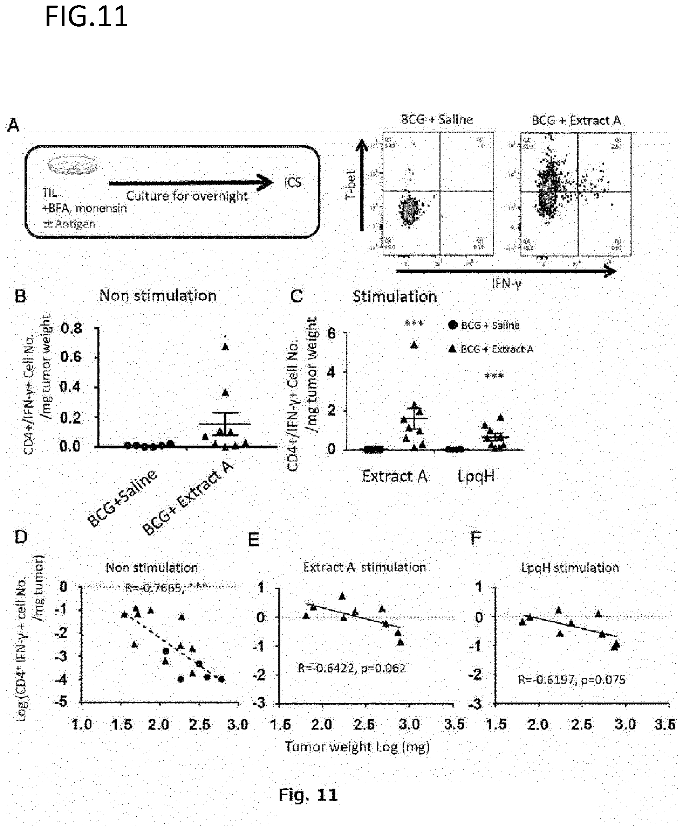

[Figure 11] Figure **11** is a diagram showing the correlation between an antitumor effect due to Extract A and TIL producing IFN-γ. The details thereof are described in Example 12. The left panel of Figure **11A** shows the experimental protocol, and the right panel shows the results of a typical flow cytometry on expression of T-bet and IFN-γ in TIL in BCG infected mice administered with saline or Extract A. Figure **11B** shows the IFN-γ positive TIL cell count per 1 mg of tumor under non-stimulating conditions. Figure **11C** shows the IFN-γ positive TIL cell count per 1 mg of tumor under Extract A or LpqH stimulation conditions. Figures **11D to F** show the correlation between tumor weight and IFN-γ positive TIL cell count under non-stimulating conditions (Figure **11D****),** IFN-γ positive TIL cell count under Extract A stimulation conditions (Figure **11E**), and IFN-γ positive TIL cell count under LpqH stimulation conditions.

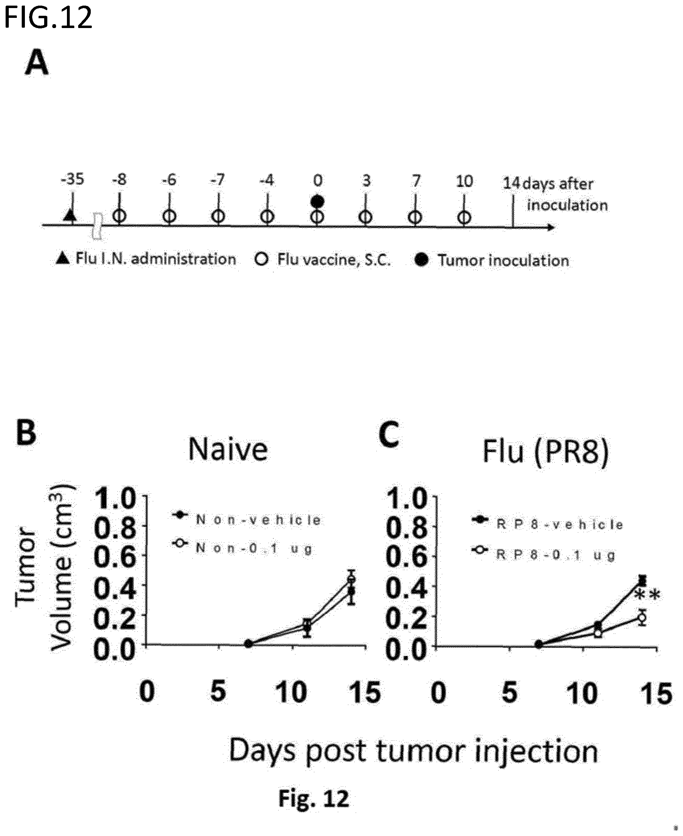

[Figure 12] Figure **12** is a diagram of testing an antitumor effect of an influenza vaccine due to a difference in the immunological state. The details thereof are described in Example 13. Figure **12A** shows the dosing schedule of influenza virus PR8, tumor cells, and influenza vaccine.
Figure **12B** shows results of implanting B16BL6 in a naive mouse, and Figure **12C** shows results thereof in a PR8 infected mouse. The horizontal axis indicates the days after tumor implantation, and the vertical axis indicates the change in tumor volume. * indicates a statistically significant difference.

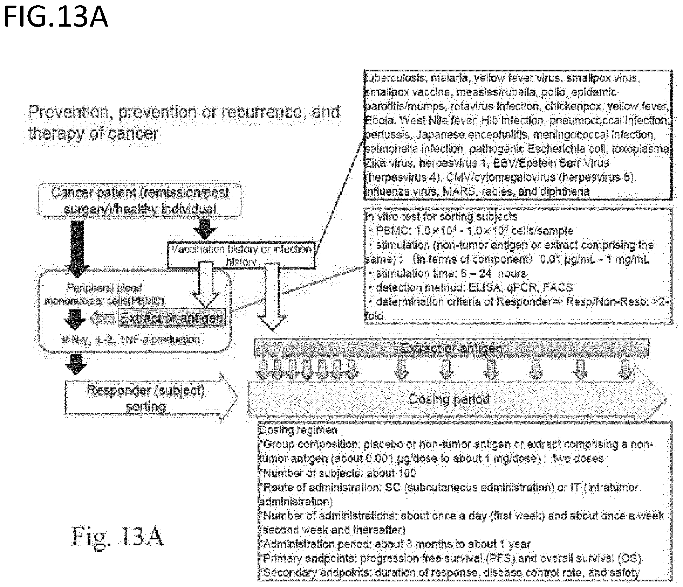

[Figure 13A] Figure **13A** is an example of schematic diagram of a clinical protocol.

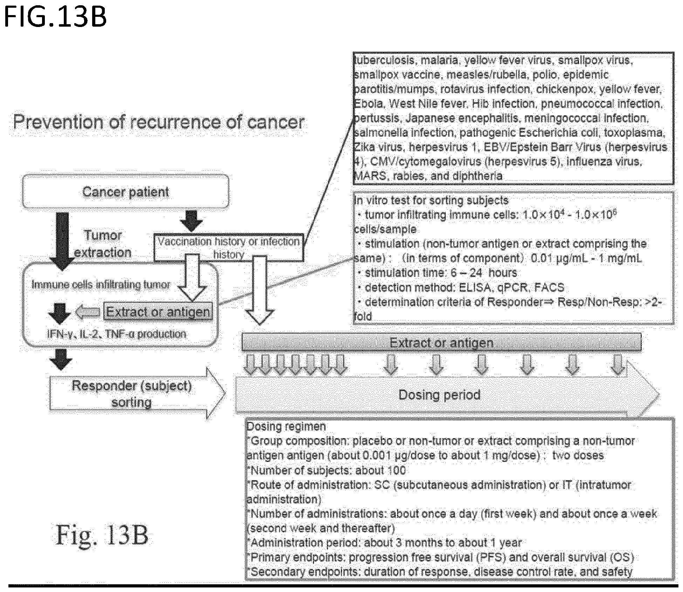

[Figure 13B] Figure **13B** is another example of schematic diagram of a clinical protocol.

### [Description of Embodiments]

The present disclosure is explained hereinafter while showing some of the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definitions)

The terms used herein are explained hereinafter.

As used herein, the term "specific", with respect to some type of substance or component, refers to the substance or component having a property of eliciting in a subject a reaction that is unique to the subject. In a typical example herein, "specific" means that the subject has immunological memory, particularly in the field of therapy or prevention. In particular, this term can mean specific to a memory T cell of a subject.

As used herein, whether a subject "has immunological memory" for a certain component or substance can be evaluated by measuring whether the component or substance, in the subject or biological component from the subject (e.g., cell or the like), (i) antigen-dependently improves cytokine production of or has growth promoting action on memory CD4 positive T cells, (ii) alters the expression of a surface antigen of a memory regulatory T cell, (iii) changes the ratio of Treg to Th1, (iv) induces IFN-γ production from T-bet positive Th1 cells, (v) alters the IFN-γ production capability, (vi) alters the IL-2 production capability, (vii) alters the TNF-α production capability, and (viii) has an antibody specific to a component or substance in blood, and confirming that at least one thereof results in being positive.

As used herein, an "antigen component" refers to a component that can elicit an antigen-antibody reaction in a subject and is also referred to as "antigen" herein. "Antigen component" can be a single component (substance) or a complex. An antigen component can be provided as a plurality of different combinations, which is then referred to as a "combination of antigen components". An antigen component herein can be provided as an isolated antigen component, a complex thereof, or an extract comprising the same.

As used herein, "non-tumor antigen component" refers to an antigen component, which is not a cancer antigen. It is possible to check whether a component or substance herein is a "non-tumor antigen component" by, for example, comprehensively identifying and comparing proteins or mRNA at a tumor site and other sites. For the identification, mass spectrometry, microarray, or next generation sequencer is primarily used. It can be verified by studying sequence information or the like. An "antibody" refers to a protein serving the role of recognizing and eliminating a foreign object. In such a case, the foreign object is referred to as an "antigen". In general, a component such as a protein that is specifically or excessively present in cancer is referred to as a "cancer antigen". Therefore, a non-tumor antigen (component) can be deemed as any component other than a cancer antigen. As used herein, a non-tumor antigen component can have immunostimulatory action (or adjuvant activity).

As used herein, an antigen component used in the present disclosure can be an antigen associated with anamnesis and vaccination history or an antigen to an infection.

As used herein, "antigen associated with anamnesis and vaccination history" refers to an antigen component included in a vaccine or anamnesis. This can be verified by an approach referred to as ELISA for studying whether there is a specific antibody, or an approached referred to as FACS or ELISPOT for checking whether there is an antigen specific T cell.

As used herein, an "antigen to an infection" refers to a component which can elicit an immune response from an organism or virus that can cause an infection. This can be checked by an approach referred to as ELISA for studying whether there is a specific antibody, or by whether there is an antigen specific T cell.

As used herein, an "infection" can be any infection such as tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, diphtheria, or the like. The infection is preferably tuberculosis. If tuberculosis is used as the infection, a subject preferably has BCG vaccination history, a history of tuberculosis infection or antigen responsiveness to Mycobacterium tuberculosis.

As used herein, "antigen responsiveness" is defined as the same as the meaning that is known in the art, referring to a subject eliciting an antigen-antibody reaction in response to a specific substance or the like for the subject.

As used herein, an "antigen responsiveness profile", in the context of a certain subject, is the collective term (profile) for antigen responsiveness of the subject to various substances or the like. An antigen responsiveness profile can be obtained by various approaches such as by checking past physical conditions such as anamnesis (e.g., history of infection) and vaccination history or by actually checking antigen responsiveness using an antigen panel. They can be materialized, for example, by interview, anamnesis or vaccination history based on a Maternal and Child Health Handbook, an equivalent thereof, or the like, and combinations thereof. Further, whether there is responsiveness can be verified by collecting bodily fluids (e.g., blood) from the subject and separating peripheral blood cells, and then measuring whether the peripheral blood cells produce a cytokine (IL-2, IFN-γ, TNF-α, a combination of two or more thereof, or the like) in response to an antigen associated with the antigen profile, and other biomarkers.

As used herein, a "memory T cell" refers to a cell that functions to maintain immunological memory by being present in the body for a long period of time. 90% of effector T cells die after 1 to 2 weeks in the body, unless continuously exposed to the same antigen. Some of the remaining T cells subsequently differentiate into roughly two cell populations, which function as a "memory cell" due to functioning to maintain immunological memory by being present in the body for a long period of time. Memory cells, when roughly classified, include central memory T cells (T_{CM}) and effector memory T cells (T_{EM}). An immune response can be quickly triggered when the same pathogen infiltrates again by the survival of such two types of memory T cells for a long period of time. It is understood that not only does the memory T cell produced upon the first encounter with an antigen continue to survive, but also a pool of memory T cells is newly formed each time the same antigen is encountered again.

T_{CM} cells are similar to naive T cells with regard to their cell surface markers; and they express CCR7, which is a chemokine receptor, CD62L, which is a cell adhesion factor, and the like. The cells are primarily present in the T cell region of secondary lymphoid tissue. It is understood that when exposed to the same antigen again, the cells produce IL-2 and quickly grow, and some differentiate into T_{EM} cells.

Meanwhile, it is understood that T_{EM} cells have reduced expression of cell adhesion factors such as CD62L and CCR7, are present not in the secondary lymphoid tissue, but mainly locally in inflammation (e.g., lung, liver, intestinal tract, or the like), and produce a large quantity of cytokines such as IL-4, IFN-γ, or IL-5 by the same antigen stimulation.

In a preferred embodiment, memory T cells targeted by the component of the present disclosure can be a memory regulatory T cell (IL-2 producing). Regulatory T cells (Treg) are a type of T cells that block excessive immune responses. Treg cells are roughly distinguished into endogenous Treg (natural Treg; nTreg) cells that are naturally produced in the thymic gland and induced Treg (iTreg) cells resulting from stimulation of a cytokine or the like at the peripheral tissue. A "memory regulatory T cell (IL-2 producing)" is a cell with features of both a "memory T cell" and "regulatory T cell".

As used herein, "immunostimulatory action" refers to an action of nonspecifically activating a biological immune function to enhance the decreased defense. It is possible to verify whether a certain substance has "immunostimulatory action" by a reporter gene assay of a natural immunoreceptor, a test of evaluating activation of immune cells using lymphocytes or the like with production of cytokines or the like as an indicator.

As used herein, "activated" refers to a state in which a function of a cell (e.g., T cell), protein, polypeptide, gene, or the like is increased. "Activated" includes a state in which a change or increase in a function of a cell is found, a state in which cell growth is elevated, a state in which expression of a protein, polypeptide, gene, or the like is elevated, a state in which the pattern of expression has changed, a state in which cells with a suppression capability are suppressed, a state in which a suppressed function is unlocked, and the like. Activation particularly refers to activation of regulatory T cells (Treg) herein. Activation of regulatory T cells (Treg) is also referred to as, and is synonymous with, conversion of regulatory T cells (Treg). As used herein, activation of Treg is also mentioned with respect to a target. In such a case, this refers to exertion of a prophylactic or therapeutic effect on a disease associated with activation of Treg with respect to a target. Examples of the target in such a case include, but are not limited to, cancer and an agent derived from cancer.

Whether an immune cell is "activated" can be checked through a test or the like described in "Immunostimulatory action". "Activation" of a gene can be quantified by real-time PCR, RNA-Seq, northern hybridization, hybridization utilizing a DNA array, or the like. The amount of expression of a polypeptide can be quantified by using an antibody that recognizes the polypeptide, staining compound that has binding affinity to the polypeptide, or the like. Conventional methods used in the art can also be used besides the quantification methods described above.

As used herein, "non-target antigen component" refers to, when envisioning a certain target (e.g., cancer), components other than the target (e.g., non-tumor component when the target is cancer).

As used herein, "adjuvant" refers to a substance used for enhancing an effect (immunogenicity) of an agent such as a vaccine (antigen) by co-administration with the agent. The term is derived from the word "adjuvare", which means "assist" in Latin. It is possible to check whether a certain substance functions as an "adjuvant" on another substance (e.g., antigen) by performing a test of administering the substance with an antigen into a mouse to evaluate antigen specific antibody production.

As used herein, an "antigen dependent" "action" with respect to a certain component, in the context of a target such as a T cell, refers to the component having an action on the target as a result of an antigen-antibody reaction. This can be verified by elimination of action when an antigen-antibody reaction is blocked or the like.

As used herein, a "memory CD4 positive T cell" refers to a memory T cell with positive CD4. In this regard, CD4 positive as used herein is considered positive in view of a higher level of staining by immunostaining or the like using an antibody specific to CD4 labeled with a fluorescent dye or the like compared to the staining level with a non-specific antibody used as a negative control.

As used herein, a "Foxp3 positive Treg cell" refers to a Treg cell with positive Foxp3. In this regard, Foxp3 positive as used herein is considered positive in view of a higher level of staining by immunostaining or the like using an antibody specific to Foxp3 labeled with a fluorescent dye or the like compared to the staining level with a non-specific antibody used as a negative control.

As used herein, an "IFN-γ producing T cell" refers to a T cell with a capability to produce interferon γ (IFN-γ). In this regard, an IFN-γ producing T cell as used herein can be identified in view of a higher level of staining by immunostaining or the like using an antibody specific to IFN-γ labeled with a fluorescent dye or the like compared to the staining level with a non-specific antibody used as a negative control.

As used herein, a "type 1 helper T cell" is also referred to as a "Th1 cell" and is a subgroup of a CD4 positive T cell (so-called helper T cell) such as naive CD4 positive T cell matured in the thymic gland. This is a type of cell that quickly enters the bloodstream, migrates to an infected site, and secretes cytokines such as IFN-γ or IL-2 to induce activation of macrophages or inflammatory reaction. Th1 cells are also responsible for cellular immunity, which is a locally occurring immune response in which CTL or macrophage directly attacks cells. Subgroups of CD4 positive T cells also include type 2 helper T cells (Th2 cells), which secrete cytokines such as IL-4 or IL-5 and activates naive B cells that recognize the same antigen in the secondary lymphoid tissue. Th2 cells are responsible for humoral immunity, which is an immune response centered around B cells and antibody.

As used herein, a "T-bet positive Th1 cell" refers to a Th1 cell with positive T-bet. In this regard, T-bet positive as used herein is considered positive in view of a higher level of staining by immunostaining or the like using an antibody specific to T-bet labeled with a fluorescent dye or the like compared to the staining level with a non-specific antibody used as a negative control. The transcription factor T-bet encoded in the Tbx21 gene in a Th1 cell controls and feed forward the production of interferon γ as a lineage determining transcription factor directly and positively. Interferon γ is classified as a type II interferon as one of the interferon family with an anti-pathogenic and antitumor effect. It is known to induce the expression of T-bet, which is a transcription factor defining a Th1 cell, and to maintain the production of interferon γ by feed forward control.

As used herein, "enhance(ment)" in the ability such as IFN-γ producing capability, IL-2 producing capability, and TNF-α producing capability is determined to be enhanced in view of a significant increase in the number of cells produced or amount of production of IFN-γ and IL-2 by stimulation of an antigen or the like compared to a negative control by immunostaining using an antibody specific to IFN-γ, IL-2, or TNF-α labeled with a label (substance or the like) such as a fluorescent dye. The quantity of cytokine produced can be measured by ELISA, and the cytokine producing cell count can be measured by FACS.

As used herein, a "biomarker" refers to a substance such as a protein or an event measured in a biological sample such as blood, wherein the presence or degree of progression of a specific physical condition such as a disease is reflected by the presence/absence, concentration, or level thereof. Therefore, as used herein, a biomarker is understood to include events such as whether the biomarker antigen-dependently acts on memory CD4 positive T cells, alters a memory regulatory T cell, alters the ratio of Treg to Th1, induces IFN-γ production from T-bet positive Th1 cells, alters the IFN-γ production capability, alters the IL-2 production capability, and alters the TNF-α production capability.

Whether a biomarker antigen-dependently acts on memory CD4 positive T cells can be determined with a statistically significant increase in the positive cell count of an antibody used in staining by FACS analysis.

Whether a biomarker alters a memory regulatory T cell can be determined with a statistically significant increase in the positive cell count of an antibody used in staining by FACS analysis.

Whether a biomarker alters the ratio of Treg to Th1 or induces IFN-γ production from T-bet positive Th1 cells can be determined from measurement with FACS after intracellular cytokine staining and transcription factor staining or measurement of produced cytokines by ELISA.

Whether a biomarker alters the IFN-γ production capability, IL-2 production capability, and TNF-α production capability can be determined from measurement with FACS after intracellular cytokine staining and transcription factor staining or measurement of produced cytokines by ELISA.

As used herein, the "bias" in the "presence ratio" of cells refers to having a ratio that is statistically significantly different from a ratio of a plurality of types of cells that are presence in a normal state. Whether there is a bias can be determined by referring to a cell analysis result obtained by any approach for analyzing cells (e.g., FACS or the like).

As used herein, a "human Mycobacterium tuberculosis hot water extract" is typically a substance produced from a human Mycobacterium tuberculosis, which is a mixture including polysaccharides with arabinose, mannose, and glucose as the primary ingredients. While anticancer effects due to human Mycobacterium tuberculosis hot water extracts have been studied for a long time, the detailed mechanism of action thereof is not necessarily elucidated. Further, the extract has not been used as a prophylactic drug. The extract can also comprise a trace amount of ingredients such as a protein, peptide, amino acid, nucleic acid, or lipid (glycolipid) when appropriate.

The following is a representative manufacturing method of human Mycobacterium tuberculosis hot water extracts.

Human Mycobacterium tuberculosis is cultured for 3 to 7 weeks in a 37°C thermostatic vessel. The film of microbial cells formed on a medium is then filtered out. The moist microbial cells with medium components removed by washing with water are used as the extracted raw material. The microbial cells are floated in a distilled water at an amount that is 15 to 40-fold of the wet weight thereof, and heated for 80 to 180 minutes at 90 to 120°C for extraction. The residue of the microbial cells is removed with a sterilization filter, and the extracted solution is concentrated to 60% or less, and then acetone, trichloroacetate, ammonium sulfate, sulfosalicylic acid, or the like is added thereto so as to arrive at 0.5 to 3% (w/v), and stirred and left standing. The deposited precipitate is then centrifuged and removed, and the supernatant is subjected to running water dialysis. Inner fraction of dialysis fluid is subjected to vacuum concentration to a 1/20 to 1/4 volume, and sodium chloride is added to the concentrate so as to arrive at 0.5 to 1% (w/v). 2 to 4-fold volume of ethanol is added and left standing, and then the precipitate is removed by centrifugation. After adding an additional 2 to 6-fold volume of ethanol and incubating, a precipitating polysaccharide is obtained by centrifugation or the like. Those skilled in the art can understand that each of the conditions described above can be appropriately changed to obtain the same product.

As used herein, "prophylaxis" or "prevention" is an act of administering an active ingredient in the present disclosure to an individual who has not developed the target disease, intended to for example prevent development of the disease.

As used herein, "therapy" is, for example, an act of administering an active ingredient of the present disclosure to an individual (subject, patient) diagnosed as having a developed disease by a physician or a similar practitioner, intended to, for example, alleviate the disease or condition, not increase carcinoma, or revert back to the state before the development of the disease. Even if the objective of administration is prevention of exacerbation of the disease or condition or prevention of increase in the carcinoma, the administration is a therapeutic act if administered to a patient.

As used herein, an "immunological abnormality" refers to any disease, disorder, or condition at least partially resulting from, or suspected to result from, an abnormality in the immune system. Examples of immunological abnormalities include, but are not limited to, autoimmune diseases and the like.

Prevention and therapy of an immunological abnormality include prevention of an immunological abnormality, recovery from an immunological abnormality, and preemptive prevention of an immunological abnormality.

### (Description of preferred embodiments)

The preferred embodiments of the present disclosure are described hereinafter. It is understood that the embodiments provided hereinafter are provided to better facilitate the understanding of the present disclosure, and thus the scope of the present disclosure should not be limited by the following descriptions. Thus, it is apparent that those skilled in the art can refer to the descriptions herein to make appropriate modifications within the scope of the present disclosure. It is also understood that the following embodiments of the present disclosure can be used alone or in combination.

The present disclosure is based on the discovery that a therapeutic and preventive effect, which is specific and effective against neoplasm such as cancer, is attained by using a non-tumor antigen component for which a subject has immunological memory.

### <Prevention and therapy of disease based on immunological memory mechanism>

The present disclosure provides, in summary, a composition for use in preventing or treating a disease, disorder, or condition of a subject based on an immunological memory mechanism, or a therapeutic or preventive method using the same principle. In this regard, the composition comprises an antigen component specific in the subject, or an antigen component for which the subject has immunological memory, to a component that is different from a causative agent of the disease, disorder, or condition.

In one representative aspect, the present disclosure provides a composition for activating (or converting) a regulatory T cell (Treg) having immunological memory of a non-target antigen component, which is suppressed in a subject, against a target, comprising the non-target antigen component. Alternatively, the present disclosure provides a method for activating (or converting) a regulatory T cell (Treg) having immunological memory of a non-target antigen component, which is suppressed in a subject, against a target, comprising administering an effective amount of the non-target antigen component to the subject.

The present disclosure has found that activation of Treg imparts an ability to kill the target or an immunostimulatory action against the target. Enhancement of healing power due to such impartation of an immunological capability was unexpected from normal immunological memory.

The present disclosure has found that a contained component (non-target component) has anti-target immunity that is the same or better than a target (antigen), independently from non-specific immunological action. By confirming this in other components, the inventors found that a non-target antigen component specific to a subject (or for which the subject has immunological memory) can be broadly used in treating or preventing a target (e.g., cancer). For example, it was demonstrated that a human Mycobacterium tuberculosis hot water extract comprising said antigen or an influenza virus antigen exhibits a prophylactic and therapeutic effect on the development of diseases other than tuberculosis and influenza (e.g., cancer) in a BCG infected animal (i.e., animal that can be deemed to have immunological memory of a component of Mycobacterium tuberculosis as an antigen) or an influenza infected animal used as a model. It was also newly found that for a therapeutic and/or preventive effect by a specific component (for BCG, human Mycobacterium tuberculosis hot water extract) in a subject with immunological memory of the specific component such as a BCG infected animal or an influenza virus infected animal, antigen responsiveness of the specific component (e.g., Mycobacterium tuberculosis hot water extract) is important in addition to conventionally understood infiltration promoting action to a target (e.g., tumor) site of lymphocytes or the like by immunostimulatory action, and the mechanism thereof is to recall an antigen response by immunological memory due to a vaccine (antigen) or the like inoculated in the past and promote an immunological action against the target by an antigen response due to a non-target antigen thereof.

In one embodiment, the present disclosure provides a composition for use in activating a regulatory T cell (Treg) having immunological memory of a non-tumor antigen component, which is suppressed in a subject, comprising the non-tumor antigen component. Alternatively, the present disclosure provides a method for activating a regulatory T cell (Treg) having immunological memory of a non-tumor antigen component, which is suppressed in a subject, comprising administering an effective amount of the non-tumor antigen component to the subject. Activation of Treg can impart an ability to kill tumor or an immunostimulatory action against the tumor in the present disclosure.

In one embodiment, the Treg is a memory T cell and/or a CD4 positive cell. Although not wishing to be bound by any theory, this is because a relationship with immunological memory and activity via CD4 positive cells have been found.

In another embodiment, the antigen component comprises a protein, a part thereof, or a peptide.

In one embodiment, an antigen component comprises an antigen selected from the group consisting of a pathogen of an infection or a part thereof, an antigen associated with anamnesis, and an antigen associated with vaccination history. In one specific embodiment, an antigen component comprises a human Mycobacterium tuberculosis hot water extract or an influenza virus antigen.

In another embodiment, a composition is characterized in that the composition is used in a method comprising checking whether the antigen component has immunological memory of Treg in the subject, and administering the antigen component to the subject if the subject has immunological memory of the antigen component.

In the conversion technology of the present disclosure, it is understood that various embodiments of the same type of constituent elements described herein can be used as the various constituent elements, and combinations thereof are also within the scope of the present disclosure.

In one aspect, the present disclosure provides a composition for use in preventing or treating cancer of a subject, wherein the composition comprises a non-tumor antigen component specific to the subject or a non-tumor antigen component for which the subject has immunological memory.

In this manner, the present disclosure identifies the responsiveness of a subject to a component that is effective as a cancer vaccine based on the past physical condition of the subject and administers the component that is responsive to the physical condition to the subject to prevent or treat cancer.

Thus in another aspect, the present disclosure provides a method for use in preventing or treating cancer, comprising: a) obtaining a past physical condition of a subject; b) identifying responsiveness of the subject to a component that is effective as a cancer vaccine based on the physical condition; and c) administering to the subject the component that is responsive to the physical condition.

In one embodiment, it is preferable in the present disclosure that an active ingredient such as a non-tumor antigen component is specific to a memory T cell of the subject. Specifically, specific to a memory T cell means that the subject has immunological memory of a certain specific antigen.

In one embodiment, cancer therapeutic drugs are specific examples found in the present disclosure. In such a case, it is notable that the antitumor action of a human Mycobacterium tuberculosis hot water extract was found in the present disclosure. Human Mycobacterium tuberculosis hot water extracts have been deemed to have a non-specific immunotherapy, and have immunostimulatory action as the primary mechanism of action. Meanwhile, the inventors found that a contained component has antitumor immunity that is the same or better than a cancer antigen, independently from non-specific immunological action, as a result of diligent study. By confirming this in other components, the inventors found that a non-tumor antigen component specific to a subject (or for which the subject has immunological memory) can be broadly used in treating or preventing cancer. For example, it was demonstrated that a human Mycobacterium tuberculosis hot water extract comprising said antigen exhibits a preventive and therapeutic effect on the development of cancer in a BCG infected animal used as a model (i.e., animal that can be deemed to have immunological memory for a component of Mycobacterium tuberculosis as an antigen). It was also newly found that for a therapeutic and/or preventive effect by a specific component (for BCG, human Mycobacterium tuberculosis hot water extract) in a subject with immunological memory for the specific component such as a BCG infected animal, antigen responsiveness of the specific component (e.g., Mycobacterium tuberculosis hot water extract) is important in addition to conventionally understood infiltration promoting action to a tumor site of lymphocytes or the like by immunostimulatory action, and the mechanism thereof is to recall an antigen response by immunological memory due to a vaccine (antigen) or the like inoculated in the past and promote an antitumor immunological action by an antigen response due to a non-tumor antigen thereof.

In one embodiment, the memory T cell associated with immunological memory targeted in the present disclosure is a memory regulatory T cell. As an indicator thereof, IL-2 production for example can be observed. Examples of the indicator include, in addition to IL-2 production, IFN-γ production, TNF-α production, a combination of two or three thereof, and the like. Specifically, when determining that there is immunological memory, immunological memory for a target antigen can be determined to be present by adding the antigen to a test system comprising a T cell and then observing that IL-2 production, IFN-γ production, TNF-α production, or a combination of two or three thereof is enhanced.

In one embodiment, a specific component such as a non-tumor antigen component of the present disclosure has immunostimulatory action (e.g., adjuvant activity). Immunostimulatory action can be checked by any approach that is known in the art. For example, evaluation of action on a natural immunoreceptor, the capability to induce production of a specific antibody, or the like can be used.

In one embodiment, a specific component such as a non-tumor antigen component of the present disclosure antigen-dependently acts on memory CD4-positive T cells. It is possible to check whether the component antigen-dependently acts on memory CD4-positive T cells by any approach known in the art. For example, flow cytometry analysis using an MHC II tetramer and a specific antigen peptide or flow cytometry analysis on IFN-γ producing cells after stimulation with a specific antigen can be used.

In one embodiment, a specific component such as a non-tumor antigen component of the present disclosure has activity to bias a ratio of the presence between Foxp3 positive Treg cells and IFN-γ producing T cells. The bias in the ratio of presence between Foxp3 positive Treg cells and IFN-γ producing T cells can be checked by any technology for analyzing cells (e.g., FACS). A typical example includes using an approach of measurement by FACS after intracellular cytokine staining and transcription factor staining. Examples of a baseline of determination in such a case includes, but are not limited to, the bias in the ratio of presence between Foxp3 positive Treg cells and IFN-γ producing T cells increasing IFN-γ producing T cells compared to Foxp3 positive Treg cells. IFN-γ producing T cells can include type 1 helper T cells. Alternatively in another embodiment, a specific component such as a non-tumor antigen component of the present disclosure has activity to bias a ratio of presence between Foxp3 positive Treg cells and type 1 helper T cells.

Although not wishing to be bound by any theory, the mechanism of action of the bias in the ratio of presence between Foxp3 positive Treg cells and IFN-γ producing T cells was considered as an important aspect, and the inventors found, as the mechanism of action thereof, that a specific component such as a human Mycobacterium tuberculosis (component eliciting immunological memory) antigen-dependently acts on memory CD4-positive T cells and biases the ratio of the presence between Foxp3 positive Treg cells and IFN-γ producing T cells such as type 1 helper T cells (Th1 cells) to cause an antitumor immunity-like change, and increase IFN-γ producing T cells in tumor. Specifically, the present disclosure can be expected to more strongly express tumor antigen dependent antitumor immunity in the body by activating dormant T cells and changing the balance of suppressor T cells and antitumor immunity with the non-tumor antigen component contained in the present agent or the like in addition to the action of promoting lymphocyte infiltration into tumor. In one embodiment in the present disclosure, an antigen component comprises a component that can elicit an immune response via CD4 positive T cells. In one embodiment in the present disclosure, the subject is checked as to whether the subject can elicit an antitumor immune response via CD4 positive T cells, and if the subject can elicit an antitumor immune response via CD4 positive T cells, the composition is administered. In another embodiment, the immune response is not mediated through CD8 positive T cells.

In one embodiment in the present disclosure, targeted IFN-γ producing T cells can comprise type 1 helper T cells. Although not wishing to be bound by any theory, this is because anticancer action can be enhanced by antigen-dependent action on memory CD4-positive T cells and causing an antitumor immunity-like change of type 1 helper T cells (Th1 cells), and increasing IFN-γ producing T cells in tumor.

In another embodiment, the IFN-γ producing T cells in the present disclosure are T-bet positive Th1 cells. Although not wishing to be bound by any theory, this is because interferon γ is known to induce the expression of a transcription factor T-bet defining a Th1 cell and maintains the production of interferon γ by feed forward control in the defense of a host. The novel role served by T-bet for recognizing autocrine interferon by a Th1 cell has been studied. Interferon γ induces an abnormal type I interferon response in the absence of T-bet. T-bet preferentially suppresses the gene and pathway activated by autocrine type I interferon, and suppresses abnormal amplification of the type I interferon signaling system. Therefore, T-bet is considered to serve the role of not only proactively inducing differentiation into Th1 cells, but also suppressing abnormal autocrine type I interferon and downstream signaling system thereof in Th1 cells (see Harms Pritchard, G., Hall, A. O., Christian, D. A. et al.: Diverse roles for T-bet in the effector responses required for resistance to infection. J. Immunol., 194, 1131-1140 (2015) and the like).

In one embodiment in the present disclosure, the non-tumor antigen component that is specific (or for which the subject has immunological memory) preferably has a capability to enhance at least one selected from the group consisting of IFN-γ producing capability, IL-2 producing capability, and TNF-α producing capability in a sample from the subject. Although not wishing to be bound by any theory, this is because IFN-γ producing capability, IL-2 producing capability, and TNF-α producing capability are advantageous in anticancer action.

Since a BCG infection model that can be used herein is an infection model that is similar to anamnesis and vaccination history, it can be understood as a fact that can be derived based on data demonstrated thereby that dormant memory T cells established by past vaccination is stimulated by a specific antigen and reactivated to tumor antigen, thus independently promoting antitumor immune response. This hypothesis was demonstrated by conducting an experiment in other immunological memory models. Therefore, it is understood that therapy of a disease based on the immunological memory model in the present disclosure can be applied to not only the Examples, but also to any disease. The same effect can also be observed at the clinical trial level.

Although not wishing to be bound by any theory, as the mechanism of action of the present disclosure, it is understood that the bias in the ratio of Th1 cells and Foxp3 positive Treg cells in an animal model and production of IFN-γ are important, while the initial response of a specific component in the present disclosure being a memory regulatory T cell (IL-2 producing) based on anamnesis and vaccination history in peripheral blood derived cells (of humans or the like), and the specific component of the present disclosure eliciting IFN-γ production to change the ratio of Foxp3 positive regulatory T cells (Treg) and T-bet positive Th1 cells and induce IFN-γ production from T-bet positive Th1 cells by a chronological experiment also serve an important role as a functioning mechanism. The findings on the involvement of the IFN-γ production, IL-2 production, and TNF-α production from memory regulatory T cells based on immunological memory of a subject such as anamnesis and vaccination history were unknown but found as a result of diligent study by the inventors. Therefore, the present disclosure has demonstrated that IFN-γ production, IL-2 production, and TNF-α production used in peripheral blood derived cells of a human or the like can be a biomarker for selecting a responder.

Specifically, the present disclosure shows that the IFN-γ producing capability, IL-2 producing capability, and TNF-α producing capability of the agent can be found in advance with human peripheral blood derived cells and suggests the possibility of being a companion diagnosis for individual subjects. Since the present biomarker is not a response from a cancer antigen, the biomarker can also be used for healthy individuals without a cancer antigen, thus enabling preventive treatment.

In this manner, the discovery of new findings based on scientific evidence on antitumor immune action of human Mycobacterium tuberculosis hot water extracts that are already confirmed to be safe has led to inventions of novel antitumor immunotherapy and preventive method, which can select and treat a suitable subject. An enhancement in the working effect of known cancer immunotherapy such as checkpoint molecule inhibitors can also be expected. Furthermore, this mechanism is not only adaptable to Mycobacterium tuberculosis, but can also be expected to enhance the antitumor effect in relevant antigen treatment based on vaccination history or infection history for other infections, and provide the optimal treatment to each individual.

Before transplanting cancer cells into an animal, a mixture of a human Mycobacterium tuberculosis hot water extract and an adjuvant base was administered in advance as a vaccine into a normal animal, and a human Mycobacterium tuberculosis hot water extract was subsequently administered by a conventional method, and then cancer cells were transplanted to evaluate an antitumor effect. It was found as a result that a potent antitumor effect which is different from a conventional effect can be elicited by pretreatment (vaccine-like treatment) of a mixture of a human Mycobacterium tuberculosis hot water extract and adjuvant base, even under conditions where human Mycobacterium tuberculosis hot water extract alone or adjuvant base alone does not exhibit an effect.

The antigen response capability (triple response of IFN-γ, TNF-α, and IL-2) in splenocyte derived CD4 positive CD44 positive cells was enhanced by administering a Mycobacterium tuberculosis extract when the effect of BCG attenuated after BCG vaccination of mice. A useful treatment method that can maintain a vaccine effect against an increase in infection due to attenuation of the vaccine effect, which is currently an issue, has been found in view of this result.

The present disclosure can be expected to enhance the antitumor effect in a relative antigen treatment based on infection history or vaccination history for not only Mycobacterium tuberculosis infections but also other infections. Specifically, the present disclosure provides an personalized cancer immunotherapy method/preventive method, which targets individuals with infection or vaccination history and can predict the efficacy in advance by a response of memory T cells. This is advantageous in that immunotherapy can be selected with a relevant antigen vaccine depending on the infection history or vaccination history of each individual unlike conventional immunotherapy that cannot be used without identifying a cancer antigen. Further, this can be applied to healthy individuals because a cancer antigen is not used, thus enabling preventive treatment. In particular, the present disclosure provides a therapeutic method and a preventive method that exhibits an effect even on carcinoma, on which a preventive effect and therapeutic effect are not exhibited with only subcutaneous administration of the present agent, by using a nonconventional combination and administration method, with a human Mycobacterium tuberculosis hot water extract established to be safe in humans as an active ingredient.

### <Biomarkers>

In one aspect, the present disclosure provides a biomarker for determining whether a non-tumor antigen component has anticancer action on a subject, wherein the biomarker in the present disclosure can be an event such as whether the (non-tumor) antigen component of the present disclosure (i) antigen-dependently acts on memory CD4 positive T cells, (ii) alters memory regulatory T cells, or (iii) induces IFN-γ production from T-bet positive Th1 cells. Therefore, the present disclosure also provides a method for determining whether a non-tumor antigen component has anticancer action on a subject, the method comprising the step of determining at least one of (i) whether the non-tumor antigen component antigen-dependently acts on memory CD4 positive T cells, (ii) whether the non-tumor antigen component alters memory regulatory T cells, (iii) whether the non-tumor antigen component alters IFN-γ producing capability, (iv) whether the non-tumor antigen component alters IL-2 producing capability, and (v) whether the non-tumor antigen component alters TNF-α producing capability.

Thus, in another aspect, the present disclosure provides a composition or a kit comprising means for detecting a biomarker for determining whether a (non-tumor) antigen component has anticancer action on a subject, wherein the means for detecting a biomarker can be any means that can check whether the non-tumor antigen component antigen-dependently acts on memory CD4 positive T cells, alters memory regulatory T cells, or alters IFN-γ, IL-2, and TNF-α producing capability from T-bet positive Th1 cells. Various known and commercially available kits can be used. As such, the present disclosure also provides a composition or a kit for determining whether a non-tumor antigen component has anticancer action on a subject, wherein the composition or the kit comprises an agent or device which determines at least one selected from the group consisting of (i) whether the non-tumor antigen component antigen-dependently acts on memory CD4 positive T cells, (ii) whether the non-tumor antigen component alters memory regulatory T cells, (iii) whether the non-tumor antigen component alters IFN-γ producing capability, (iv) whether the non-tumor antigen component alters IL-2 producing capability, and (v) whether the non-tumor antigen component alters TNF-α producing capability.

A specific embodiment includes isolating peripheral mononuclear cells from a subject, stimulating memory CD4 positive cells contained therein with various antigens, and staining an intracellular cytokine or transcription factor to obtain an antigen responsiveness profile and the like. In another embodiment, any one or more features described in <Prevention and therapy of disease based on immunological memory mechanism> can be combined and applied in implementing the biomarker of the present disclosure.

### <Manufacturing or otherwise providing methods>

In another aspect, the present disclosure provides a method of manufacturing or otherwise providing a composition for use in preventing or treating a disease or disorder of a subject. The method comprises: A) identifying an antigen specific to the subject but not specific to the disease or disorder; B) identifying whether a subject has immunological memory of the non-tumor antigen, and selecting a non-tumor antigen for which the subject has the immunological memory; and C) manufacturing or otherwise providing the selected non-tumor antigen. As used herein, "or otherwise providing" refers to any method of providing other than newly producing an item, and can be, for example, a method of isolating, purifying, or obtaining the item from a suitable source. In the step of C), the non-tumor antigen may be produced de novo, or otherwise provided such as by isolation from other source or the like.

In another aspect, the present disclosure provides a method of manufacturing or otherwise providing a composition for use in preventing or treating cancer of a subject. The method comprises: A) identifying a non-tumor antigen specific to the subject; B) identifying whether a subject has immunological memory of the non-tumor antigen, and selecting a non-tumor antigen for which the subject has the immunological memory; and C) manufacturing or otherwise providing the selected non-tumor antigen. In the step of C), the non-tumor antigen may be produced de novo, or otherwise provided such as by isolation from other source or the like.

In the manufacturing method of the present disclosure, any one or more features described in <Prevention and therapy of disease based on immunological memory mechanism> can be appropriately applied.

### <Therapeutic/preventive method, companion diagnosis/therapy, and medicament>

In one aspect, the present disclosure provides a method for use in preventing or treating a disease, comprising: a) obtaining an antigen responsiveness profile of a subject; b) identifying an antigen or a combination of antigens responsive to the subject based on the antigen responsiveness profile; and c) administering to the subject the component at a sufficient amount to elicit an immune response in the subject. In one embodiment, the present disclosure provides a method for use in preventing or treating a disease, disorder, or condition associated with an immunological abnormality, comprising: a) obtaining an antigen responsiveness profile of a subject; b) identifying an antigen or a combination of antigens from the antigen responsiveness profile, wherein the antigen or the combination of antigens has shown or shows to present immune response to the subject; and c) administering to the subject the antigen or the combination of antigens identified in step b) at a sufficient amount to elicit an immune response in the subject. Further, the present disclosure also provides an antigen or a combination of antigens for use in a method of preventing or treating a disease, disorder, or condition associated with an immunological abnormality, comprising: a) obtaining an antigen responsiveness profile of a subject; b) identifying an antigen or a combination of antigens from the antigen responsiveness profile, wherein the antigen or the combination of antigens has shown or shows to present immune response to the subject; and c) administering to the subject the antigen or the combination of antigens identified in step b) at a sufficient amount to elicit an immune response in the subject.

In a specific aspect, the present disclosure provides a method for use in preventing or treating cancer, comprising: a) obtaining an antigen responsiveness profile of a subject; b) identifying an antigen or a combination of antigens responsive to the subject based on the antigen responsiveness profile; and c) administering to the subject the component at a sufficient amount to elicit an immune response in the subject.

In one aspect, the present disclosure provides a method of determining whether a non-tumor antigen of a subject can prevent or treat cancer of the subject. The method comprises: B) identifying whether the subject has immunological memory of the non-tumor antigen, and identifying that cancer of the subject can be prevented or treated if the subject has the immunological memory of the non-tumor antigen. In the method of the present disclosure, any one or more features described in <Prevention and therapy of disease based on immunological memory mechanism> can be appropriately applied.

The present disclosure also provides a composition or pharmaceutical composition comprising an antigen or a combination of antigens responsive to a subject obtained based on such an antigen responsiveness profile. Alternatively, the present disclosure provides an antigen or a combination of antigens responsive to a subject obtained based on such an antigen responsiveness profile for use in treating or preventing a disease or disorder. Alternatively, the present disclosure provides use of an antigen or a combination of antigens responsive to a subject obtained based on such an antigen responsiveness profile in a medicament for use in treating or preventing a disease or disorder.

Therefore, the present disclosure provides a composition for use in preventing or treating a disease, disorder, or condition of a subject using a component identified by the method described above. The composition comprises an antigen component that is specific in the subject (or for which the subject has immunological memory) to a component that is different from a causative agent of the disease, disorder, or condition.

In one embodiment, the method of preventing or treating in the present disclosure can comprise a) obtaining a past physical condition of a subject; b) identifying responsiveness of the subject to a component that is effective as a cancer vaccine based on the physical condition; and c) administering to the subject the component that is responsive to the physical condition.

In a specific embodiment, obtaining of an antigen responsiveness profile in the present disclosure can be confirmed by approaches including checking the past physical condition of a subject, checking whether one or more antigen candidates have responsiveness in a sample derived from the subject, or both.

In another embodiment, the present disclosure provides a method of preventing or treating cancer, comprising a) obtaining a past physical condition of a subject; b) identifying responsiveness of the subject to a component that is effective as a cancer vaccine based on the physical condition; and c) administering to the subject the component that is responsive to the physical condition.

In a specific embodiment, the past physical condition that can be used comprises anamnesis and vaccination history. Anamnesis and vaccination history can be obtained by referring to, for example, a Maternal and Child Health Handbook or an equivalent thereof, medical records, other medical information (including electronically recorded information on a subject stored in the cloud, electronic chip, or the like). A Maternal and Child Health Handbook (MCH handbook) is a book containing essential information maintained by a family for promoting and maintaining health of a mother and a child (2009 International Committee on MCH Handbook: ICMCHH)

In a specific embodiment, the physical condition that can be used comprises history of infection, and the cancer vaccine that can be used comprises an antigen to the infection. Infections that can be used can be any type of infection such as tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.

Infections applied by the present disclosure can be any infection described herein. Thus, physical condition, when described as "BCG vaccination history", "tuberculosis infection history", "antigen responsiveness to Mycobacterium tuberculosis", or the like in the context of a physical condition herein, can be replaced with any infection described herein. As a non-limiting example of any infection, the therapeutic method, prophylactic method, and medicament of the present disclosure can also be used when having "influenza infection history" as described and demonstrated in Example 13.

In a preferred embodiment, the infection used is tuberculosis. In such a case, the physical condition comprises BCG vaccination history, tuberculosis infection history, or antigen responsiveness to Mycobacterium tuberculosis. In a preferred embodiment, if the infection is tuberculosis, it can be advantageous for an antigen component or cancer vaccine to comprise a human Mycobacterium tuberculosis hot water extract. The present disclosure can be used in prevention as well as therapy.

The antigen or cancer vaccine of the present disclosure can be provided in a dosage form of a pharmaceutical composition. In a specific embodiment, a pharmaceutical composition can comprise one or more compounds and at least one pharmaceutically acceptable carrier, wherein the one or more compounds can be converted into, for example, at least one type of compound of Extract A (see the Examples) (i.e., prodrug) in a subject. In a specific embodiment, a pharmaceutical composition can comprise one or more compounds and at least one pharmaceutically acceptable carrier, wherein the one or more compounds can be converted into, for example, at least one type of (non-tumor) antigen component (i.e., prodrug) in a subject. A plurality of agents, when included, can be included in a single composition (combined agent) or in separate compositions. The agents can be formulated as a single composition using a known embodiment in the art, including those exemplified herein. A plurality of agents can be provided to achieve therapy (e.g., anticancer agent administration, radiation therapy, or the like) or with one or more other medicaments (e.g., surgery, chemotherapeutic agent, immune checkpoint inhibitor, or other anticancer agents) in addition to the antigen (component) or cancer vaccine of the present disclosure. The antigen (component) or cancer vaccine of the present disclosure can be provided or administered in combination with one or more other medicaments or therapeutic methods (e.g., surgery, chemotherapeutic agent, radiation therapy, immune checkpoint inhibitor, or other anticancer agents). In one embodiment, one or more other medicaments or therapeutic methods (e.g., surgery, chemotherapeutic agent, radiation therapy, or other anticancer agents) can be administered after an appropriate period has elapsed from administration of the antigen or cancer vaccine of the present disclosure. When administered separately, two or more medicaments can be provided as a kit. Non-limiting examples of anticancer agents include immune checkpoint inhibitors (PD-1 inhibitors (e.g., anti-PD-1 antibody), PD-L1 inhibitors (e.g., anti-PD-Ll antibody), CTLA-4 inhibitors (e.g., anti-CTLA-4 antibody), and the like), chemotherapeutic agents such as antimetabolites and alkylating agents, growth inhibitors, cytotoxic agents, agents used in radiation therapy, antiangiogenesis agents, apoptosis agents, anti-tubulin agents, anticancer antibiotics, anti-microtubule drugs, tyrosine kinase inhibitors, proteasome inhibitors, anaplastic lymphoma kinase inhibitors, Janus kinase inhibitors, CDK inhibitors, MEK inhibitors, Raf kinase inhibitors, PARP inhibitors, antibody drugs, other molecularly targeted drugs, platinum formulations, immunotherapy such as dendritic cell therapy, gene therapy, other low molecule drugs, other agents for treating cancer, and the like.

The term "carrier" as used herein refers to a pharmaceutically acceptable substance, composition, or excipient such as, for example, a liquid or solid bulking agent, diluent, additive, solvent, or capsule forming agent, which is associated with or enables the transport or carriage of a target pharmaceutical compound from an organ or portion of the body to another organ or portion of the body. "Pharmaceutically acceptable" refers to being compatible with other raw materials in a formulation and being harmless to patients. Non-limiting examples of pharmaceutically acceptable carriers, carriers, and/or diluents can include sugars such as lactose, glucose, and sucrose, starch such as corn starch and potato starch, cellulose and derivatives thereof such as carboxymethylcellulose sodium, ethyl cellulose, and cellulose acetate, excipients such as powdered tragacanth, malt, gelatin, talc, cocoa powder, and suppository wax, oil such as peanut oil, cotton seed oil, safflower oil, sesame oil, olive oil, corn oil, and soybean oil, glycols such as propylene glycol, polyols such as glycerin, sorbitol, mannitol, and polyethylene glycol, esters such as ethyl oleate and ethyl laureate, buffering agents such as agar, magnesium hydroxide, and aluminum hydroxide, alginic acid, pyogenic substance-free water, isotonic saline, Ringer's solution, ethyl alcohol, phosphate buffer, and other nontoxic compatible substances used in a pharmaceutical formulation. A humectant, emulsifier, and lubricant such as sodium lauryl sulfate, magnesium stearate, or polyethylene oxide-polypropylene oxide copolymer, as well as a colorant, releasing agent, coating agent, sweetener, flavoring agent, fragrance, preservative, and antioxidant can also be included in a composition.

As used herein, "parenteral administration" refers to a dosage form for any route that is not oral administration. Any mode for administration in a mode and level that are effective for use in treating or preventing a disease intended for cancer therapy or prevention is employed. Examples of means of parenteral administration include administration through transdermal absorption or transmucosal absorption, as well as injection, infusion, and combinations thereof. For example, administration through transdermal absorption or transmucosal absorption exerts an effect by contacting a transdermally absorbed formulation such as a paste agent, adhesive formulation, or spray with the skin or mucous membrane so that a drug in the formulation migrates into the body through the skin or mucous membrane. Examples of administration via injection or infusion include intravenous, intradermal, subcutaneous, intramuscular, and enteral administration (intestinal infusion), which can also be administered as a bolus and/or sustained infusion. Injection or infusion can use a suspension, liquid agent, emulsion, or implanted agent in an oily or aqueous medium, comprising another formulation substance such as a suspending agent, stabilizer, and/or a dispersant. Enteral administration (intestinal infusion) can provide sustained drug delivery to the proximal small intestine by using a tube or portable infusion pump by percutaneous endoscopic gastrostomy. More preferably, the administration can be subcutaneous or intradermal administration. Parenteral administration (e.g., transdermal administration) can be performed with a tape/patch agent or powder, spray, ointment, paste, cream, lotion, gel, solution, or the like. A composition suitable for parenteral administration can comprise at least one type of a pharmaceutically acceptable aseptic isotonic aqueous or non-aqueous solution, dispersant, suspension, emulsion, implanted agent, or aseptic powder that can be reconstituted in an aseptic injection solution or dispersant immediately before use.

The compositions disclosed herein that are suitable for oral administration can be in a dosage form of a capsule, cachet, pill, tablet, lozenge (generally using a fragrance base tragranth or acacia and sucrose), powder, granule, aqueous or non-aqueous liquid solution, aqueous or non-aqueous liquid suspension, oil-in-water emulsion, water-in-oil emulsion, elixir, syrup, troche (using inactive base such as gelatin, glycerin, sucrose, and/or acacia), and/or mouthwash, which can each comprise a predetermined amount of at least one compound in the present disclosure.

A composition disclosed herein can be administered as a bolus, an electuary or paste.

The antigen or cancer vaccine in the present disclosure can be administered in any dosage form. Any dosage form, whether oral administration or parenteral administration, can be used, as long as the effect can be exerted. Preferably, parenteral administration is used.

A solid dosage form for oral administration (capsule, tablet, pill, sugar coated tablet, powder, granule, or the like) can be mixed with any of one or more of pharmaceutically acceptable carrier such as sodium citrate or dicalcium phosphate, and/or a filler or bulking agent such as starch, lactose, sucrose, glucose, mannitol, and/or silisic acid, binding agent such as carboxymethyl cellulose, alginic acid salt, gelatin, polyvinyl pyrrolidone, sucrose, and/or acacia, a moisturizing agent such glycerol, a disintegrant such as agar, calcium carbonate, potato or tapioca starch, alginic acid, specific silicic acid salt, sodium carbonate, and sodium starch glycolate, a dissolution delaying agent such as paraffin, an absorption promotor such as a quaternary ammonium compound, humectant such as cetyl alcohol, glycerol monostearate, and polyethylene oxide-polypropylene oxide copolymer, an absorbent such as kaolin and bentonite clay, a lubricant such as talc, calcium stearate, magnesium stearate, solid polyethylene glycol, sodium lauryl sulfate, and mixture thereof, and a colorant. For a capsule, tablet, and pill, a pharmaceutical composition can also comprise a buffering agent. A similar type of solid composition can also be used as a filler in a soft and hard filled gelatin capsule using an additive such as lactose and high molecular weight polyethylene glycol.

A liquid dosage form for oral administration can comprise a pharmaceutically acceptable emulsion, microemulsion, solution, suspension, syrup, and elixir. In addition to the active ingredient, a liquid dosage form can comprise an inactive diluent used in conventional art, such as water or other solvent, solubilizing agent, and emulsifying agent, e.g., ethyl alcohol, isopropyl alcohol, ethyl carbonate, ethyl acetate, benzyl alcohol, benzyl benzoate, propylene glycol, 1,3-butylene glycol, oil (especially cotton seed oil, peanut oil, corn oil, germ oil, olive oil, castor oil, and sesame oil), glycerol, tetrahydrofuryl alcohol, polyethylene glycol, sorbitan fatty acid ester, mixture thereof, and the like. Furthermore, a compound can be dissolved using cyclodextrin such as hydroxypropyl-β-cyclodextrin.

The component of the present disclosure can comprise an adjuvant such as a humectant, emulsifying and suspending agent, sweetener, flavoring agent, colorant, fragrance, or preservative. In addition to one or more compounds according to the present disclosure, a suspension can comprise a suspending agent such as ethoxylated isostearyl alcohol, polyoxyethylene sorbitol and sorbitan ester, microcrystalline cellulose, aluminum methhydroxide, bentonite, agar, tragacanth, or mixture thereof.

The composition disclosed herein can be a suppository for rectal or vaginal administration, which can be prepared by mixing one or more compounds according to the present disclosure with one or more suitable non-stimulatory additives or carriers including cocoa butter, polyethylene glycol, suppository wax, salicylate, or the like. The composition is a solid at room temperature, but a liquid at body temperature. Thus, the composition melts in the rectal or vaginal cavity and releases the compound of the present disclosure. A pharmaceutical composition suitable for vaginal administration can comprise a pessary, tampon, cream, gel, paste, foam, or spray formulation comprising a carrier known to be suitable in conventional art.

The dosage form for topical or transdermal administration of the composition of the present disclosure can comprise powder, spray, ointment, paste, cream, lotion, gel, solution, patch, and inhalant. A pharmaceutical composition or pharmaceutical tablet can be mixed with a pharmaceutically acceptable carrier and a preservative, buffering agent, or high pressure gas that may be required under aseptic conditions.

An ointment, paste, cream, and gel can comprise, in addition to the composition of the present disclosure, an additive such as animal or vegetable fat, oil, wax, paraffin, starch, tragacanth, cellulose derivative, polyethylene glycol, silicone, bentonite, silicic acid, talc, zinc oxide, or mixture thereof.

Powder or spray can comprise, in addition to the pharmaceutical composition or pharmaceutical tablet of the present disclosure, an additive such as lactose, talc, silicic acid, aluminum hydroxide, calcium silicate, or polyamide powder, or a mixture thereof. Furthermore, spray can comprise common high pressure gas such as chlorofluorohydrocarbon and volatile unsubstituted hydrocarbon such as butane and propane.

Ophthalmic formulations, optical ointment, powder, solution, and the like are also understood to be within the scope of the present disclosure.

A composition suitable for parenteral administration can comprise at least one type of pharmaceutically acceptable aseptic isotonic aqueous or non-aqueous solution, dispersant, suspension, emulsion, or aseptic powder that can be reconstituted in an aseptic injection solution or dispersant immediately before use.

The term "salt" as used herein includes acid and/or base salt formed with inorganic and/or organic acid and base. As used herein, the term "pharmaceutically acceptable salt" refers to a salt which is suitable for use in contact with tissue of a subject without excessive toxicity, stimulation, allergic reaction, and/or similar events with a reasonable balance of effect/risk ratio within the scope of a definitive medical judgment. Pharmaceutically acceptable salts are well known in the art. For example, pharmaceutically acceptable salts are described in detail in Berge et al., J. Pharmaceutical Sciences (1977) 66: 1-19.

Pharmaceutically acceptable salts can be produced with an inorganic or organic acid. Non-limiting examples of suitable inorganic salts include hydrochloric acid, hydrobromic acid, phosphoric acid, sulfuric acid, and perchloric acid. Non-limiting examples of suitable organic salts include acetic acid, oxalic acid, maleic acid, tartaric acid, citric acid, succinic acid, and malonic acid. Other non-limiting examples of suitable pharmaceutically acceptable salts include adipic acid salt, alginic acid salt, ascorbic acid salt, aspartic acid salt, benzenesulfonic acid salt, besilate, benzoic acid salt, bisulfuric acid salt, boric acid salt, butyric acid salt, camphoric acid salt, camphorsulfonic acid salt, citric acid salt, cyclopentanepropionic acid salt, digluconic acid salt, dodecylsulfuric acid salt, ethanesulfonic acid salt, formic acid salt, fumaric acid salt, glucoheptonic acid salt, glycerophosphoric acid salt, gluconic acid salt, hemisulfuric acid salt, heptanoic acid salt, hexanoic acid salt, hydroiodic acid salt, 2-hydroxy-ethanesulfonic acid salt, lactobionic acid salt, lactic acid salt, lauric acid, lauryl sulfate, malic acid salt, maleic acid salt, malonic acid salt, methanesulfonic acid salt, 2-naphthalenesulfonic acid salt, nicotinic acid salt, nitric acid salt, oleic acid salt, oxalic acid salt, palmitic acid salt, pamoic acid salt, pectinic acid salt, persulfuric acid salt, 3-phenylpropionic acid salt, phosphoric acid salt, picric acid salt, pivalic acid salt, propionic acid salt, stearic acid salt, succinic acid salt, sulfuric acid salt, tartaric acid salt, thiocyanic acid salt, p-toluenesulfonic acid salt, undecanoic acid salt, and valeric acid salt. In some embodiments, examples of organic acids that can produce a salt include acetic acid, propionic acid, glycolic acid, pyruvic acid, oxalic acid, lactic acid, trifluoroacetic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, and salicylic acid.

A salt can be prepared at the time of separation and purification of a disclosed compound or separately by reacting the compound with a suitable base or acid. Non-limiting examples of pharmaceutically acceptable salts obtained from a base include alkali metals, alkali earth metals, ammonium, and N+(C1-4 alkyl)4 salts. Non-limiting examples of suitable alkali or alkali earth metal salts include sodium, lithium, potassium, calcium, magnesium, iron, zinc, copper, manganese, and aluminum salt. Furthermore, non-limiting examples of suitable pharmaceutically acceptable salts optionally include nontoxic ammonium, quaternary ammonium, and amine cation formed using a counter ion such as a halide ion, hydroxide ion, carboxylic acid ion, sulfuric acid ion, phosphoric acid ion, nitric acid ion, lower alkyl sulfonic acid ion, and aryl sulfonic acid ion. Non-limiting examples of suitable organic bases that can produce a salt include primary amine, secondary amine, tertiary amine, substituted amine including naturally-occurring substituted amine, cyclic amine, isopropylamine, trimethylamine, diethylamine, triethylamine, tripropylamine, ethanol amine, and other basic ion exchange resins. In a specific embodiment, a pharmaceutically acceptable base addition salt can be selected from ammonium, potassium, sodium, calcium, and magnesium salt.

In an embodiment of the present disclosure, a target subject can be a patient in a state before onset of cancer, after a cancer treatment, an early stage of onset of cancer, or a precancerous condition. Alternatively, a target subject can be a healthy individual. If a healthy individual is a subject, the disclosure is performed as a preventive method.

Examples of cancer targeted in the present disclosure include, but are not limited to esophageal cancer, gastroesophageal junction cancer, renal cell cancer, lung cancer, digestive organ cancer, leukemia, lymphoma, myeloma, brain cancer, pancreatic cancer, endometrial cancer, prostate cancer, liver cancer, bladder cancer, gastroesophageal adenocarcinoma, chondrosarcoma, colorectal adenocarcinoma, colorectal cancer, breast cancer, renal cell cancer, ovarian cancer, head and neck cancer, melanoma, gastric adenocarcinoma, sarcoma, urogenital cancer, gynecological cancer, and adrenocortical cancer. In a specific embodiment, cancer is colorectal cancer. In a specific embodiment, cancer is colorectal adenocarcinoma. In a specific embodiment, cancer is melanoma. In a specific embodiment, cancer is breast cancer. In a specific embodiment, cancer is bladder cancer. In a specific embodiment, cancer is renal cell cancer. In a specific embodiment, cancer is pancreatic cancer. In a specific embodiment, cancer is endometrial cancer. In a specific embodiment, cancer can be unresectable. In a specific embodiment, cancer can be progressive. In a specific embodiment, cancer can be refractory. In a specific embodiment, cancer can be recurrent. In a specific embodiment, cancer can be metastatic. In various embodiments of the present disclosure, target cancer can include normal carcinoma, carcinoma with a relatively slow progression (e.g., cancer with low sensitivity to the immune system), oral squamous cell cancer, cervical cancer, MHC class I negative carcinoma on which CD8 positive T cells are generally less effective, immune checkpoint inhibitor resistant cancer, and the like. A cancer patient refers to a patient suffering from a "cancer" described above. In one embodiment, the target disease, disorder, or condition of the present disclosure comprises melanoma.

A target subject in the present disclosure can be a subject exhibiting immunological resistance. The present disclosure can also target a subject on which the effect of an immune checkpoint inhibitor is weak, a subject resistant to therapy by chimeric antigen receptor expressing cells, a subject on which an effect of therapy by a monoclonal antibody is not exhibited, and the like.

The present disclosure performs a step of identifying an antigen or a combination of antigens that are responsive to the subject based on the antigen responsiveness profile. In this regard, responsiveness to a subject can be any responsiveness associated with disease therapy or prevention, especially responsiveness suggested to be associated with immunological memory. For example, the responsiveness can include responsiveness to IFN-γ production, IL-2 production, TNF-α production, or responsiveness to any two or three thereof.

In another embodiment, the present disclosure provides a companion diagnostic drug or diagnostic method for use in preventing or treating cancer and a therapeutic or preventive method or a medicament based thereon.

In a specific embodiment, checking the responsiveness is characterized by administering companion diagnosis in advance based on anamnesis and vaccination history. It is demonstrated in the Examples herein that such companion diagnosis is possible. Those skilled in the art can understand that those skilled in the art can similarly administer companion diagnosis in advance for other diseases or disorders based on such an Example or other specific descriptions.

"Administering companion diagnosis in advance based on anamnesis and vaccination history" can be performed herein as follows:
*obtaining an antigen response profile by a non-invasive method, interview, anamnesis or vaccination history based on a Maternal and Child Health Handbook, an equivalent thereof, or the like;
*checking whether there is actually responsiveness using peripheral mononuclear cells isolated a subject and an antigen panel; and
*determining an antigen or a combination thereof exhibiting a reaction above as a prophylactic drug or therapeutic drug.

Examples of such companion diagnosis or therapy include determining whether a human Mycobacterium tuberculosis hot water extract can be used as an antigen component by utilizing tuberculosis infection history as the past physical condition. Although not wishing to be bound by any theory, for example, a subject whose cancer or the like is preventable can be identified using a human Mycobacterium tuberculosis hot water extraction by determination utilizing tuberculosis infection history as the past physical condition, and prevention of cancer can be materialized thereby. Alternatively, for example, a subject whose cancer or the like is preventable can be identified using a human Mycobacterium tuberculosis hot water extraction by determination utilizing tuberculosis infection history as the past physical condition, and cancer therapy can be materialized thereby.

The past physical condition and the component can be one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.

In one embodiment, a subject targeted by the present disclosure is a patient with BCG vaccination history or tuberculosis infection history or a healthy individual confirmed to have antigen responsiveness, and the method of the present disclosure is a personalized therapeutic method. In this regard, the targeted subject can be an individual with vaccination or infection history, a Mycobacterium tuberculosis infection history, or BCG vaccination history.

A Mycobacterium tuberculosis extract that can be used in the present disclosure is prophylactically administered before onset, administered to prevent recurrence after therapy, or administered in an early stage of onset of cancer, and an extract manufactured by the manufacturing method exemplified herein, as well as an extract from Mycobacterium tuberculosis obtained by a manufacturing method improved therefrom, are subcutaneously or intradermally administered in an early stage of onset of cancer or precancerous condition.

In one specific embodiment, the present disclosure provides a method of preventing or treating cancer immunity by administering companion diagnosis in advance based on anamnesis and vaccination history, comprising revaccinating the antigen. Example 2 describes and demonstrates that cancer immunity can be prevented or treated by administering companion diagnosis in advance based on anamnesis and vaccination history.

In a specific embodiment, the present disclosure provides a method of preventing or treating cancer of a subject with a non-tumor component, wherein the component is an antigen or extract identified by interview and/or is identified by reference to the anamnesis or vaccination history of the subject, wherein the subject is an individual with an infection history or a subject confirmed to have vaccination history, based on anamnesis and vaccination history, wherein the component is administered prophylactically before onset, administered to prevent recurrence after therapy, or administered in an early stage of onset of cancer to the subject, and the component is optionally administered in an early stage of onset of cancer or a precancerous condition. Examples of such methods include the method demonstrated in Examples 1, 2, 3, 10, and 13. In the present disclosure, a non-tumor component for use in a method of preventing or treating cancer in a subject is also provided, wherein the component is an antigen or extract identified by interview and/or identified by reference to anamnesis or vaccination history of the subject, wherein the subject is an individual with an infection history or vaccination history described herein, wherein the component is administered prophylactically before onset, administered to prevent recurrence after therapy, or administered in an early stage of onset of cancer to the subject, and the component is optionally administered in an early stage of onset of cancer or a precancerous condition.

In an aspect, the present disclosure provides a vaccine formulation comprising the antigen of any one of the present disclosure and an adjuvant base. In one embodiment, the vaccine formulation is for use in personalized medicine. In this personalized medicine, based on the companion medicine such as those provided in the present disclosure, a vaccine can be provided appropriately for the respective patient or subject. In a specific embodiment of the present disclosure, the cancer targeted by the present disclosure can be normal carcinoma, carcinoma with a relatively slow progression (with low sensitivity to the immune system), oral squamous cell cancer, cervical cancer, MHC class I negative carcinoma on which CD8 positive T cells are generally less effective, or the like. In a specific embodiment, a subject of the present disclosure is a patient exhibiting immunological resistance.

In one embodiment, responsiveness of a subject can be identified using infection history, vaccination history, and responsiveness to IFN-γ production, IL-2 production, TNF-α production using peripheral blood, or any two or three thereof. "Responsiveness to IFN-γ production, IL-2 production, TNF-α production using peripheral blood, or any two or three thereof" can be performed as follows:
*isolating peripheral blood mononuclear cells from blood of a subject;
*culturing peripheral blood monocytic cells in the presence of said substance or PPD (purified protein derivative; purified tuberculin), a stimulant such as a tuberculosis antigen, and a protein transport inhibitor such as brefeldin A or monensin;
*collecting peripheral blood monocytic cells after culturing for a certain period of time, staining with a cell surface marker and intracellular cytokine specific fluorescent labeled antibody, and analyzing the stained cells using flow cytometer; and
*determining, with an increase in various cytokine producing cells as an indicator, with Extract A or tuberculosis antigen stimulation in memory CD4 T cells (CD3 positive CD4 positive CD45RA negative). Extract A is exemplified and described in detail in Example 1 and the like.

In a specific embodiment, the Mycobacterium tuberculosis extract used in the present disclosure is a hot water extract of human Mycobacterium tuberculosis or other extract from Mycobacterium tuberculosis (highly safe extract). A composition used in tuberculin or the like is also preferred.

In one aspect, the present disclosure provides a vaccine formulation comprising the antigen provided in the present disclosure and an adjuvant base (e.g., substance promoting a Th1 immune response). In one specific embodiment, a vaccine formulation is used for personalized medicine. Such personalized medicine provides a suitable vaccine for each patient or subject based on companion medicine such as those provided in the present disclosure. In one embodiment, the medicament or antigen component provided in the present disclosure comprises a human Mycobacterium tuberculosis hot water extract or other extract from Mycobacterium tuberculosis (highly safe extract) or an extracted component or an antigen and an adjuvant base and can be provided as a vaccine formulation. Such a vaccine formulation can be manufactured by mixing each material substance at any concentration. The result thereof is shown in Example 4. In a particular embodiment, the vaccine formulation is for use in a method of personalized medicine.

Examples of adjuvant bases that can be used as a vaccine formulation include the following:
*natural immunoreceptor activating adjuvant base (e.g., microbial membrane derived substance TLR agonist, DNA, RNA, dinucleotide, NOD1, NOD2 agonist);
*adjuvant base containing aluminum such as aluminum hydroxide;
*adjuvant base that can create an emulsion such as ISA51 or ISA720; and
*cytokines (IL-2, IL-12, IFN-α, GM-CSF) containing adjuvant base. Preferably, an adjuvant base comprises a substance promoting a Th1 immune response (e.g., nucleic acid based base such as CpG).

In a specific embodiment, the present disclosure provides a composition for use in treating or preventing a disease, disorder, or condition associated with an immunological abnormality of a subject, the composition comprising an antigen component that is specific in the subject to a component that is different from a causative agent of the disease, disorder, or condition, wherein the disease, disorder, or condition comprises melanoma, the antigen component is a protein, a part thereof, or a peptide, and can elicit an immune response via CD4 positive T cells, and hehehethe cancer comprises cancer that is treatable and preventable with an immune response via CD4 positive T cells,
wherein the subject is checked as to whether the subject can elicit an antitumor immune response via CD4 positive T cells, and if the subject can elicit an antitumor immune response via CD4 positive T cells, the composition is administered. The present disclosure also provides a prophylactic method and therapeutic method associated with the composition.

In another embodiment, the immune response is not mediated through CD8 positive T cells.

In another embodiment, the present disclosure provides a composition for use in treating or preventing cancer or tumor in a subject, the composition comprising a non-tumor antigen component, wherein the non-tumor antigen component activates a regulatory T cell (Treg) having immunological memory of the non-tumor antigen component, which is suppressed in the subject, and wherein the Treg has an effect of promoting regulatory activity or antitumor immunological action on cancer or tumor. The present disclosure also provides a prophylactic method and therapeutic method associated with the composition.

In another aspect, the present disclosure provides a composition for use in treating or preventing a disease, disorder, or condition associated with an immunological abnormality of a subject, wherein the composition comprises an antigen component (the antigen component can be isolated, an extract comprising the same, or another form) that is specific in the subject to a component that is different from a causative agent of the disease, disorder, or condition, and is administered at a suitable dosage and administration (subcutaneously or intratumorally administered once a day (first week) and about once a week (second week and thereafter)). The present disclosure also provides a prophylactic method and therapeutic method associated with the composition. In a certain embodiment, the antigen component is contained at about 0.001 µg ofofofor more per unit formulation.

In another aspect, the present disclosure provides a method of treating or preventing cancer or tumor in a subject, comprising: a) identifying a non-tumor antigen specific to the subject based on an antigen responsiveness profile; b) identifying whether the subject has immunological memory of the non-tumor antigen to identify a subject having the immunological memory; and c) administering the non-tumor antigen to the subject identified as having the immunological memory. The present disclosure can comprise a prophylactic method, therapeutic method, pharmaceutical composition, antigen component, and the like associated with the method.

In one embodiment, the antigen responsiveness profile comprises vaccination history and/or infection history.

In another embodiment, the identifying a subject having the immunological memory comprises stimulating peripheral blood mononuclear cells (PBMC) isolated from the subject or infiltrating immune cells isolated from a tumor mass with the non-tumor antigen, measuring cytokine production, and identifying a subject with an amount of cytokine production increased a predetermined factor compared to the amount before stimulation as a subject having the immunological memory (also referred to as a Responder).

In one embodiment, a non-tumor antigen is administered once a day, three times a week, twice a week, once a week, once every two weeks, or once a month. The dosing can be initially once a day, and then appropriately increased or decreased thereafter, such as once a week from the second dosing.

In another embodiment, the non-tumor antigen in the present disclosure is administered at about 0.1 pg/dose to about 1 mg/dose.

### <Component>

In another aspect, the present disclosure provides a composition for use in treating or preventing a disease, disorder, or condition associated with an immunological abnormality of a subject, comprising mHSP10 and/or MTB12 and/or lipoprotein LpqH. The present disclosure also provides a prophylactic method and therapeutic method associated with the composition.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure described hereinafter is based on the Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. When necessary, animals used in the following Examples were handled in compliance with the institutional guidelines of the National Institute of Biomedical Innovation, Health and Nutrition and other relevant ethical standards and guidelines, based on the Declaration of Helsinki. While the specific products described in the Examples were used, reagents can be substituted with an equivalent product from another manufacturer (Sigma-Aldrich, Wako Pure Chemical, Nacalai Tesque, R & D Systems, USCN Life Science INC, or the like).

### (Manufacturing Example)

Extract A used in this Example was manufactured in the following manner.

Human Mycobacterium tuberculosis strain Aoyama B which had been lyophilized and stored (-20°C) was subjected to seed culture at 37 ± 1°C in a Sauton potato medium⁽¹⁾. The cultured bacteria were transferred to a production medium⁽²⁾ and cultured (primary culture) for 5 to 7 weeks at 37 ± 1°C. The resulting cells were washed with water for injection. To the cells, water for injection was then added in an amount 20-fold of the weight of the wet cells. The mixture was heated at 100°C for 120 minutes to obtain an extract. The extract was filtered with a 0.45 µm-membrane filter and then concentrated under reduced pressure so that the saccharide content (in terms of D-arabinose by the phenol-sulfuric acid method) would be 4.0 to 6.0 mg/ml to obtain a concentrate. Subsequently, in order to remove proteins, 1 w/v% of sulfosalicylic acid was added to the concentrate. The mixture was left standing for 15 to 20 minutes at 10°C or lower. Precipitates were then removed by centrifugation (10°C or lower, 1,150 x G, 10 minutes) to recover the supernatant. The protein concentration of the supernatant was 0.30 mg/ml or lower (Lowry method, in terms of tyrosine). The supernatant was further processed to remove sulfosalicylic acid until the concentration was at or below the detection limit (10 ppm or less, method using ferric chloride solution). The resultant solution was concentrated under reduced pressure so that the saccharide content would be 1.8 to 2.2 mg/ml, and the concentrate was combined with sodium chloride (0.9 w/v%) and cold ethanol at the same volume as the concentrate. The mixture was left standing for 40 hours or longer at 10°C or lower, and then the precipitates (polysaccharide of high molecular weight region) were removed by centrifugation (10°C or lower, 2,040 × G, 10 minutes). Subsequently, the supernatant was combined with four times the amount of cold ethanol, and the mixture was left standing for 40 hours or longer at 10°C or lower and centrifuged (10°C or lower, 2,040 × G, 10 minutes) to recover precipitates. The precipitates were dissolved in water for injection. After the saccharide content was adjusted to 1.8 to 2.2 mg/ml, the solution was filtered with a 0.45 µm membrane filter and sterilized with high pressure steam (121°C, 20 minutes) to prepare an Extract (A) solution.

### (1) Sauton-potato medium

Washed potato slices were soaked in a Sauton medium, sterilized for 15 minutes at 115°C, and then used as a Sauton-potato medium.

### Sauton Medium

L-asparagine (monohydrate) 4.0 g
Citric acid (monohydrate) 2.0 g
Magnesium sulfate (heptahydrate) 0.5 g
Potassium monohydrogenphosphate (anhydride) 0.5 g
Ammonium iron citrate 0.05 g
Glycerol 60 ml

The above ingredients were dissolved in water to prepare a 1,000 ml solution. pH was adjusted to 7.0 to 7.3 by using a sodium hydroxide solution.

### (2): Production medium

L-asparagine (monohydrate) 4.0 g
Citric acid (monohydrate) 2.0 g
Magnesium sulfate (heptahydrate) 0.5 g
Potassium monohydrogenphosphate (anhydride) 0.5 g
Ammonium iron citrate 0.05 g
Glycerol 60 ml

The above ingredients were dissolved in water to prepare a 1,000 ml solution and sterilized with high pressure steam (121°C, 20 minutes). pH was adjusted to 7.0 to 7.3 by using a sodium hydroxide solution.

The physicochemical properties of the resulting Extract A solution were as follows.
(1) Appearance:
   Pale yellow clear liquid
(2) pH:
   4.50-5.30
(3) Protein content:
   3.5 wt.% (as an amino acid) in a lyophilized product
(4) Nucleic acid content:
   0.1 wt.% in a lyophilized product
(5) Primary constituent monosaccharides of polysaccharide:
   Mannose 43.4 wt.%, arabinose 18.2 wt.%, and glucose 10.4 wt.% (hydrolyzed with 2N trifluoroacetic acid for two hours at 100°C, and then subjected to liquid chromatography using 2-cyanoacetamide fluorescent derivative (S. Honda, et al., Anal. Chem., 52, 1079 (1980)).

Extract A prepared by the method described in the Manufacturing Example described above can be appropriately diluted prior to use. In the following Examples, Extract A was diluted 1 to 50,000-fold and adjusted to a suitable concentration for use.

### (Example 1: Examination of correlation between Mycobacterium tuberculosis infection and memory T cells)

In this Example, patients with BCG vaccination history or tuberculosis infection history or a healthy individual confirmed to have responsiveness to an antigen were stimulated with an antigen, and the responses of memory T cells were analyzed to examine the efficacy of personalized therapy.

### (Method)

Frozen human peripheral blood mononuclear cells (PBMCs) were purchased from CTL (US), washed using CTL anti-aggregate wash (CTL Europe, Germany), and suspended in RPMI 1640 (R10) comprising 10% fetal bovine serum (FBS). PBMCs were seeded in a 96 well plate at a concentration of 1 × 10⁶ cells/well and cultured overnight under 37°C, 5% COO₂ conditions. Subsequently, Extract A (100 µg/mL), PPD (3 µg/mL), CMV pp65 overlapping peptides (3 µg/mL), or recombinant Mycobacterium tuberculosis (TB) protein (10 µg/mL each) was added to each culture to stimulate the PBMCs, and 1 µg/mL of anti-CD28 (CD28.2), Golgi stop, and Golgi plug (BD Biosciences) were concurrently added. After 6 hours from the stimulation, the PBMCs were collected and washed with PBS. PBMCs were then stained with Live/Dead fixable blue stain kit (Life technologies) and blocked with Human TruStain FcX (Biolegend), and the surface was stained using the fluorescent labeled antibodies, i.e., anti-CD4 (OKT4), anti-CD8 (RPA-T8), anti-CD45RA (HI100), anti-CCR7 (G043H) antibodies. The PBMCs were then fixed and permeabilized with BD Cytofix/Cytoperm (BD Biosciences), and stained with anti-CD3 (SK7), anti-CD154 (24-31), anti-IL-2 (MQ1-17H12), anti-TNF-a (MAb11), and anti-IFN-γ (4SB3). The stained cells were subjected to flow cytometry analysis using LSRII and FlowJo software (BD Biosciences).

### (Results)

The results are shown in Figure 1. Extract A promoted secretion of Th1 cytokines (IFN-γ, IL-2, and TNF-α) from memory T cells of human PBMCs. IFN-γ secretion was also induced by stimulation with PPD. A strong positive correlation was observed in human PBMCs between Extract A and PPD. Meanwhile, correlation was not found between Extract A and CMV. Furthermore, MHSP10, which is one of the antigens contained in Extract A, induced IFN-γ production and exhibited a positive correlation with Extract A. Correlation was also found between Extract A and PPD and Extract A and MHSP10 for IL-2 and TNF-α production.

### (Discussion)

The presence of memory T cells responsive to Extract A was demonstrated in PBMCs where memory T cells producing Th1 cytokines in response to PPD are present.

### (Example 2: Difference in immunological conditions with respect to antitumor effect of Extract A)

This Example examined the antitumor effects of Extract A using mice with different immunological conditions.

### (Method)

### (BCG infection model)

12 mg of dry BCG vaccine was suspended in 12 mL of saline to prepare 1 mg/mL of BCG vaccine solution. BCG infection models were created by infecting mice with subcutaneous administration of 100 µL of vaccine solution at the base of the tail twice, i.e., 2 weeks before and 5 weeks before transplantation of tumor cells.

### (Extract A emulsion immunized model)

Extract A and Freund's incomplete adjuvant (FIA) were mixed at a volume ratio of 1:1 to prepare an Extract A emulsion using a GP syringe connector (BrightPath Biotherapeutics) and a Luer lock syringe (HENKE SASS WOLF). Extract A emulsion immunized models were created by subcutaneous administration of 100 µL of Extract A emulsion to mice at the base of the tail twice, i.e., 2 weeks before and 4 weeks before transplantation of tumor cells, by using a 1 mL syringe with a 25 G needle.

### (Transplantation of tumor cells)

As for the tumor cells, B16BL6 cells and B16F10 cells were used. As for these cells, cells that were initiated about 1 week before transplantation and passaged two or more times were used. The B16BL6 cells were diluted with PBS to 3 × 10⁶ cells/mL and transplanted into anesthetized naive mice, BCG infected mice, and Extract A emulsion immunized model at a cell count of 3.0 × 10⁵ cells/mouse. Extract A was diluted 10-fold with saline and administered subcutaneously to the right groin at 100 µL per mouse with a 1 mL syringe with a 26 G needle. The same amount of saline was administered to a control animal. Extract A was subcutaneously administered every other day from 8 days before transplantation to dissection.

The three sides (width, length, and height) of tumor were measured with an electronic caliper from day 6 after transplantation. The tumor volume was calculated from the product thereof. Statistical analysis was conducted with Excel (Microsoft). A p-value of 0.05 or less by a t-test when homoscedasticity is not assumed was considered a significant difference.

### (Results)

When Extract A was administered to naive mice, an antitumor effect was not observed on B16BL6 cells or B16F10 cells (Figures 2B and 2E). In contrast, an antitumor effect due to Extract A was observed in BCG inoculated mice (Figures 2C and F). Furthermore, an antitumor effect was observed on both B16BL6 cells and B16F10 cells by administration of Extract A in Extract A emulsion immunized mice (Figures 2D and G).

### (Discussion)

It was elucidated that a change in a mouse due to BCG infection plays an important role in antitumor action of Extract A. Furthermore, the same effect observed in an Extract A emulsion immunized model strongly suggests the relationship with acquired immune response.

### (Example 3: Antitumor effect from administering a model antigen to an animal with model antigen specific memory cells)

This Example examined the effect of suppressing tumor growth after immunization with a model antigen.

### (Method)

### (Effect of suppressing tumor growth after immunization with model antigen)

Mice were immunized by adding 1/50 amount of 10 mg/mL OVA protein solution (final dosage of 20 µg) to prepare an emulsion solution upon emulsion immunity. Subsequently, 2 µg of ovalbumin was administered instead of Extract A. Other methods were carried out in accordance with the method of transplanting tumor cells and Extract A emulsion model in Example 2.

### (Effect of suppressing tumor growth by Th1 introduction)

CD4 T cells and CD11c positive cells were isolated with AutoMACS from OT-II mice (mice expressing TCR that is MHC class II restricted and specific to ovalbumin) or OT-II rag1 knockout (KO) mice and wild-type mice by using CD4 T cell isolation kit (Miltenyi Biotec) or CD11c beads (Miltenyi Biotec), respectively, in accordance with the respective user manual. CD4 T cells and CD11c positive cells were prepared at a concentration of 10⁷ cells and 1 to 2 × 10⁶ cells per 1 mL, respectively, and cultured after adding 10 µg/mL of anti-IL-4 antibodies, 7.5 ng/mL of IL-12p70, 10 ng/mL of IL-2, and 10 µg/mL of OVA₃₂₃₋₃₃₉ peptide. The medium was exchanged after 3 to 4 days of culturing, and each cell was collected after 6 to 7 days. Dead cells were removed by centrifugation using Lymphoryte (Cedarlane).

CD4 T cells from an OT-II mouse that differentiated into Th1 cells in vitro were introduced into Rag2 KO mice or Rag2, IL-2 receptor γ chain double KO mice. Ovalbumin was administered every other day until the day of dissection for a total of 11 to 15 times. On day 8 or 16 after transplantation of OT-II mouse derived cells, B16BL6 cells were transplanted, and the change in tumor volume was measured over time.

### (Results)

### (Tumor growth suppression effect after immunization with model antigen)

Tumor growth suppression action was observed after ovalbumin administration in mice immunized with an emulsion comprising ovalbumin (Figure **3-1).**

### (Tumor growth suppression effect due to ovalbumin administration to ovalbumin specific Th1 introduced mice)

When CD4 T cells separated from an OT-II mouse and differentiated in vitro into Th1 cells were transplanted into Rag2 KO mice, tumor growth was found to be suppressed by the administration of ovalbumin (p = 0.017, left side of Figure **3-2****).** Tumor growth was also found to be suppressed by the administration of ovalbumin for Rag2, IL-2 receptor γ chain double KO mice (p = 0.09, right side of Figure **3-2****).**

### (Example 4: Antitumor action of vaccine formulation combining various adjuvant bases)

This Example examined the antitumor action of a vaccine formulations combining various adjuvant bases.

### (Method)

Mice were immunized by administering Extract A combined with FIA, K3-SPG, K3-cGAMP, and K3-Alum adjuvants to the mice. Saline combined with PBS, FIA, K3-SPG, and K3-cGAMP adjuvant alone was administered to a control mouse. In this regard, each adjuvant was prepared at a dose comprising K3 at a final dosage of 10 µg, K3-SPG at a final dosage of 5 µg, and 2,3 cGAMP at a final dosage of 10 µg. An administered solution was prepared to comprise 50% or 25% of each of FIA and alum (alhydrogel, InvivoGen) in a 100 µL solution. Extract A was diluted to 1 mg/mL with PBS, and 100 µL was intradermally administered (id) to the base of the tail. Tumor cells were transplanted, and the weight of tumor in mice on the day of dissection was measured by the same method in (Extract A emulsion immunized model) and (Transplantation of tumor cells) of Example 2.

### (Results)

The results are shown in Figure **4****.** It was demonstrated that antitumor action of Extract A is exerted when Extract A is administered after treatment with Extract A emulsion or Extract A combined with an existing adjuvant.

### (Example 5: Evaluation of effect on tumor infiltrating lymphocytes (TIL) by flow cytometry)

This Example evaluated the effect of Extract A on tumor infiltrating lymphocytes (TIL) in transplanted tumor by flow cytometry.

### (Method)

Mice were infected with BCG, and 2 × 10⁵ B16BL6 cells were subcutaneously transplanted to the mice after 5 weeks from BCG infection. In this regard, Extract A or saline was subcutaneously administered every other day from 8 days before tumor cell transplantation. The mice were euthanized to extract the tumor after 14 days from tumor cell transplantation. After dispersing the tumor using a Tumor dissociation kit (Miltenyi Biotec), tumor cells and lymphocytes were separated by density gradient centrifugation. After washing the separated lymphocytes, dead cells were stained and Fc receptors were blocked. After staining the cell surface with various fluorescent labeled antibodies, cells were fixed and permeabilized, and stained inside with fluorescently labeled antibodies.

### (Results)

The results are shown in Figure **5****.** The T-bet positive cell count in the tumor was significantly increased in mice administered with Extract A after BCG infection compared to mice administered with saline.

### (Example 6: Evaluation of effect on tumor infiltrating lymphocytes (TIL) by flow cytometry)

This Example evaluated the effect of Extract A on tumor infiltrating lymphocytes (TIL) in transplanted tumor by flow cytometry.

### (Method)

BCG infected mice were administered with Extract A or saline, and tumor cells were transplanted and the tumor was extracted by the same method as Example 5. After dispersing tumor using a Tumor dissociation kit (Miltenyi Biotec), tumor cells and lymphocytes were separated by density gradient centrifugation. Each antigen and Brefeldin A were added to the culture of separated lymphocytes, which was cultured overnight under 37°C, 5% carbon dioxide gas conditions. After collecting and washing the cells, dead cells were stained and Fc receptors were blocked. After staining the cell surface with various fluorescent labeled antibodies, cells were fixed and permeabilized, and stained inside with fluorescently labeled antibodies.

### (Results)

The results are shown in Figure **6****.** The IFN-γ expressing T-bet positive cell count significantly increased in tumor tissue in mice administered with Extract A after BCG infection compared to mice administered with saline.

### (Example 7: Companion therapy/diagnosis)

Companion therapy/diagnosis can be administered as follows:
*obtaining an antigen response profile by a non-invasive method, interview, anamnesis or vaccination history based on a Maternal and Child Health Handbook, or an equivalent thereof, or the like;
*checking whether there is actually any response to using peripheral mononuclear cells isolated from a subject and an antigen panel; and
*determining an antigen or a combination thereof exhibiting a reaction above as a prophylactic drug or therapeutic drug.

### (Example 8: Identification of cells that are essential for antitumor effect due to Extract A)

This Example identified cells that are essential for an antitumor effect due to Extract A.

### (Method)

### (Administration of Extract A to CD4 deficient mice)

Figure **7A** shows the administration schedule for BCG, B16BL6 cells, Extract A, anti-CD4 antibody, and anti-IFN-γ antibody. Specifically, BCG was injected into mice 35 and 14 days before tumor inoculation, and then 3.0 × 10⁵ B16BL6 melanoma cells were inoculated. Extract A was subcutaneously administered every other day from 8 days before tumor inoculation. In an experiment for depleting CD4 positive cells, 200 µg of anti-CD4 antibody was intraperitoneally injected 1 day before, 3 days after, 7 days after, and 11 days after tumor inoculation. In an experiment for depleting CD8, 200 µg of anti-CD8 antibody was intraperitoneally injected 1, 2, and 3 days before, and 2, 5, 8, and 11 days after tumor inoculation. In an experiment for depleting IFN-γ, 200 µg of anti-IFN-γ antibody was intraperitoneally injected immediately before, and 3, 6, 9, and 12 days after tumor inoculation. Naive mice, CD4 deficient mice, and MHC class II deficient mice were used as the mice. Equal amount of saline was administered to a control animal. The tumor volume was measured by the same method as Example 2 or the like.

### (Measurement of intracellular cytokine)

Mice were infected with BCG, and then saline or Extract A was administered every other day from 8 days before slaughtering. The spleen was isolated from the mice and ground and centrifuged to obtain a pellet of cells. After 15 minutes of suspending the cell pellet in Pharm lysis buffer (BD), the cell suspension was centrifuged and suspended in R10. The cell count was counted with a Z1 particle counter (Beckman Coulter) and adjusted to 2 × 10⁷ cells/mL with R10. 100 µL of the cell suspension was dispended into a 96-well round bottom microplate (Asahi Techno Glass). The cells were stimulated with 100 µL of R10 comprising Extract A. After 30 minutes, Golgi Plug (BD Biosciences) and Golgi Stop (BD Biosciences) were added. The cells were collected after about 6 hours and washed with PBS. The cells were stained with a Live/Dead fixable blue stain kit and blocked with anti-mouse CD16/32 antibodies (Biolegend), and then fixed and permeabilized with BD Cytofix/Cytoperm. Antibodies to IFN-γ (XMG1.2), antibodies to IL-13 (eBio13A), and antibodies to IL-17 (TC11-18H10.1) were used for staining. The stained cells were analyzed by flow cytometry using LSRII and FlowJo software (BD Biosciences).

### (Results)

### (Administration of Extract A to CD4 deficient mice)

Administration of Extract A did not exhibit an antitumor effect in mice depleted with CD4 positive cells using anti-CD4 antibodies (Figure **7B****).** The need for CD4 T cells with respect to an antitumor effect of Extract A was also confirmed in an experiment using CD4 deficient mice (Figure **7C****)** and an experiment using MHC class II deficient mice (Figure **7D****).** Meanwhile, an antitumor effect due to Extract A was also observed in mice depleted with CD8 positive cells using anti-CD8 antibodies (Figure **8C****)** and Batf3 deficient mice that are missing CD8 positive DC (Figure **8G****),** thus revealing that CD8 positive cells are not essential for the antitumor effect due to Extract A.

### (Measurement of intracellular cytokines)

When Extract A was administered to BCG infected mice, the ratio of IFN-γ producing cells in memory CD4 T cells significantly increased. Such a change was not observed in IL-17 or IL-13 (Figure **7E****).** Furthermore, the antitumor effect due to Extract A disappeared in mice administered with IFN-γ neutralizing antibodies, suggesting that IFN-γ plays an important role in the antitumor effect due to Extract A (Figure **7F****).** Furthermore, secretion of IFN-γ from splenocytes of BCG infected mice induced by Extract A was eliminated in each of CD4 deficient mice and MHC class II deficient mice (Figure **7G****).** Meanwhile, production of IFN-γ was maintained in CD1d1 deficient mice (Figure **7H****).** Furthermore, IFN-γ secretion in the spleen in the response to Extract A was completely eliminated when CD4 positive cells were removed from splenocytes (Figure **71).** These results suggest that CD4 T cells change to Th1, and these cells are the primary source of IFN-γ in BCG infected mice induced by Extract A.

### (Example 9: Experiment in subcutaneous injection experiment model)

This Example shows analysis of an antitumor effect in a subcutaneous injection experiment model.

### (Method)

Figure **8A** shows the schedule of an intratumor injection experiment. Specifically, BCG was inoculated to Rag2 deficient mice, CD1d1 deficient mice, FcR deficient mice, IL12p40 deficient mice, and Batf3 deficient mice 35 and 14 days before tumor cell inoculation. Extract A was subcutaneously administered every other day from 8 days before tumor inoculation. In the experiment corresponding to Figure **8C****,** an anti-CD8 antibody was administered 3, 2, and 1 day before, and 3, 7, and 11 days after tumor inoculation. In the experiment corresponding to Figure **8E****,** an anti-NK1.1 antibody was administered 1 day before, and 3, 7, and 11 days after tumor inoculation. The tumor volume was measured thereafter in accordance with the method described in Example 2.

### (Results)

An antitumor effect for BCG infection due to Extract A was not observed in Rag2 deficient mice infected with BCG (Figure **8B****).** An antitumor effect from administration of Extract A was observed in mice depleted with CD8 positive cells (Figure **8C****).** Furthermore, antitumor activity was also observed in Batf3 deficient mice (Figure **8G****),** showing that CD8 positive cells are not essential for antitumor activity. Since antitumor activity due to Extract A was retained in FcR deficient mice and mice depleted with NK1.1 positive cells, it was demonstrated that ADCC activity is not essential for the antitumor effect due to Extract A (Figures **8D** and **8E****).** To study the contribution of NKT cells, the effect was also evaluated in CD1d1 deficient mice, and an antitumor effect due to Extract A was also observed in these mice (Figure **8D****).** Therefore, it was confirmed that CD4 T cells are essential for an antitumor effect due to Extract A, but CD8 T cells, NKT cells, and ADCC activity are not essential for an antitumor effect.

### (Example 10: Experiment of injecting a tumor unrelated protein into pre-immunized mice)

This Example shows antitumor effect on BCG infected mice when an unrelated tumor protein contained in Extract A was administered.

### (Method)

### (Protease treatment of Extract A)

As a protease, an EDTA-0.5% trypsin solution (Nacalai Tesque) and subtilisin (P5380) (Sigma-Aldrich) were used. These proteases were heat inactivated (HI) by incubating at 96°C for 15 minutes. Proteases subjected to HI treatment and proteases not subjected to HI treatment were mixed with Extract A and incubated for 16 hours. These solutions were then incubated at 96°C for 15 minutes, and active enzymes were inactivated.

### (Antitumor activity and IFN-γ secretion due to Extract A subjected to protease treatment)

IFN-γ secretion from splenocytes was induced in BCG infected mice with saline, Extract A, Extract A treated with subtilisin (Sub), Extract A treated with heat inactivated subtilisin (HI-Sub), Extract A treated with trypsin (Trp), or Extract A treated with heat inactivated trypsin (HI-Trp). Each protease was used at 4 different concentrations. The amount of IFN-γ by each stimulation was divided by the amount of IFN-γ secreted by stimulation of Extract A.

### (Antitumor activity by LpqH)

Extract A was subcutaneously administered to BCG infected mice every other day from 8 days before tumor cell transplantation. The mice were euthanized after 14 days from tumor cell transplantation, and tumor was extracted. A small piece of tumor was digested using a Tumor dissociation kit (Miltenyi Biotec). After digestion, a fragment of tumor was crushed with a 70 µm mesh. Density gradient centrifugation was performed using Lympholyte-M (Cedarlane) to remove tumor cells, red blood cells, and dead cells. The intermediate layer after centrifugation was collected and used as TIL. LpqH (10 µg/mL) was then added, and Golgi Plug and Golgi Stop were added therewith. After 10 hours, the cells were collected and washed with PBS. The cells were stained with a Live/Dead fixable blue stain kit and blocked with anti-mouse CD16/32 antibodies (Biolegend), and then the cells were fixed and permeabilized with BD Cytofix/Cytoperm. Antibodies to IFN-γ (XMG1.2) were used for staining, and the stained cells were analyzed by flow cytometry using LSRII and FlowJo software (BD Biosciences).

Saline, 0.1 µg of LpqH, or 1 µg of LpqH were subcutaneously administered to a naive mouse and BCG infected mouse every other day from 8 days before tumor inoculation. The tumor volume was then measured using an electronic caliper in accordance with the method described in Example 2 or the like.

### (Results)

Protease treatment, unlike heat inactivated protease treatment, tended to reduce IFN-γ secretion activity due to Extract A (Figure **9A****).** At least two types of proteins MTB12 and LpqH were identified as a result of analyzing the treated Extract A by mass spectrometry.

When BCG infected mice were stimulated with LpqH, IFN-γ secretion was induced (Figure **9B****).** Injection of a recombinant LpqH protein resulted in an antitumor effect in BCG infected mice that is similar to the antitumor effect due to Extract A (Figure **9C****).** In contrast, an antitumor effect from injection of LpqH protein was not observed in a naive mouse (Figure **9C****).** These results suggest that tumor growth can be suppressed by injection of an antigen to a BCG infected mouse, and one of the active ingredients of Extract A is a protein.

### (Example 11: Analysis of correlation between antitumor effect due to Extract A and tumor infiltrating lymphocytes)

This Example studied the tumor microenvironment after administration of Extract A and analyzed the correlation with tumor infiltrating lymphocytes.

### (Method)

### (Separation of TIL (tumor infiltrating lymphocytes))

Extract A was subcutaneously administered to BCG infected mice every other day from 8 days before tumor cell transplantation. The mice were euthanized after 14 days from tumor cell transplantation, and tumor was extracted. A small fragment of tumor was digested using a Tumor dissociation kit (Miltenyi Biotec). After digestion, a fragment of tumor was crushed using a 70 µm mesh. Density gradient centrifugation was performed using Lympholyte-M (Cedarlane) to remove tumor cells, red blood cells, and dead cells. The intermediate layer after centrifugation was collected and used as TIL. TIL was stained using anti-CD45 antibodies (30-F11, BD), anti-CD62L antibodies (MEL-14, BD), anti-FOXP3 antibodies (FJK-16S, ebioscience), or anti-T-bet antibodies (4B10, Biolegend). Antibodies to FOXP3 and T-bet were stained after permeabilization using BD Pharmingen Transcription Factor buffer (BD). The stained cells were analyzed with LSRII (BD) and FlowJo software.

### (Results)

Administration of Extract A to a BCG infected mouse significantly increased the CD3 positive CD45 positive cell count in the tumor microenvironment, and a significant negative correlation between tumor weight and tumor infiltrating CD3 positive CD45 positive cells was found (Figure **10A****).** A positive correlation between tumor weight and TIL count was observed in BCG infected mice (Figure **10B****).** When cells with an increased ratio in the tumor microenvironment was investigated, the ratio of CD4 positive cells to CD3 positive CD45 positive cells increased, while the ratio of CD8 positive cells to CD3 positive CD45 positive cells did not have such a change (Figure **10C****).** When the T-bet positive cell and Foxp3 positive cell count was measured while focusing on the phenotype of CD4 positive T cells, it was found that the T-bet positive cell count had significantly increased. The ratio of T-bet positive Foxp3 negative cells and T-bet positive CD4 positive T cells increased in the tumor microenvironment, and a significant negative correlation between tumor weight and tumor infiltrating T-bet positive CD4 positive cell count was observed (Figure **10E****).**

### (Example 12: Analysis of correlation between antitumor effect due to Extract A and TIL producing IFN-γ)

This Example analyzed the correlation between the antitumor effect due to Extract A and TIL producing IFN-γ.

### (Method)

Intracellular IFN-γ with or without stimulation by Extract A was measured in accordance with the experimental procedure described in Figure **11A****.** Isolated TIL was seeded on a culture plate, and an antigen and a protein transport inhibitor were added. After overnight culture, TIL was collected and analyzed by FACS.

### (Results)

It was demonstrated that IFN-γ producing CD4 positive memory T cells are detected under no stimulation and induced Extract A antigen independently (Figures **11B** and **11D****).** Under this experimental condition, tumor derived cells and fragments thereof are preferably contained in a medium. Thus, there is a possibility that a T cell response to a tumor antigen is detected. Furthermore, it was revealed that CD4 positive T cells which can produce IFN-γ in response to Extract A and LpqH are present in tumor tissue of BCG infected mice and Extract A immunized mice (Figures **11C, 11E,** and **11F**).

### (Example 13: Antitumor effect from influenza vaccine)

This Example investigated an antitumor effect of influenza vaccines depending on the difference in immunological conditions.

### (Method)

### (PR8 infection model)

Influenza virus PR8 was suspended in PBS so that the concentration would be 10 pfu/mL to prepare a viral solution. A mouse was infected through intranasal administration of 30 µL of viral solution to prepare a PR8 infected model. The mouse was infected with a virus under anesthesia.

### (Administration of influenza vaccine)

An influenza vaccine was adjusted with saline so that the concentration would be 1 µg/mL, and 100 µL was subcutaneously administered to the right inguinal region of a mouse.

### (Results)

When the influenza vaccine was administered to a naive mouse, an antitumor action was not observed against a transplanted B16BL6 cell (Figure **12B**). In contrast, when an influenza vaccine was administered to a mouse infected with influenza, an antitumor action was observed (Figure **12C**).

### (Discussion)

Since a vaccine which is an influenza virus antigen is administered for a change in a mouse from an influenza virus infection, it is suggested that acquired immunity to a non-tumor antigen contributes to an antitumor action. This suggests that even when infected with a pathogenic microorganism other than tuberculosis, acquired immunity induced by various infections, which is not limited to tuberculosis as an antigen associated with the pathogen, exerts an antitumor action by re-exposure to the same pathogenic antigen.

### (Example 14: Prevention, prevention of recurrence, and therapy of cancer)

This Example performs clinical tests on prevention, prevention or recurrence, and/or therapy of cancer.

### (Method)

### (Identification of non-tumor antigen)

See Figure **13A** for the protocol of this Example.

Peripheral blood mononuclear cells (PBMCs) are isolated from subjects with cancer remission and/or post-surgery subjects, and normal subjects. An antigen responsiveness profile is identified by checking vaccination history and/or infection history of the subject. Examples of vaccination history and infection history include, but are not limited to, tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza (virus), MARS, rabies, diphtheria, and the like.

The isolated PBMC (e.g., 1.0 × 10⁴ to 1.0 × 10⁶ cells) are stimulated for about 6 to 24 hours with the non-tumor antigen described above (0.01 µg/mL to 1 mg/mL) or an extract comprising a non-tumor antigen (0.01 µg/mL to 1 mg/mL), and production of cytokine (e.g., IFN-γ, IL-2, and/or TNF-α) is measured by a detection method that is commonly used in the art (e.g., ELISA, qPCR, and/or FACS). Subjects with amount of cytokine production after stimulation that is twice or more of the amount of cytokine production before stimulation are selected as responders.

### (Administration of non-tumor antigen)

An identified non-tumor antigen is administered in accordance with the following dosing regimen to evaluate clinical efficacy.
*Group composition: placebo or non-tumor antigen (about 0.001 µg/dose to about 1 mg/dose) or an extract comprising a non-tumor antigen (about 0.001 µg/dose to about 1 mg/dose in terms of non-tumor antigen): 2 doses
*Number of subjects: about 100
*Route of administration: SC (subcutaneous administration) or IT (intratumor administration)
*Number of administrations: about once a day (first week) and about once a week (second week and thereafter)
*Administration period: about 3 months to about 1 year
*Primary endpoints: progression free survival (PFS) and overall survival (OS)
*Secondary endpoints: duration of response, disease control rate, and safety

### (Results)

Both the primary endpoints and secondary endpoints were more significant in the group administered with a non-tumor antigen in comparison to a placebo. Examples with a response within the range of tested doses can be observed.

### (Example 15: Prevention of recurrence of cancer)

This Example performs a clinical test on prevention of recurrence of cancer in a cancer patient.

### (Method)

### (Identification of non-tumor antigen)

See Figure **13B** for the protocol.

Tumor mass is isolated from a post-surgery cancer patient. In this Example, an antigen responsiveness profile is identified by checking vaccination history and/or infection history of the cancer patient described above for immune cells infiltrating into tumor mass. Vaccination history and infection history can comprise, but are not limited to, tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza (virus), MARS, rabies, diphtheria, and the like.

The tumor infiltrating immune cell described above (e.g., 1.0 × 10⁴ to 1.0 × 10⁶ cells/sample) are stimulated for about 6 to 24 hours with the non-tumor antigen described above (0.01 µg/mL to 1 mg/mL) or an extract comprising a non-tumor antigen (0.01 µg/mL to 1 mg/mL), and production of cytokine (e.g., IFN-γ, IL-2, and/or TNF-α) is measured by a detection method that is commonly used in the art (e.g., ELISA, qPCR, and/or FACS). Subjects with the amount of cytokine production after stimulation that is twice or more of the amount of cytokine production before stimulation are selected as responders.

### (Administration of non-tumor antigen)

An identified non-tumor antigen is administered in accordance with the following dosing regimen to evaluate clinical efficacy.
*Group composition: placebo or non-tumor antigen (about 0.001 µg/dose to about 1 mg/dose) or extract comprising a non-tumor antigen (about 0.001 µg/dose to about 1 mg/dose in terms of non-tumor antigen): two doses
*Number of subjects: about 100
*Route of administration: SC (subcutaneous administration) or IT (intratumor administration)
*Number of administrations: about once a day (first week) and about once a week (second week and thereafter)
*Administration period: about 3 months to about 1 year
*Primary endpoints: progression free survival (PFS) and overall survival (OS)
*Secondary endpoints: duration of response, disease control rate, and safety

### (Results)

Both the primary endpoints and secondary endpoints were more significant in the group administered with a non-tumor antigen in comparison to a placebo. Examples with a response within the range of tested doses can be observed.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2019-148551 filed on August 13, 2019 with the Japan Patent Office, PCT/JP2019/047945 submitted to the International Bureau on December 6, 2019, and 108144810 filed on December 6, 2019 with the Taiwan Intellectual Property Office. It is understood that the entire content thereof is incorporated herein by reference in the same manner as if the contents are specifically described herein in its entirety.

### [Industrial Applicability]

The present disclosure provides a method of preventing or treating a disease such as cancer based on a conventionally unavailable mechanism.

## Claims

1. A composition for use in activating a regulatory T cell (Treg) having immunological memory of a non-target antigen component, which is suppressed in a subject, against a target, comprising the non-target antigen component.

2. The composition of claim 1, wherein the activation of Treg imparts an ability to kill the target or an immunostimulatory action against the target.

3. A composition for use in activating a regulatory T cell (Treg) having immunological memory of a non-tumor antigen component, which is suppressed in a subject, comprising the non-tumor antigen component.

4. The composition of claim 3, wherein the activation of Treg imparts an ability to kill tumor or an immunostimulatory action against tumor.

5. The composition of any one of claims 1 to 4, wherein the Treg is a memory T cell.

6. The composition of any one of claims 1 to 5, wherein the Treg is CD4 positive.

7. The composition of any one of claims 1 to 6, wherein the antigen component comprises a protein.

8. The composition of any one of claims 1 to 7, wherein the antigen component comprises an antigen selected from the group consisting of a pathogen of an infection or a part thereof, an antigen associated with anamnesis, and an antigen associated with vaccination history.

9. The composition of any one of claims 1 to 8, wherein the antigen component comprises a human Mycobacterium tuberculosis hot water extract or an influenza virus antigen.

10. A composition **characterized in that** the composition is used in a method comprising checking whether the antigen component has immunological memory of Treg in the subject, and administering the antigen component to the subject if the subject has immunological memory for the antigen component.

11. A composition for use in treating or preventing a disease, disorder, or condition associated with an immunological abnormality of a subject, the composition comprising an antigen component that is specific in the subject to a component that is different from a causative agent of the disease, disorder, or condition.

12. The composition of claim 11, wherein the disease, disorder, or condition comprises cancer, wherein, preferably, the antigen component is a non-tumor antigen component.

13. The composition of claim 11 or 12, wherein the antigen component is specific to a memory T-cell of the subject.

14. The composition of claim 13, wherein the memory T cell is a memory regulatory T cell (IL-2 producing).

15. The composition of any one of claims 11 to 14, wherein the antigen component has an immunostimulatory action.

16. The composition of any one of claims 11 to 15, wherein the antigen component antigen-dependently acts on memory CD4 positive T cells.

17. The composition of any one of claims 11 to 16, wherein the antigen component has activity to bias a ratio of presence between Foxp3 positive Treg cells and IFN-γ producing T cells.

18. The composition of claim 17, wherein the bias is an increase in IFN-γ producing T cells compared to Foxp3 positive Treg cells.

19. The composition of any one of claims 11 to 18, wherein the antigen component has activity to bias a ratio of presence between Foxp3 positive Treg cells and type 1 helper T cells.

20. The composition of claim 17 or 18, wherein the IFN-γ producing T cells comprise type 1 helper T cells.

21. The composition of any one of claims 11 to 20, wherein the antigen component has activity to bias a ratio of presence of Th1 cells.

22. The composition of any one of claims 17 to 21, wherein the IFN-γ producing T cells are T-bet positive Th1 cells.

23. The composition of any one of claims 11 to 22, wherein the antigen component that is specific has a capability to enhance at least one selected from the group consisting of IFN-γ producing capability, IL-2 producing capability, and TNF-α producing capability in a sample from the subject.

24. The composition of any one of claims 1 to 23, wherein the antigen component is a protein.

25. A biomarker for determining whether a non-tumor antigen component has anticancer action on a subject, the biomarker comprising at least one selected from the group consisting of whether the antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.

26. A composition or a kit comprising an agent or means for detecting a biomarker for determining whether a non-tumor antigen component has anticancer action on a subject, the biomarker comprising at least one selected from the group consisting of whether the non-tumor antigen component (i) antigen-dependently acts on memory CD4 positive T cells, (ii) alters memory regulatory T cells, (iii) alters IFN-γ producing capability, (iv) alters IL-2 producing capability, and (v) alters TNF-α producing capability.

27. The composition of any one of claims 1 to 23, wherein the (non-tumor) antigen component comprises an antigen associated with anamnesis or vaccination history.

28. The composition of any one of claims 1 to 23, wherein the subject is a subject with a history of an infection, and the antigen component comprises an antigen to the infection.

29. The composition of claim 28, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpes type 1 virus, EBV/Epstein Barr Virus (herpesvirus 4), CMV/cytomegalovirus (herpesvirus 5), influenza virus, MARS, rabies, and diphtheria.

30. The composition of any one of claims 1 to 23 and 27 to 29, wherein the subject is a subject with BCG vaccination history, tuberculosis infection history, or antigen responsiveness to Mycobacterium tuberculosis, and the antigen component comprises a human Mycobacterium tuberculosis hot water extract.

31. The composition of any one of claims 1 to 23 and 27 to 29, wherein the subject is a subject with influenza vaccination history, influenza infection history, or antigen responsiveness to an influenza virus, and the antigen component comprises an influenza virus.

32. The composition of any one of claims 1 to 23 and 27 to 31, wherein the disease, disorder, or condition comprises melanoma.

33. The composition of any one of claims 1 to 23 and 27 to 32, wherein the antigen component comprises a protein, a part thereof, or a peptide.

34. The composition of any one of claims 1 to 23 and 27 to 33, wherein the antigen component comprises a component that can elicit an immune response via CD4 positive T cells.

35. The composition of any one of claims 1 to 23 and 27 to 34, wherein the subject is checked as to whether the subject can elicit an antitumor immune response via CD4 positive T cells, and if the subject can elicit an antitumor immune response via CD4 positive T cells, the composition is administered.

36. A composition for use in treating or preventing a disease, disorder, or condition associated with an immunological abnormality of a subject, the composition comprising an antigen component that is specific in the subject to a component that is different from a causative agent of the disease, disorder, or condition, wherein
the disease, disorder, or condition comprises melanoma,
the antigen component is a protein, a part thereof, or a peptide, and can elicit an immune response via CD4 positive T cells, and
the cancer comprises cancer that is treatable and preventable with an immune response via CD4 positive T cells,
wherein the subject is checked as to whether the subject can elicit an antitumor immune response via CD4 positive T cells, and if the subject can elicit an antitumor immune response via CD4 positive T cells, the composition is administered.

37. A composition for use in treating or preventing cancer or tumor in a subject, the composition comprising a non-tumor antigen component, wherein the non-tumor antigen component activates a regulatory T cell (Treg) having immunological memory of the non-tumor antigen component, which is suppressed in the subject, and wherein the Treg has an effect of promoting regulatory activity or antitumor immunological action on cancer or tumor.

38. A method of manufacturing or otherwise providing a composition for use in preventing or treating cancer of a subject, the method comprising:
A) identifying a non-tumor antigen specific to the subject;
B) identifying whether a subject has immunological memory of the non-tumor antigen, and selecting a non-tumor antigen for which the subject has the immunological memory; and
C) manufacturing or otherwise providing the selected non-tumor antigen.

39. The method of claim 38, having one or more features in any one of claims 2 to 37 in B).

40. A method of determining whether a non-tumor antigen of a subject can prevent or treat cancer of the subject, the method comprising:
B) identifying whether the subject has immunological memory of the non-tumor antigen, and identifying that cancer of the subject can be prevented or treated if the subject has the immunological memory.

41. The method of claim 40, having one or more features in any of claims 2 to 37 in B).

42. A method for use in preventing or treating a disease, disorder, or condition associated with an immunological abnormality, comprising:
a) obtaining an antigen responsiveness profile of a subject;
b) identifying an antigen component or a combination of antigen components from the antigen responsiveness profile, wherein the antigen component or combination of antigen components has shown or shows to present immune response to the subject; and
c) administering to the subject the antigen component or combination of antigen components identified in step b) at a sufficient amount to elicit an immune response in the subject.

43. The method of claim 42, wherein the disease, disorder, or condition comprises cancer.

44. The method of claim 42 or 43, wherein the obtaining an antigen responsiveness profile comprises checking a past physical condition of a subject, checking whether one or more of candidate antigens has responsiveness in a sample from the subject, or both.

45. The method of any one of claims 42 to 44, wherein the obtaining an antigen responsiveness profile comprises identifying an antigen component or a combination of antigen components that elicit an immune response via CD4 positive T cells.

46. The method of any one of claims 42 to 44, wherein
i) the antigen responsiveness profile comprises at least one selected from the group consisting of interview, anamnesis or vaccination history based on a Maternal and Child Health Handbook, an equivalent thereof or the like, and a combination thereof, and/or
ii) the checking whether one or more of candidate antigens has responsiveness comprises collecting a bodily fluid (e.g., blood) from the subject and separating peripheral blood cells, and then measuring whether the peripheral blood cells produce a cytokine in response to an antigen associated with the antigen profile, and other biomarkers.

47. The method of any one of claims 42 to 46, further comprising periodically testing responsiveness of the antigen and confirming that responsiveness is maintained.

48. The method of any one of claims 42 to 47, wherein a) and b) are performed with the following steps:
i) obtaining a past physical condition of a subject;
ii) collecting blood from the subject and separating peripheral blood cells, and then measuring whether the peripheral blood cells produce a cytokine in response to an antigen corresponding to the physical condition, and other biomarkers; and
iii) identifying a suitable antigen component or combination of antigen components from a result of ii).

49. The method of claim 48, wherein the past physical condition comprises anamnesis and vaccination history.

50. The method of claim 48 or 49, wherein the physical condition comprises history of infection, and the cancer vaccine comprises an antigen component or a combination of antigen components to the infection.

51. The method of claim 50, wherein the infection comprises at least one selected from the group consisting of tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.

52. The method of any one of claims 48 to 51, wherein the physical condition comprises BCG vaccination history, tuberculosis infection history, or antigen responsiveness to Mycobacterium tuberculosis, and the antigen component or combination of antigen components comprises a human Mycobacterium tuberculosis hot water extract.

53. The method of any one of claims 48 to 51, wherein the physical condition comprises influenza vaccination history, influenza infection history, or antigen responsiveness to an influenza virus, and the antigen component or combination of antigen components comprises an influenza virus.

54. The method of any one of claims 48 to 53, wherein the administration comprises subcutaneous administration or intradermal administration.

55. The method of any one of claims 48 to 54, wherein the subject is in a state before onset of cancer, after a cancer treatment, an early stage of onset of cancer, or a precancerous condition.

56. The method of any one of claims 48 to 55, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.

57. The method of any one of claims 48 to 56, wherein the subject exhibits immunological resistance.

58. The method of any one of claims 48 to 57, wherein step ii) comprises measuring induction of cells producing IFN-γ, IL-2, TNF-α, or a plurality of the cytokines concurrently.

59. The method of any one of claims 42 to 58, wherein the antigen responsiveness profile is obtained by performing companion diagnosis in advance based on anamnesis and vaccination history.

60. The method of any one of claims 48 to 59, wherein the past physical condition and the antigen component or combination of antigen components are tuberculosis infection history and a human Mycobacterium tuberculosis hot water extract.

61. The method of any one of claims 48 to 59, wherein the past physical condition and the antigen component or combination of antigen components are influenza infection history and an influenza virus.

62. The method of any one of claims 48 to 61, wherein the past physical condition and the antigen component or combination of antigen components are one or more selected from tuberculosis, malaria, yellow fever virus, smallpox virus, smallpox vaccine, measles/rubella, polio, epidemic parotitis/mumps, rotavirus infection, chickenpox, yellow fever, Ebola, West Nile fever, Hib infection, pneumococcal infection, pertussis, Japanese encephalitis, meningococcal infection, salmonella infection, pathogenic Escherichia coli, toxoplasma, Zika virus, herpesvirus 1, EBV/Epstein Barr Virus (herpesvirus 4), CMV/cytomegalovirus (herpesvirus 5), influenza virus, MARS, rabies, and diphtheria.

63. The method of any one of claims 52 to 60 and 62, wherein the subject is a subject who has BCG vaccination history or tuberculosis infection history, or is confirmed to have antigen responsiveness,
wherein the Mycobacterium tuberculosis extract is prophylactically administered before onset or administered in an early stage of onset of cancer, and an extract from Mycobacterium tuberculosis is subcutaneously or intradermally administered by a conventional method in an early stage of onset of cancer or a precancerous condition.

64. The method of any one of claims 53 to 59 and 61, wherein the subject is a subject who has influenza vaccination history or influenza infection history, or is confirmed to have antigen responsiveness,
wherein the influenza vaccine is prophylactically administered before onset, administered to prevent recurrence after therapy, or administered in an early stage of onset of cancer, and an influenza vaccine is subcutaneously or intradermally administered in an early stage of onset of cancer or a precancerous condition.

65. A method of preventing or treating cancer immunity based on claim 63 or 64, comprising revaccinating the antigen component or combination of antigen components.

66. A method of preventing or treating cancer of a subject with a non-tumor component, wherein the component is an antigen or extract identified by interview and/or identified by reference to anamnesis or vaccination history of the subject, wherein the subject is an individual with an infection history or vaccination history described in claim 49, wherein the component is administered to prevent recurrence after therapy, administered prophylactically before onset, or administered in an early stage of onset of cancer to the subject, and the component is optionally administered in an early stage of onset of cancer or a precancerous condition.

67. The method of any one of claims 63 to 66, wherein the cancer is selected from the group consisting of normal carcinoma, carcinoma with a relatively slow progression, cancer with low sensitivity to the immune system, oral squamous cell cancer, cervical cancer, and MHC class I negative carcinoma on which CD8 positive T cells are generally less effective.

68. The method of claims 63, 66, or 67, wherein the subject is a patient exhibiting immunological resistance.

69. The method of any one of claims 48 to 68, wherein the identification of responsiveness is **characterized by** infection history, vaccination history, and measuring induction of cells producing IFN-γ, IL-2, TNF-α, or a plurality of the cytokines concurrently using peripheral blood.

70. The method of claim 52, wherein the Mycobacterium tuberculosis extract is a hot water extract of human Mycobacterium tuberculosis or other extract from Mycobacterium tuberculosis (highly safe extract).

71. The method of claim 53, wherein the influenza virus is a human influenza virus or other extract from an influenza virus (highly safe extract).

72. The method of claim 42, wherein the antigen component is a protein.

73. A vaccine formulation comprising the antigen of any one of claims 48 to 53 and an adjuvant base.

74. The vaccine formulation of claim 73, wherein the adjuvant base comprises a substance promoting a Th1 immune response.

75. The vaccine formulation of claim 73 or 74, wherein the vaccine formulation is used for personalized medicine.

76. A composition for use in treating or preventing a disease, disorder, or condition associated with an immunological abnormality of a subject, wherein the composition comprises an antigen component that is specific in the subject to a component that is different from a causative agent of the disease, disorder, or condition, and is subcutaneously or intratumorally administered once a day (first week) and once a week (second week and thereafter).

77. The composition of claim 76, wherein the antigen component is contained at about 0.001 µg of more per unit formulation.

78. A method of treating or preventing cancer or tumor in a subject, comprising:
a) identifying a non-tumor antigen specific to the subject based on an antigen responsiveness profile;
b) identifying whether the subject has immunological memory of the non-tumor antigen to identify a subject having the immunological memory; and
c) administering the non-tumor antigen to the subject identified as having the immunological memory.

79. The method of claim 78, wherein the antigen responsiveness profile comprises vaccination history and/or infection history.

80. The method of claim 78 or 79, wherein the identifying a subject having the immunological memory comprising stimulating peripheral blood mononuclear cells (PBMC) isolated from the subject or infiltrating immune cells isolated from a tumor mass with the non-tumor antigen, measuring cytokine production, and identifying a subject with cytokine production increased a predetermined factor compared to before stimulation as a subject having the immunological memory.

81. The method of any one of claims 78 to 80, wherein the non-tumor antigen is administered once a day (first week) and once a week (second week and thereafter).

82. The method of any one of claims 78 to 81, wherein the non-tumor antigen is administered at about 0.001 µg/dose to about 1 mg/dose.

83. A composition for use in treating or preventing a disease, disorder, or condition associated with an immunological abnormality of a subject, comprising mHSP10 and/or MTB12 and/or lipoprotein LpqH.
